# EUROPEAN PATENT SPECIFICATION

(11) **EP 1 003 548 B1**
(45) Date of publication and mention of the grant of the patent: **10.05.2006**
(21) Application number: 98936827.9
(22) Date of filing: 10.07.1998
(51) Int. Cl.: A61K 39/00, A61K 38/19, A61K 9/00

(54) **DEVICE FOR INDUCING A CTL RESPONSE**
VORRICHTUNG ZUR INDUKTION EINER CTL-ANTWORT
DISPOSITIF POUR INDUIRE UNE REACTION DES LYMPHOCYTES T CYTOTOXIQUES

(30) Priority: 10.07.1997 CA 2209815; 10.12.1997 US 988320
(43) Date of publication of application: 31.05.2000
(73) Proprietor: Mannkind Corporation, Valencia, California 91355 (US)
(72) Inventor: KÜNDIG, Thomas, M., CH-8050 Zurich (CH); SIMARD, John, J., L., Northridge, CA 91326 (US)
(74) Representative: Maiwald Patentanwalts GmbH
(86) International application number: PCT/US1998/014289
(87) International publication number: WO 1999/002183

(56) References cited:
- WO-A-95/17167
- R. PETERS ET AL.: "Tryptophan and serotonin metabolism after sustained tryptophan infusion." NEUROCHEMISTRY INTERNATIONAL, vol. 6, no. 5, 1984, pages 685-692, XP002095831
- S. OEHEN ET AL.: "Antivirally protective cytotoxic T cell memory to lymphocytic choriomeningitis virus is governed by persisting antigen." THE JOURNAL OF EXPERIMENTAL MEDICINE, vol. 176, no. 5, 1 November 1992, pages 1273-1281, XP002095832 New York, NY, USA
- M. OLDSTONE ET AL.: "Discriminated selection among viral peptides with the appropriate anchor residues: Implications for the size of the cytotoxic T-lymphocyte repertoire and control of viral infection." JOURNAL OF VIROLOGY, vol. 69, no. 12, December 1995, pages 7423-7429, XP002095833 Washington, DC, USA
- T. KÜNDIG ET AL.: "On the role of antigen in maintaining cytotoxic T-cell memory." PROCEEDINGS OF THE NATIONAL ACADEMY OF SCIENCES OF THE U.S.A., vol. 93, no. 18, 3 September 1996, pages 9716-9723, XP002095834 Washington, DC, USA
- D. MOSKOPHIDIS ET AL.: "Immunobiology of cytotoxic T-cell escape mutants of lymphocytic choriomeningitis virus." JOURNAL OF VIROLOGY, vol. 69, no. 4, April 1995, pages 2187-2193, XP002095835 Washington, DC, USA
- L. DURRANT: "Cancer vaccines." ANTI-CANCER DRUGS, vol. 8, no. 8, September 1997, pages 727-733, XP002095836 Oxford, GB

## Description

### FIELD OF THE INVENTION

The invention relates to a method of inducing a CTL response to an antigen by sustained, regular delivery of the antigen to an animal so that the antigen reaches the lymphatic system.

### BACKGROUND OF THE INVENTION

Cytotoxic T lymphocytes (CTL) are white blood cells found in the blood, spleen and lymph. CTL have the ability to attack and kill other cells of the body in a highly specific manner. When CTL are stimulated by specific antigen, they migrate through the tissues of the body on a "search and destroy" mission for cells bearing the specific antigen. Whether of viral origin or tumor associated, CTL detect antigen that is bound to major histocompatability complexes (MHC) on the surface of potential target cells. Once CTL have identified the antigen on the cell surface, their function is to deliver a lethal hit to the cell.

Although there are hundreds of millions of CTL that reside in the spleen, each individual CTL exclusively responds to a unique and specific antigen. These individual CTL, dubbed CTL precursors (CTLp), undergo cell division or proliferate upon activation by specific antigen to produce daughter cells with precisely the same antigen specificity as the parent cell. This proliferation increases the total number, and thus the frequency; of that specific CTLp in the body. A proportion of these newly generated CTL briefly recirculate through the body (termed effector CTL), and have the ability to identify and destroy cells bearing the specific antigen which they recognize. A significant body of experimental evidence suggests that CTL specific for tumor antigens can inhibit tumor growth. Unfortunately, most tumors have only a very weak capacity to stimulate CTL responses and there has been no means of inducing a CTL response then sustaining it over a period of time sufficient to continuously inhibit tumor growth. While many attempts to directly increase the capacity of tumor cells to stimulate tumor-clearing CTL responses in patients have been made, such attempts have met with limited success. Technical advances over the past ten years have, however, enabled the identification of natural peptide antigens that are present on tumor cells and which are recognized by CTL. These antigen targets include proteins expressed in significant overabundance, abnormally expressed embryonic proteins, protein products from mutated oncogenes or suppressor genes, or proteins derived from cancer-causing viruses present in tumor cells. The challenge has been to find a way in which to administer an antigen so that it induces an anti-tumor CTL response and maintains it over time. While many attempts have now been made to use these antigens clinically in a vaccine, the results have been less than satisfactory.

An explanation of why CTL therapies have been largely ineffective at eradicating or controlling tumors in a clinical setting include the following:
(a) Vaccine designs have been inadequate at initiating strong CTL responses;
(b) Tumor cells can down regulate MHC molecules, resulting in the loss of antigen presentation from the surface of cells, thereby escaping detection by CTL;
(c) After induction, effector CTL recirculation through the body is highly transient;
(d) After recirculation, CTL return to the spleen where they reside in a non-active or resting state, and an increase in the numbers of CTLp residing in the spleen does not reflect active CTL immunity;
(e) In the case of tumors, regrowth of residual tumor cells following immunization goes undetected by CTLp residing in spleen in a "resting" state;
(f) Because CTL-stimulating antigen-presenting cells (APC) are targeted for destruction by the same CTL that they have activated, the CTL response is self-limiting, which precludes, under normal circumstances, the continuous stimulation for a long-lived CTL response.

A growing repertoire of tumor associated antigens are being discovered that are recognized by CTL. A variety of techniques have been suggested to render these antigens effective in CTL vaccines. These include immunization using synthetic peptide antigens mixed with an immunostimulatory adjuvant, such as the bacterial toxin BCG; immunization with multiple antigenic peptide systems (MAPS); immunization with "professional" antigen presenting cells, which are isolated from the patient, pulsed with peptide antigen and inoculated back into the patient as a vaccine; immunization with peptides designed to stimulate both CTL and T helper cell populations; immunization with viruses or bacteria engineered to express tumor antigens; and immunization with polynucleotide expression vectors (so called DNA vaccines). Unfortunately, none of these approaches has been an unqualified success. As discussed above, the lack of vigorous therapeutic effects with these vaccine platforms reflects at least to some degree problems associated with inducing a strong initial CTL response and with maintaining ongoing "active" CTL immunity.

Studies by Glenny during the first quarter of the century revealed that aluminum compounds could enhance the strength of diphtheria vaccines. This was ostensibly the first of a long history of observations supporting a "depot" theory of immunization, which postulates that antigen slowly leaking into the tissues over an extended time correlates with the antigenic potency of a vaccine. Today, this antigen depot paradigm forms the intellectual backdrop to most adjuvant development programs. In one form or another, depot type adjuvants are intended to prolong the course of antigen delivery, by forming a lesion at the site of injection, or simply by the slow degradability of the adjuvant itself, which mixed with the specific antigen forms a depot at the site of injection. A second function generally attributed to adjuvants are their immunostimulatory effects, which appears to trigger the immune system to respond to the vaccine. However, adjuvants are a double-edged sword. They have inherent toxicities. But it is a feature of these toxicities that achieves a desired immunostimulatory and/or depot effect. Side effects such as tissue damage and granulomatous reaction at the site of injection, fever, and in some cases systemic reactions, such as Reiter's syndrome-like symptoms, uveitis and arthritis, are some of the risks associated with the use of adjuvants. Currently, the only adjuvant approved by the FDA is alum. It is relatively safe but does have side effects such as erythema, subcutaneous nodules, contact hypersensitivity, and granulomatous inflammation. More importantly, alum only acts to potentiate a limited number of antigens, and it very predominantly stimulates humoral antibody responses rather than CTL immunity. Thus so far adjuvants have proved to be very ineffective components for vaccines aimed at inducing clinically relevant CTL responses.

Recent attempts to induce CTL responses using dendritic cells or other antigen presenting cells, despite being cumbersome, have shown some promise. New recombinant virus or bacterial systems carrying genes for specific antigen are effective at inducing primary CTL responses. The most effective viruses, for example, that induce strong CTL responses are those which replicate aggressively in the host. Yet because of the risk for serious or lethal complications as a result of infection, recombinant virus used in a cancer vaccine must be only weakly replicative, or be completely replication deficient. This trade-off between virulence and efficacy is at present an intractable problem.

DNA (or polynucleotide) vaccines are also being developed for the purpose of inducing CTL immunity (See e.g. Durrant (1997) Anti-Cancer Drugs 8: 727-733). Once again, the system has intrinsic limitations that preclude its efficacy in inducing long-lasting CTL immunity. The DNA vaccines consist of a plasmid or similar genetic construct for expressing the antigen of interest. Uptake of the plasmid system by cells of the body results in expression of the antigen and induction of CTL. However, once cells expressing the construct have succeeded in inducing CTL, they are themselves targets for eradication by the CTL. The CTL inducing effect is thus again transient. Moreover, the polynucleotide vaccines have thus far suffered from poor efficiency in terms of CTL induction.

With difficulties in achieving strong primary and/or persisting CTL responses, there are a number of clinical trial groups now using repeated injections of cancer vaccines. The use of antigenically complex materials in the vaccine formulation, such as recombinant virus, or the costs associated with repetitive treatment using cultured APC will, however, make such an approach difficult. On the one hand, repetitive immunization with antigenically complex materials drives the immune system to elaborate a humoral antibody, as opposed to a CTL response, while on the other hand, use of a minimal CTL antigen (such as a nonamer peptide) which does not efficiently drive an antibody response, has also failed to induce a CTL response. Attempts to develop adjuvants that enhance the immunostimulatory aspects of minimal CTL antigens have resulted in the production of materials (i.e. adjuvants) that also induce a competing humoral immune response, or, which simply offer little CTL stimulatory effect.

It has also been suggested that certain controlled release technology using microspheres or liposomes with subunit antigens and peptides might be effective to enhance immunogenecity. The combination of sustained release and depot effect is suggested to reduce the amount of antigen needed and eliminate booster shots. However, the preparation of such compositions is difficult and unpredictable, and vaccine formulations based on this technology have not been translated into effective clinical treatments.

As can be seen from the foregoing, there has been little success at developing a CTL vaccine that is both capable of inducing a strong CTL response then sustaining that response over time. The development of a vaccine with these capabilities is essential before effective anti-tumor therapy based on CTL immunity can be contemplated.

### OBJECTS OF THE INVENTION

An object of this invention is to provide an article of manufacture useful for inducing and sustaining a specific immunological CTL response in a mammal over time.

It is a further object of this invention to provide an article of manufacture useful for treating a mammal having a malignant tumor or infectious disease, which article is designed to induce and maintain an immunological attack on the malignant tumor or infectious disease in the mammal.

It is a further object of this invention to provide a portable device for sustained delivery of an antigen to a mammal having a malignant tumor or infectious disease, where the antigen stimulates the mammal's immune system to attack the tumor or infectious disease and the device is located outside the mammal.

It is still a further object of this invention to provide an implantable device for sustained delivery of an antigen to a mammal having a malignant tumor or infectious disease, where the antigen stimulates the mammal's immune system to attack the tumor or infectious disease.

It is a further object of this invention to provide antigen compositions and containers therefor that are useful in the devices, and/or articles of manufacture of this invention.

Other objects of this invention may be apparent to those of skill in the art by reading the following specification and claims.

### SUMMARY OF THE INVENTION

In one aspect of the invention, an article of manufacture is provided for delivering an antigen that induces a CTL response in an animal. In particular, the article comprises a reservoir of a physiologically-acceptable, antigen-containing composition that is capable of inducing a CTL response in an animal; a pump connected to the reservoir to deliver the composition at a defined rate; a transmission line to discharge the composition from the reservoir; and, optionally, a delivery line connected to the transmission line, which delivery line is of a size suitable for positioning in the animal and for delivery of the composition in a manner that reaches the lymphatic system of the animal.

In a further aspect of the invention, a process is provided for preparing a system useful for inducing a sustained CTL response in an animal needing such a response, which comprises placing a physiologically-acceptable, antigen-containing composition in a reservoir having a pump for delivering the composition at a defined rate through a transmission line to the animal.

### BRIEF DESCRIPTION OF THE DRAWINGS

The invention will now be described in relation to the drawings in which:
Figure 1 is a graph showing the lysis of target cells by CTL versus the effector/target ratio when antigen is delivered as a single dose (circles) and when antigen is delivered by a continuous pump (triangles).
Figure 2 (A and B) are graphs showing the lysis of target cells by CTL versus the effector/target ratio when antigen is delivered as a single dose (circles), when antigen is delivered by a continuous pump (triangles) and negative control (squares) at (A) 36 hours and (B) 7 days.
Figure 2C is a graph showing the footpad swelling versus time when antigen is delivered as a single dose (circles) and when antigen is delivered by a continuous pump (triangles).
Figure 3 is a graph showing the lysis of target cells by CTL versus the dose of the peptide antigen when the antigen is delivered subcutaneously, intravenously and intrasplenically.
Figure 4 is a bar graph showing tritiated thymidine uptake in CTL cells induced by antigen introduced intravenously, intrasplenically and subcutaneously.
Figure 5 is a rough schematic of a human lymphatic system.

### DETAILED DESCRIPTION OF THE INVENTION

### Method of treatment

One aspect of this invention is a method for inducing or sustaining a specific immunological response (*i*.*e*., a CTL response) in an animal that has a disease (or predisposition to a disease) in which the animal's immune system may attack the disease with a natural CTL response. The response and diseases are discussed in greater detail hereinafter. The method has particular value for treating an animal having a malignant tumor in order to inhibit the growth of the tumor or for treating a chronic infectious disease such as hepatitis or AIDS.

The method, along with other aspects of the invention, is useful in an animal having an immune system that includes a lymphatic system. This generally includes vertebrates, specifically mammals and particularly humans. Thus, this invention will find use in treating humans of all ages as well as in treating animals, i.e. in veterinary uses. The invention may be used for treating livestock such as cattle, sheep, pigs, goats, and the like or for treating household pets such as dogs, cats, rabbits, hamsters, mice, rats, and the like. The primary use will be for treating humans that are in need of having a specific immunological response sustained for treatment of a disease such as cancer or chronic infections.

A key aspect of this invention is the delivery of an appropriate antigen to the lymphatic system of the animal being treated and sustaining the delivery over time. This is based in part on the observation that a strong induction and a sustained CTL response require ongoing antigenic stimulation of the lymphatic system (Oehen et al. (1992) J.Exp.Med. 176 : 1273-1281). In a human, the lymphatic system includes lymph, lymphocytes, lymph vessels, lymph nodes, tonsils, the spleen, the thymus gland, and bone marrow. The lymphatic system performs three basic functions. First, it helps maintain fluid balance in the tissues. Approximately 30 L of fluid pass from the blood capillaries into the interstitial spaces each day, whereas only 27 L pass from the interstitial spaces back into the blood capillaries. If the extra 3 L of interstitial fluid were to remain in the interstitial spaces, edema would result, causing tissue damage and eventual death. These 3 L of fluid (*i.e.* lymph) enter the lymph capillaries, then passes through the lymph vessels to return to the blood. Lymph is similar in composition to plasma. In addition to water, lymph contains solutes derived from two sources: (1) substances in plasma such as ions, nutrients, gases, and some proteins pass from blood capillaries into the interstitial spaces to become part of the lymph; and (2) substances derived from cells within the tissues such as hormones, enzymes, and waste products are also found in lymph.

The lymphatic system's second basic function is to absorb fats and other substances from the digestive tract. Special lymph vessels called lacteals are in the lining of the small intestine. Fats enter into the lacteals and pass through the lymph vessels to the venous circulation. The lymph passing through these capillaries has a milky appearance because of its fat content, and it is called chyle.

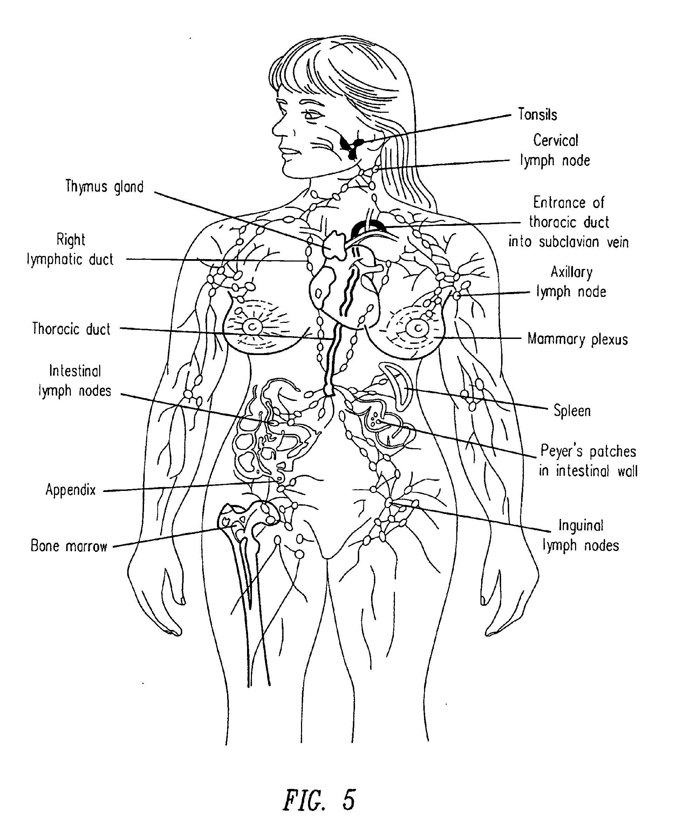

The third basic function of the lymphatic system is to act as part of the body's defense system. The lymph nodes filter lymph, and the spleen filters blood, removing microorganisms and other foreign substances. This third function is the function most important to this invention in that the antigen must be delivered to the lymph system at a level sufficient to elicit the desired, specific immunological response in the animal. Figure 5 is a schematic representation of a human lymphatic system showing the major lymphatic organs and vessels.

The antigen is delivered to the animal so that the antigen is present in the animal's lymphatic system on a sustained basis over a period of time. That is to say, it is delivered in such a way that the presence of the antigen is maintained over the period of time in the animal's lymphatic system. Thus, the antigen is delivered to the animal on a regular basis, i.e. the antigen is delivered regularly without significant interruption over the period of time. This regular delivery is achieved by the constant delivery of the antigen at low levels directly to the lymphatic system using an external device or an implantable device, as discussed hereinafter. Alternatively, the antigen can be delivered at higher levels to the animal by subcutaneous injection with indirect absorption or equilibration with the lymph system. Delivery on a regular basis is meant to include intermittent (stopping and transmitting at intervals) as well as continuous (transmitting without interruption) delivery. In intermittent delivery, the times transmission is stopped will not be enough to reduce the level of antigen in the animal's lymphatic system to eliminate the desired specific immunological response. Thus, the antigen may be delivered in pulses or small doses over time.

Preferably, the sustained delivery is achieved by the positioning of a means of delivery so that the animal being treated does not have to receive multiple injections of the antigen, but instead has only one insertion of the means for delivery, e.g. an insertion of a catheter or needle for infusion of a suitable antigen-containing composition or the surgical implantation of an implantable device that release an appropriate, antigen-containing composition on a sustained basis.

The period of time over which the antigen will be released will be a time sufficient to induce and maintain the desired specific immunological response, e.g. to maintain a CTL response, and in the case of an animal with a tumor or infection, at a level sufficient to stimulate the immune system to attack the tumor and inhibit its growth or to attack the infection. Generally, this period of time may vary from a few days, e.g. a week, to a year or more. Preferably, the treatment, i.e. sustained delivery of the antigen, will extend for at least seven days and no more than six months. It has been found that the CTL response is induced by administration for at least seven days. To determine the period of time, the attending physician will evaluate, i.e., the severity of the condition, the strength of the patient, the antigenic response (e.g., the level of CD8+ cells measurable in the patient's system), the presence of toxic effects, and other factors known to one of skill in the art. Ultimately the time for sustained delivery in a cancer patient will be that necessary for improvement in the patient as evidenced by reduction in the size of the tumor, the rate of growth of the tumor, and/or the improvement in the overall health of the patient being treated. In the treatment of infectious diseases the treatment is continued until the health of the patient improves sufficiently to stop treatment.

The underlying immunological rationale for the utility of this invention arises from certain immunological considerations. The immune system has evolved to protect the host from microbial infection. CD4+ T cells together with B cells are the main components of the immune system humoral effector arm, which is crucial to eliminate extracellular pathogens or toxins. In contrast, the CD8+ T cell arm of the immune system is mainly responsible for eliminating intracellular pathogens, i.e. most importantly viruses, either via cytokine release or by cytotoxic activity. It is now emerging that these most efficient "killer cells" of the immune system would best serve as the primary effector cells in tumor immunotherapy. An object of this invention is to mount a disease-specific CTL response (CD8+ T cell response) against the disease and sustain it over time, *e*.*g*., a tumor specific or microbial specific CTL response.

CD8+ T cells recognize antigenic oligopeptides presented on HLA class I molecules of target cells, e.g., tumor cells. The sequences of many HLA-A1 and HLA-A2 presented tumor and pathogen specific antigen peptides have recently been characterized. These peptides may be used in this invention to induce, *e*.*g*., a melanoma-specific CD8+ T cell response. These peptides are discussed hereinafter.

In contrast to viral infection, class I-binding oligopeptides show only low immunogenicity. Most viruses induce peak CD8+ T cell responses around 7-10 days after systemic spread. This invention aims at enhancing the immunogenecity of class I binding oligopeptides by sustained, regular release of peptide into a lymphatic system and continued release into the lymphatic system.

In contrast to antibody-mediated B cell memory, which is long lived, T cell memory appears to be short lived or non-existent. In accordance with this invention, maintenance of functional T cell memory depends on persistence of antigen through continued, regular administration of the desired antigen. Having made this invention and looking at past concepts that might support this underlying rationale, some evidence includes the observation that delayed type hypersensitivity (DTH) of the tuberculin type (the only functional test for T cell memory in humans), can be elicited only in granulomatous disease, such as tuberculosis (tuberculin test), leprosy (lepromin test), brucellosis (brucellin test), sarcoidosis (Kveim test), Histoplasmosis (histoplasimin test) etc., but no such test could be established for non-granulomatous infectious disease. A factor that all granulomatous diseases have in common, is that the antigen persists within the granuloma - professional antigen presenting cells can use this reservoir to continuously restimulate specific T cells in lymphoid organs. In mice models (see Example 3) it is demonstrated that maintenance of functional CD8+ T cell memory was strictly dependent on continuous antigenic restimulation.

To determine whether a CTL response is obtained in an animal being treated in accordance with this invention, one measures the level of CD8+ cells (*i*.*e*. CTL) present in the blood or lymphatic organs such as the spleen or lymph nodes. This determination is done by first measuring the level of CD8+ cells before performing the method of this invention and measuring the level during treatment, e.g. at 7, 10, 20, 40 days, etc. The level or strength of the CD8+ (CTL) response can be assessed *in vivo* or *in vitro*. In humans, there exists so far only one *in vivo* test to measure CD8+ T cell responses, which is a skin test. In this skin test, HLA class I binding peptides are injected intradermally (such as described in Jäger, E. *et al.* Granulocyte-macrophage-colony-stimulating Factor Enhances Immune Responses To Melanoma-associated Peptides *in vivo Int. J. Cancer* 67, 54-62 (1996)). If a CTL response is present, these cells will recognize and attack peptide pulsed dermal cells, causing a local inflammatory reaction either via cytokine release or the cytotoxic mechanism (Kündig, T.M., Althage, A., Hengartner, H. & Zinkernagel, R.M. A skin test to assess CD8+ cytotoxic T cell activity. *Proc. Natl. Acad. Sci. USA* 89:7757-776 (1992)). This inflammatory reaction can be quantified by measuring the diameter of the local skin rash and/or by measuring the diameter of the infiltrate (i.e., the swelling reaction). As an alternative to the injection of soluble free peptide, the HLA-class I binding peptide can also be injected intradermally in a bound form, e.g., bound to extracorporally derived dendritic cells. In other mammals, additional, although experimental, *in vivo* tests to assess CD8+ T cell responses exist. For example, in a mouse model, CD8+ T cell responses can be measured by challenge infection with a vaccinia recombinant virus expressing the peptide used for immunization. While naive mice succumb to the infection with the vaccina recombinant virus, mice with preexisting CD8+ T cell immunity against the peptide epitope expressed by the vaccinia recombinant virus, are immune to reinfection. The level of immunity to reinfection can be quantified as the factor of reduction of the vaccinia virus titer recovered from mouse organs after challenge infection (Bachmann, M.F. & Kundig, T.M. *In vitro* vs. *in vivo* assays for the assessment of T-and B- cell function. *Curr.Opin.Immunol*. 6, 320-326 (1994)). For example, 5 days after challenge infection, a typical vaccinia recombinant virus titer recovered from a mouse ovary would be around 10⁷ pfu per ovary, whereas the vaccinia recombinant virus titer in a mouse with a preexisting CD8+ T cell response against the recombinant gene product would for example be around 10³ pfu per ovary. Such a 10,000 fold-reduction in virus titer reflects biologically significant preexisting CD8+ T cell activity against the recombinant gene product.

The level of CD8+ T cell responses can also be quantified *in vitro,* by estimating the number of CD8+ T cells specific for the antigenic peptide in question. In a naive mammal the so called "frequency", i.e., the number of specific CD8+ T cells divided by the number of non-specific white blood cells, is less than 10⁻⁶. After successful immunization, the frequency increases due to proliferation of specific T cells. During an acute viral infection, for example, the frequency of specific CD8+ T cells may rise to 10⁻². Then, after elimination of the virus, the frequency of specific CD8+ T cells usually drops to a "memory" level of around 10⁻⁴. Thus, the CD8+ T cell response can be quantified by measuring the frequency of specific CD8+ T cells. The higher the frequency, the stronger the response. The classical assays used to measure the frequency of specific CD8+ T cells are based on limiting dilution cell culture techniques, as described in detail by Kündig, T.M. et al. (On the role of antigen in maintaining cytotoxic T cell memory. *Proceedings of the National Academy of Sciences of the United States of America* 93, 9716-9723 (1996)). A novel approach to estimate the frequency of specific CD8+ T cells is to construct soluble class I MHC (for use in mice) or HLA molecules (for use in humans) with a peptide bound to their groove, so that the specific T cell receptors will bind to these complexes. These complexes can be labeled for detection, for example, with a fluorescent substance, allowing for detection by flow cytometry.

One current procedure to render peptides immunogenic is to inject them in context with "nature's most potent adjuvant", *i.e.,* professional antigen presenting cells (APCs) such as dendritic cells (DCs) (Steinmann, R.M., The dendritic cells system and its role in immunogenicity, *Annual Review of Immunology* 9, 271-96 (1991)). DCs are the most potent APCs of the immune system. They can now be cultured in vitro by adding granulocyte macrophage colony stimulating factor (GM-CSF) and tumor necrosis factor alpha (TNF-alpha) or interleukin-4 (IL-4) to progenitors isolated from the blood of patients or mice (Inaba, K. *et al*., Identification of proliferating dendritic cell precursors in mouse blood, *Journal of Experimental Medicine* 175, 1157-1167 (1992)). Large numbers of DCs can then be pulsed with tumor specific antigen peptides and are injected back into the patient, where they migrate into lymphatic organs to induce T cell responses (Young, J.W. & Inaba, K., Dendritic Cells As Adjuvants For Class I Major Histocompatibility Complex-restricted Anti-tumor Immunity, *Journal of Experimental Medicine* 183, 7-11 (1996)). An object of this invention is to circumvent the time-consuming, labor intensive procedure of culturing DCs after isolation of DC progenitors and deliver the antigen to the lymphatic system free of APCs such as DCs. The method of this invention, i.e., the sustained, regular delivery of antigen into a lymphatic organ, allows sufficiently high local concentrations of antigen inside the lymphatic organ, such that professional antigen presenting cells, e.g., dendritic cells, can be loaded with peptide *in vivo.* This can be viewed as a method of loading antigen presenting cells (dendritic cells) *in vivo* for inducing a CTL response.

The method of the present invention is clearly advantageous over the prior art methods for inducing a CTL response against a tumor or virus. For example, the present invention does not require repetitive immunizations to effect for prolonged anti-tumor immunotherapy. The sustained delivery of the antigen maintains the CTL response that could ultimately afford a prolonged aggressive posture of CTL against tumor cells, more thorough eradication, and protection against recurrence during the vaccine treatment. In the absence of antigen, CTL that have undergone primary activation soon cease to recirculate through the body, soon finding their way to the spleen where they become quiescent. Since CTL must immediately deliver a lethal hit, their residence in the spleen precludes an active role in protection against infections or tumor growth at distant sites in the body. The controlled release of antigen recognized by CTL in this invention circumvents this outcome as antigen delivery is maintained. Sustained released antigen delivery to the lymphatic system by this invention solves two major problems: it provides for potent CTL stimulation that takes place in the milieu of the lymphoid organ, and it sustains stimulation that is necessary to keep CTL active, cytotoxic and recirculating through the body.

Another fundamental improvement of the present method over prior art is that it facilitates the use of inherently non-immunogenic peptide antigens for CTL stimulation without the combined use of conventional adjuvants. This is very beneficial as most experimental adjuvants are toxic and poorly suited for use in humans. In addition adjuvants stimulate the TH2-type humoral immune response that negatively affects the CTL response. Further, since conventional adjuvants are not required, only the minimal antigenic epitope for a CTL response is required in the formulation.

An additional advantage to the method of the present invention, where it embodies the use of mechanical delivery systems, is that the antigen delivery can be stopped if any adverse immunological effects are observed. For example, in vaccines against melanoma, CTL have been induced to attack not only malignant melanocytes but also healthy tissue, causing "vitiligo." The ability to discontinue a CTL vaccine at any time is a significant advance in vaccine safety. Peptides have a short half-life due to catabolism in the liver. Therefore, the stimulation-effect falls soon after cessation of delivery.

As pointed out before, the method of this invention has two parts: (1) inducing an increased CTL response and (2) maintaining the response. The inducing and maintaining may be performed using the same device, as discussed hereinafter, or the inducing may be done separately, e.g., by a separate injection of an antigen then following up with sustained delivery of the antigen over time to maintain the response.

### Diseases treated according to the invention

In general, this invention is useful for treating an animal having (or being predisposed to) any disease to which the animal's immune system mounts a cell-mediated response to a disease-related antigen in order to attack the disease. Thus, the type of disease may be a malignant tumor or a chronic infectious disease caused by a bacterium, virus, protozoan, helminth, or other microbial pathogen that enters intracellularly and is attacked, *i.e*., by the cytotoxic T lymphocytes. In addition, the invention is useful for treating an animal that may be at risk of developing such diseases.

### Malignant Tumors

In a mature animal, a balance usually is maintained between cell renewal and cell death in most organs and tissues. The various types of mature cells in the body have a given life span; as these cells die, new cells are generated by the proliferation and differentiation of various types of stem cells. Under normal circumstances, the production of new cells is so regulated that the numbers of any particular type of cell remain constant. Occasionally, though, cells arise that are no longer responsive to normal growth-control mechanisms. These cells give rise to clones of cells that can expand to a considerable size, producing a *tumor*, or *neoplasm.* A tumor that is not capable of indefinite growth and does not invade the healthy surrounding tissue extensively is *benign.* A tumor that continues to grow and becomes progressively invasive is *malignant;* the term *cancer* refers specifically to a malignant tumor. In addition to uncontrolled growth, malignant tumors exhibit *metastasis;* in this process, small clusters of cancerous cells dislodge from a tumor, invade the blood or lymphatic vessels, and are carried to other tissues, where they continue to proliferate. In this way a primary tumor at one site can give rise to a secondary tumor at another site. The methods, devices and articles of manufacture discussed herein are useful for treating animals having malignant tumors.

Malignant tumors treated according to this invention are classified according to the embryonic origin of the tissue from which the tumor is derived. *Carcinomas* are tumors arising from endodermal or ectodermal tissues such as skin or the epithelial lining of internal organs and glands. A melanoma is a type of carcinoma of the skin for which this invention is particularly useful. *Sarcomas,* which arise less frequently, are derived from mesodermal connective tissues such as bone, fat, and cartilage. The *leukemias* and *lymphomas* are malignant tumors of hematopoietic cells of the bone marrow. Leukemias proliferate as single cells, whereas lymphomas tend to grow as tumor masses. The malignant tumors may show up at numerous organs or tissues of the body to establish a cancer. The types of cancer that can be treated in accordance with this invention include the following: bladder, brain, breast, cervical, colo-rectal, esophageal, kidney, liver, lung, nasopharangeal, pancreatic, prostate, skin, stomach, uterine, and the like. The present invention is not limited to the treatment of an existing tumor or infectious disease but can also be used to prevent or lower the risk of developing such diseases in an individual, *i.e.,* for prophylactic use. Potential candidates for prophylactic vaccination include individuals with a high risk of developing cancer, *i.e.*, with a personal or tuminal history of certain types of cancer.

The incidence of skin cancer has increased substantially over the last decades. Lifetime analysis indicates that around 1/1500 humans born in 1935, 1/600 born in 1960, 1/100 born in 1990 and a projected 1/75 humans born in the year 2000 will have melanoma in their lifetime. Surgical excision usually cures melanoma. However, even small looking lesions may have already metastasized at the time of diagnosis. The prognosis of metastasized melanoma is very poor and correlates with the thickness of the primary tumor and with its localization.

The current treatment of malignant melanoma aims at surgical removal of the primary tumor. If metastases are present, chemotherapy and biological response modifiers are additionally used. However, patients with stage IV malignant melanoma are almost invariably incurable and treatments are palliative. Patients with Stage IV malignant melanoma have a median survival time of approximately one year and only a 10% chance of long-term survival. There is at present no generally accepted standard therapy for metastatic melanoma. Objective response rates to mono- or polychemotherapy are low in comparison with other tumors, reaching no more than 15-35%. An improved treatment outcome in stage IV malignant melanoma seems unachievable either by chemotherapeutic combinations or by increasing doses to levels where autologous bone marrow transplantation becomes necessary. The method of this invention is useful for treating malignant melanoma, even at Stage IV.

### Infectious Diseases

Infectious diseases, which have plagued animal populations (particularly humans) throughout history, still cause millions of deaths each year. The infectious diseases that can be treated using this invention include those caused by pathogens such as bacteria, viruses, protozoa, helminths, and the like. These diseases include such chronic diseases such as acute respiratory infections, diarrheal diseases, tuberculosis, malaria, hepatitis (hepatitis A, B C, D, E, F virus), measles, mononucleosis (Epstein-Barr virus), whooping cough (pertussis), AIDS (human immunodeficiency virus 1 & 2), rabies, yellow fever, and the like. Other diseases caused by human papilloma virus or various strains of virus are treatable by this method.

In some instances, the mammal, in particular human, can be treated prophylactically, such as when there may be a risk of developing disease. An individual travelling to or living in an area of endemic infectious disease may be considered to be at risk and a candidate for prophylactic vaccination against the particular infectious agent. For example, the CTL response can be induced in a human expecting to enter a malarial area and/or while in the malarial area by using a CTL-inducing, malaria-specific antigen to lower the risk of developing malaria. Preventative treatment can be applied to any number of diseases including those listed above, where there is a known relationship between the particular disease and a particular risk factor, such as geographical location or work environment.

### Antigens useful in the invention

An antigen useful in this invention is one that stimulates the immune system of a mammal having a malignant tumor or infectious disease to attack the tumor and inhibit its growth or to destroy the pathogen causing the disease. Thus, the antigen used in the invention is matched to the specific disease found in the animal being treated. In this regard the antigen may be said to induce a CTL response (also referred to as a cell-mediated immune response), i.e. a cytotoxic reaction by the immune system that results in lysis of the target cells (*e*.*g*., the malignant tumor cells or pathogen-infected cells).

To determine whether an antigen is matched to a particular patient, whether human or other animal, the tissue type of the patient is first determined. If human, the tissue must demonstrate the appropriate human leukocyte antigen (HLA) capable of binding and displaying the antigen to CTL. It is preferable that the HLA typing be performed on the target cells, since a significant portion of tumors escape immune detection by downregulating expression of HLA. Therefore HLA expression on normal cells of the patient does not necessarily reflect that found on tumor cells in their body. A tumor from a patient is also screened to determine if he or she expresses the antigen that is being used in the vaccine formulation. Immunohistochemistry and/or polymerase chain reaction (PCR) techniques both can be used to detect antigen in the tumor cells. Immunohistochemistry offers the advantage in that it stains a cross-section of tumor in a slide preparation, allowing investigators to observe the antigen expression pattern in cross-section of tumor, which is typically heterogeneous for antigen expression. PCR has the advantage of not requiring specific monoclonal antibodies for staining and is a fast and powerful technique. In addition, PCR can be applied in situ. Ideally, both immunohistochemical and PCR methods should be combined when assessing antigen expression in tumors. While the antigen compositions useful in this invention are designed to include the most commonly expressed tumor antigens (as discussed hereafter), not all tumors will express the desired antigen(s). Where a tumor fails to express the desired antigen, the patient is excluded for consideration for that particular antigen composition. Thus, an aspect of this invention is a process for preparing a device useful for providing a sustained CTL response over time by matching a subject's antigen specific to the tumor or pathogen in the subject, preparing a physiologically-acceptable composition of the antigen so matched, and combining the composition in a suitable delivery device as discussed in hereinafter.

Immune activation of CD8+ T cells generates a population of effector cells with lytic capability called cytotoxic T lymphocytes, or CTL. These effector cells have important roles in the recognition and elimination of malignant cells and pathogens. In general, CTL are CD8+ and are therefore class I MHC restricted, although in rare instances CD4+ class II - restricted T cells have been shown to function as CTL. Since virtually all nucleated cells in the body express class I MHC molecules, CTL can recognize and eliminate almost any altered body cell. CD8+T cells recognize antigen presented on HLA class I molecules of tumor cells through T cell receptors.

The CTL-mediated immune response can be divided into two phases, reflecting different aspects of the cytotoxic T-cell response. The first phase involves the activation and differentiation of T_{c} (CD8+) cells into functional effector CTLs. In the second phase, CTLs recognize antigen - class I MHC complexes on specific target cells, initiating a sequence of events that culminates in target-cell destruction. Further detailed discussion of the process is found at Chapter 15 of the Second Edition of "Immunology" by Janis Kuby, W.H. Freeman and Company (1991).

The type of tumor antigen useful in this invention may be a *tumor-specific antigen (TSA)* or a *tumor-associated antigen (TAA).* A *TSA* is unique to tumor cells and does not occur on other cells in the body. A *TAA* associated antigen is not unique to a tumor cell and instead is also expressed on a normal cell under conditions that fail to induce a state of immunologic tolerance to the antigen. The expression of the antigen on the tumor may occur under conditions that enable the immune system to respond to the antigen. *TAA*s may be antigens that are expressed on normal cells during fetal development when the immune system is immature and unable to respond or they may be antigens that are normally present at extremely low levels on normal cells but which are expressed at much higher levels on tumor cells. TSAs and TAAs can be jointly referred to as TRA or a tumor related antigen.

Tumor antigens useful in the present invention, whether tumor-specific or tumor-associated, must be capable of inducing a CTL-mediated immune response. The presence of tumor antigens that elicit a cell-mediated response has been demonstrated by the rejection of tumors transplanted into syngeneic recipients; because of this phenomenon, these tumor antigens are referred to as *tumor-specific transplantation antigens (TSTAs)* or *tumor-associated transplantation antigens (TATAs)*. It has been difficult to characterize tumor transplantation antigens because they do not generally elicit an antibody response and therefore they cannot be isolated by immunoprecipitation. Many are peptides that are presented together with MHC molecules on the surface of tumor cells and have been characterized by their ability to induce an antigen-specific CTL.

The type of pathogen specific antigen useful in this invention may be short oligopeptides derived from pathogen proteins. These oligopeptides must bind to class I MHC (for use in mice), class I HLA (for use in humans), or class I molecules of any other mammals. Also, such class I molecule bound peptides should be recognizable by specific T cell receptors. Such oligopeptides usually have a length of 8-15 amino acids. Several examples of such pathogen derived oligopeptides, so called T cell epitopes, are given in Tables I and II.

The tumor antigens and pathogen-specific antigens useful in this invention are generally thought to be presented at the surface of an antigen presenting cell (APC) to stimulate the immune system through class I molecules of the major histocompatability complex (MHC) interactively with the CD8+ cells.

Antigens useful in the invention are generally protein-based entities of a molecular weight of up to 100,000 daltons. Appropriate antigens include, but are not limited to differentiation antigens, tumor-specific multilineage antigens, embryonic antigens, antigens of oncogenes and mutated tumor-suppressor genes, unique tumor antigens resulting from chromosomal translocations, viral antigens, and others that may be apparent presently or in the future to one of skill in the art. It is preferable that the antigen be a peptide of 8 to 15 amino acids in length that is an epitope of a larger antigen, i.e. it is a peptide having an amino acid sequence corresponding to the site on the larger molecule that is recognized and bound by a particular T-cell receptor. These smaller peptides are available to one of skill in the art by following the teachings of U.S. Patents 5,747,269 to Rammensee et al. issued May 5, 1998; 5,698,396 to Pfreundschuh issued December 16, 1997; and WO96/01429, WO 96/27008 and WO 98/13489.

A powerful method has been recently developed for identifying new peptides that are useful in the invention. Genes determined to express protein with high exclusivity in tumor cells or microbial cells (e.g. viruses) can be identified using a so called SEREX process, which involves expression cloning using tumor cell libraries and screening these libraries against immunoglobulin in patient sera. Over one hundred genes have recently been identified from tumor biopsies using this process. These genes can now be used in a peptide prediction algorithm developed by Hans-Georg Rammensee. Algorithms have been developed for all major HLA types found in the human population. First the protein sequence is "translated" based on the gene sequence. The algorithms can predict peptide epitopes for various HLA types based on the protein sequence. Since the predicted peptides are indeed predictions and are not always naturally found on cells, tumor samples are used to confirm the predicted peptides by actually isolating minute trace peptide from tumors. Being able to calculate the exact mass of the predicted peptides allows trace peptide identification using ultrasensitive mass spectrophotometry, which can detect peptides in quantities less than that which would permit peptide sequencing and identification. Once these tumor-associated peptides have been identified they are suitable for use in the invention, since peptides of a known sequence may be synthesized in large quantities (several grams) providing for sufficient amounts of peptides for use in this invention.

Thus it can be seen that another aspect of this invention is a process for preparing a composition useful in a device of this invention as discussed hereinafter. The process comprises identifying a gene determined to express a protein with high exclusivity in a tumor or microbial cell, cloning cell libraries, screening the libraries against immunoglobulin in patient sera, using the algorithm defined in the literature developed by Hans-George Rammensee to predict an epitope for the HLA type protein based on the gene sequence, matching the predicted antigen sequence to a patient tumor sample, isolating the matched antigen, and preparing a composition of the antigen for use in a delivery device as discussed hereinafter.

Examples of large, protein-based antigens include the following:
differentiation antigens such as MART-1/MelanA (MART-I), gp100 (Pmel 17), tyrosinase, TRP-1, TRP-2 and tumor-specific multilineage antigens such as MAGE-1, MAGE-3, BAGE, GAGE-1, GAGE-2, p15; overexpressed embryonic antigens such as CEA; overexpressed oncogenes and mutated tumor-suppressor genes such as p53, Ras, HER-2/neu; unique tumor antigens resulting from chromosomal translocations such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, β-Catenin, CDK4, Mum-1, p15, p16. These protein-based antigens are known and available to those of skill in the art in the literature or commercially.

Examples of peptide antigens of 8-15 amino acids include those set forth in Table I, Table II, and Table III. Table I sets forth antigens that are virally derived. The Table shows the virus type, the protein expressed by the virus, the amino acid (AA) position on the viral protein, the AA sequence of the T-cell epitope/MHC ligand, the type of MHC molecule presenting the antigen, and a reference source. A more complete list is provided in the book by Han-Georg Rammensee, Jutta Bachmann, and Stefan Stevanovic entitled "MHC Ligands and Peptide Motifs," Springer-Verlag, Germany, 1997 Landes Bioscience, Austin, Texas). The reference number given in Table I is the same number (and reference source) given in Table 5.3 of the above Rammensee book.

**Table I: Viral epitopes on MHC class 1 molecules**

| **Virus** | **Protein** | **AA position** | **T cell epitope/ MHC ligand (Antigen)** | | **MHC molecule** | **Ref.** |
|---|---|---|---|---|---|---|
| Adenovirus 3 | E3 9Kd | 30-38 | LIVIGILIL | | HLA-A*0201 | 104 |
| | | | | (SEQ. ID NO.:1) | | |
| Adenovirus 5 | E1A | 234-243 | SGPSNTPPEI | | H2-D^{b} | 105 |
| | | | | (SEQ. ID NO.:2) | | |
| Adenovirus 5 | E1B | 192-200 | VNIRNCCYI | | H2-D^{b} | 106 |
| | | | | (SEQ. ID NO.:3) | | |
| Adenovirus 5 | E1A | 234-243 | SGPSNIPPEI (T>I) | | H2-D^{b} | 106 |
| | | | | (SEQ. ID NO.:4) | | |
| CSFV | NS polyprotein | 2276-2284 | ENALLVALF | | SLA, haplotype d/d | 107 |
| | | | | (SEQ. ID NO.:5 | | |
| Dengue virus 4 | NS3 | 500-508 | TPEGIIPTL | | HLA-B*3501 | 108, 109 |
| | | | | (SEQ. ID NO.:6 | | |
| EBV | LMP-2 | 426-434 | CLGGLLTMV | | HLA-A*0201 | 110 |
| | | | | (SEQ. ID NO.:7) | | |
| EBV | EBNA-1 | 480-484 | NIAEGLRAL | | HLA-A*0201 | 111 |
| | | | | (SEQ. ID NO.:8) | | |
| EBV | EBNA-1 | 519-527 | NLRRGTALA | | HLA-A*0201 | 111 |
| | | | | (SEQ. ID NO.:9) | | |
| EBV | EBNA-1 | 525-533 | ALAIPQCRL | | HLA-A*0201 | 111 |
| | | | | (SEQ. ID NO.:10) | | |
| EBV | EBNA-1 | 575-582 | VLKDAIKDL | | HLA-A*0201 | 111 |
| | | | | (SEQ. ID NO.:11) | | |
| EBV | EBNA-1 | 562-570 | FMVFLQTHI | | HLA-A*0201 | 111 |
| | | | | (SEQ. ID NO.:12) | | |
| EBV | EBNA-2 | 15-23 | HLIVDTDSL | | HLA-A*0201 | 111 |
| | | | | (SEQ. ID NO.:13) | | |
| EBV | EBNA-2 | 22-30 | SLGNPSLSV | | HLA-A*0201 | 111 |
| | | | | (SEQ. ID NO.:14) | | |
| EBV | EBNA-2 | 126-134 | PLASAMRML | | HLA-A*0201 | 111 |
| | | | | (SEQ. ID NO.:15) | | |
| EBV | EBNA-2 | 132-140 | RMLWMANYI | | HLA-A*0201 | 111 |
| | | | | (SEQ. ID NO.:16) | | |
| EBV | EBNA-2 | 133-141 | MLWMANYIV | | HLA-A*0201 | 111 |
| | | | | (SEQ. ID NO.:17) | | |
| EBV | EBNA-2 | 151-159 | ILPQGPQTA | | HLA-A*0201 | 111 |
| | | | | (SEQ. ID NO.:18) | | |
| EBV | EBNA-2 | 171-179 | PLRPTAPTI | | HLA-A*0201 | 111 |
| | | | | (SEQ. ID NO.:19) | | |
| EBV | EBNA-2 | 205-213 | PLPPATLTV | | HLA-A*0201 | 111 |
| | | | | (SEQ. ID NO.:20) | | |
| EBV | EBNA-2 | 246-254 | RMHLPVLHV | | HLA-A*0201 | 111 |
| | | | | (SEQ. ID NO.:21) | | |
| EBV | EBNA-2 | 287-295 | PMPLPPSQL | | HLA-A*0201 | 111 |
| | | | | (SEQ. ID NO.:22) | | |
| EBV | EBNA-2 | 294-302 | QLPPPAAPA | | HLA-A*0201 | 111 |
| | | | | (SEQ. ID NO.:23) | | |
| EBV | EBNA-2 | 381-389 | SMPELSPVL | | HLA-A*0201 | 111 |
| | | | | (SEQ. ID NO.:24) | | |
| EBV | EBNA-2 | 453-461 | DLDESWDYI | | HLA-A*0201 | 111 |
| | | | | (SEQ. ID NO.:25) | | |
| EBV | BZLF1 | 43-51 | PLPCVLWPV | | HLA-A*0201 | 111 |
| | | | | (SEQ. ID NO.:26) | | |
| EBV | BZLF1 | 167-175 | SLEECDSEL | | HLA-A*0201 | 111 |
| | | | | (SEQ. ID NO.:27) | | |
| EBV | BZLF1 | 176-184 | EIKRYKNRV | | HLA-A*0201 | 111 |
| | | | | (SEQ. ID NO.:28) | | |
| EBV | BZLF1 | 195-203 | QLLQHYREV | | HLA-A*0201 | 111 |
| | | | | (SEQ. ID NO.:29) | | |
| EBV | BZLF1 | 196-204 | LLQHYREVA | | HLA-A*0201 | 111 |
| | | | | (SEQ. ID NO.:30) | | |
| EBV | BZLF1 | 217-225 | LLKQMCPSL | | HLA-A*0201 | 111 |
| | | | | (SEQ. ID NO.:31) | | |
| EBV | BZLF1 | 229-237 | SIIPRTPDV | | HLA-A*0201 | 111 |
| | | | | (SEQ. ID NO.:32) | | |
| EBV | EBNA-6 | 284-293 | LLDFVRFMGV | | HLA-A*0201 | 112 |
| | | | | (SEQ. ID NO.:33) | | |
| EBV | EBNA-3 | 464-472 | SVRDRLARL | | HLA-A*0203 | 113 |
| | | | | (SEQ. ID NO.:34) | | |
| EBV | EBNA-4 | 416-424 | IVTDFSVIK | | HLA-A*1101 | 114, 115 |
| | | | | (SEQ. ID NO.:35) | | |
| EBV | EBNA-4 | 399-408 | AVFDRKSDAK | | HLA-A*0201 | 116 |
| | | | | (SEQ. ID NO.:36) | | |
| EBV | EBNA-3 | 246-253 | RYSIFFDY | | HLA-A24 | 113 |
| | | | | (SEQ. ID NO.:37) | | |
| EBV | EBNA-6 | 881-889 | QPRAPIRPI | | HLA-B7 | 117 |
| | | | | (SEQ. ID NO.:38) | | |
| EBV | EBNA-3 | 379-387 | RPPIFIRRL | | HLA-B7 | 117 |
| | | | | (SEQ. ID NO.:39) | | |
| EBV | EBNA-1 | 426-434 | EPDVPPGAI | | HLA-B7 | 111 |
| | | | | (SEQ. ID NO.:40) | | |
| EBV | EBNA-1 | 228-236 | IPQCRLTPL | | HLA-B7 | 111 |
| | | | | (SEQ. ID NO.:41) | | |
| EBV | EBNA-1 | 546-554 | GPGPQPGPL | | HLA-B7 | 111 |
| | | | | (SEQ. ID NO.:42) | | |
| EBV | EBNA-1 | 550-558 | QPGPLRESI | | HLA-B7 | 111 |
| | | | | (SEQ. ID NO.:43) | | |
| EBV | EBNA-1 | 72-80 | RPQKRPSCI | | HLA-B7 | 111 |
| | | | | (SEQ. ID NO.:44) | | |
| EBV | EBNA-2 | 224-232 | PPTPLLTVL | | HLA-B7 | 111 |
| | | | | (SEQ. ID NO.:45) | | |
| EBV | EBNA-2 | 241-249 | TPSPPRMHL | | HLA-B7 | 111 |
| | | | | (SEQ. ID NO.:46) | | |
| EBV | EBNA-2 | 244-252 | PPRMHLPVL | | HLA-B7 | 111 |
| | | | | (SEQ. ID NO.:47) | | |
| EBV | EBNA-2 | 254-262 | VPDQSMHPL | | HLA-B7 | 111 |
| | | | | (SEQ. ID NO.:48) | | |
| EBV | EBNA-2 | 446-454 | PPSIDPADL | | HLA-B7 | 111 |
| | | | | (SEQ. ID NO.:49) | | |
| EBV | BZLF1 | 44-52 | LPCVLWPVL | | HLA-B7 | 111 |
| | | | | (SEQ. ID NO.:50) | | |
| EBV | BZLF1 | 222-231 | CPSLDVDSII | | HLA-B7 | 111 |
| | | | | (SEQ. ID NO.:51) | | |
| EBV | BZLF1 | 234-242 | TPDVLHEDL | | HLA-B7 | 111 |
| | | | | (SEQ. ID NO.:52) | | |
| EBV | EBNA-3 | 339-347 | FLRGRAYGL | | HLA-B8 | 118 |
| | | | | (SEQ. ID NO.:53) | | |
| EBV | EBNA-3 | 26-34 | QAKWRLQTL | | HLA-B8 | 113 |
| | | | | (SEQ. ID NO.:54) | | |
| EBV | EBNA-3 | 325-333 | AYPLHEQHG | | HLA-B8 | 116 |
| | | | | (SEQ. ID NO.:55) | | |
| EBV | EBNA-3 | 158-166 | YIKSFVSDA | | HLA-B8 | 116 |
| | | | | (SEQ. ID NO.:56) | | |
| EBV | LMP-2 | 236-244 | RRRWRRLTV | | HLA-B*2704 | 119 |
| | | | | (SEQ. ID NO.:57) | | |
| EBV | EBNA-6 | 258-266 | RRIYDLIEL | | HLA-B*2705 | 119 |
| | | | | (SEQ. ID NO.:58) | | |
| EBV | EBNA-3 | 458-466 | YPLHEQHGM | | HLA-B*3501 | 120 |
| | | | | (SEQ. ID NO.:59) | | |
| EBV | EBNA-3 | 458-466 | YPLHEQHGM | | HLA-B*3503 | 113 |
| | | | | (SEQ. ID NO.:59) | | |
| HCV | NS3 | 389-397 | HSKKKCDEL | | HLA-B8 | 145 |
| | | | | (SEQ. ID NO.:60) | | |
| HCV | env E | 44-51 | ASRCWVAM | | HLA-B*3501 | 146 |
| | | | | (SEQ. ID NO.:61) | | |
| HCV | core protein | 27-35 | GQIVGGVYL | | HLA-B*40012 | 147 |
| | | | | (SEQ. ID NO.:62) | | |
| HCV | NS1 | 77-85 | RPLTDFDQGW | | HLA-B*5301 | 145 |
| | | | | (SEQ. ID NO.:63) | | |
| HCV | core protein | 18-27 | LMGYIPLVGA | | H2-D^{d} | 138 |
| | | | | (SEQ. ID NO.:64) | | |
| HCV | core protein | 16-25 | ADLMGYIPLV | | H2-D^{d} | 148 |
| | | | | (SEQ. ID NO.:65) | | |
| HCV | NS5 | 409-424 | MSYSWTGALVTPCAEE | | H2-D^{d} | 149 |
| | | | | (SEQ. ID NO.:66) | | |
| HCV | NS1 | 205-213 | KHPDATYSR | | Papa-A06 | 150 |
| | | | | (SEQ. ID NO.:67) | | |
| HCV-1 | NS3 | 400-409 | KLVALGINAV | | HLA-A*0201 | 141 |
| | | | | (SEQ. ID NO.:68) | | |
| HCV-1 | NS3 | 440-448 | GDFDSVIDC | | Patr-BI6 | 151 |
| | | | | (SEQ. ID NO.:69) | | |
| HCV-1 | env E | 118-126 | GNASRCWVA | | Patr-B16 | 151 |
| | | | | (SEQ. ID NO.:70) | | |
| HCV-1 | NS1 | 159-167 | TRPPLGNWF | | Patr-B13 | 151 |
| | | | | (SEQ. ID NO.:71) | | |
| HCV-1 | NS3 | 351-359 | VPHPNIEEV | | Patr-B13 | 151 |
| | | | | (SEQ. ID NO.:72) | | |
| HCV-1 | NS3 | 438-446 | YTGDFDSVI | | Patr-B01 | 151 |
| | | | | (SEQ. ID NO.:73) | | |
| HCV-1 | NS1 | 328-335 | SWAIKWEY | | Patr-A11 | 151 |
| | | | | (SEQ. ID NO.:74) | | |
| HCV-1 | NS1 | 205-213 | KHPDATYSR | | Patr-A04 | 150 |
| | | | | (SEQ. ID NO.:75) | | |
| HCV-1 | NS3 | 440-448 | GDFDSVIDC | | Patr-A04 | 150 |
| | | | | (SEQ. ID NO.:76) | | |
| HIV | gp41 | 583-591 | RYLKDQQLL | | HLA-A24 | 152 |
| | | | | (SEQ. ID NO.:77) | | |
| HIV | gagp24 | 267-275 | IVGLNKIVR | | HLA-A*3302 | 153, 154 |
| | | | | (SEQ. ID NO.:78) | | |
| HIV | gagp24 | 262-270 | EIYKRWIIL | | HLA-B8 | 155,156 |
| | | | | (SEQ. ID NO.:79) | | |
| HIV | gagp24 | 261-269 | GEIYKRWII | | HLA-B8 | 155, 156 |
| | | | | (SEQ. ID NO.:80) | | |
| HIV | gagp17 | 93-101 | EIKDTKEAL | | HLA-B8 | 155, 157 |
| | | | | (SEQ. ID NO.:81) | | |
| HIV | gp41 | 586-593 | YLKDQQLL | | HLA-B8 | 158 |
| | | | | (SEQ. ID NO.:82) | | |
| HIV | gagp24 | 267-277 | ILGLNKIVRMY | | HLA-B*1501 | 153 |
| | | | | (SEQ. ID NO.:83) | | |
| HIV | gp41 | 584-592 | ERYLKDQQL | | HLA-B14 | 158 |
| | | | | (SEQ. ID NO.:84) | | |
| HIV | nef | 115-125 | YHTQGYFPQWQ | | HLA-B17 | 159 |
| | | | | (SEQ. ID NO.:85) | | |
| HIV | nef | 117-128 | TQGYFPQWQNYT | | HLA-B17 | 159 |
| | | | | (SEQ. ID NO.:86) | | |
| HIV | gp120 | 314-322 | GRAFVTIGK | | HLA-B*2705 | 160, 184 |
| | | | | (SEQ. ID NO.:87) | | |
| HIV | gagp24 | 263-271 | KRWIILGLN | | HLA-B*2702 | 161 |
| | | | | (SEQ. ID NO.:88) | | |
| HIV | nef | 72-82 | QVPLRPMTYK | | HLA-B*3501 | 159 |
| | | | | (SEQ. ID NO.:89) | | |
| HIV | nef | 117-125 | TQGYFPQWQ | | HLA-B*3701 | 159 |
| | | | | (SEQ. ID NO.:90) | | |
| HIV | gagp24 | 143-151 | HQAISPRTL | | HLA-Cw*0301 | 162 |
| | | | | (SEQ. ID NO.:91) | | |
| HIV | gagp24 | 140-151 | QMVHQAISPRTL | | HLA-Cw*0301 | 162 |
| | | | | (SEQ. ID NO.:92) | | |
| HIV | gp120 | 431-440 | MYAPPIGGQI | | H2-K^{d} | 163 |
| | | | | (SEQ. ID NO.:93) | | |
| HIV | gp160 | 318-327 | RGPGRAFVTI | | H2-D^{d} | 164, 165 |
| | | | | (SEQ. ID NO.:94) | | |
| HIV | gp120 | 17-29 | MYGRAFVTI | | H2-L° | 166, 167 |
| | | | | (SEQ. ID NO.:95) | | |
| HIV-1 | RT | 476-484 | ILKEPVHGV | | HLA-A*0201 | 168, 169 |
| | | | | (SEQ. ID NO.:96) | | |
| HIV-1 | nef | 190-198 | AFHHVAREL | | HLA-A*0201 | 170 |
| | | | | (SEQ. ID NO.:97) | | |
| HIV-1 | gp160 | 120-128 | KLTPLCVTL | | HLA-A*0201 | 171 |
| | | | | (SEQ. ID NO.:98) | | |
| HIV-1 | gp160 | 814-823 | SLLNATDIAV | | HLA-A*0201 | 171 |
| | | | | (SEQ. ID NO.:99) | | |
| HIV-1 | RT | 179-187 | VIYQYMDDL | | HLA-A*0201 | 172 |
| | | | | (SEQ. ID NO.:100) | | |
| HIV-1 | gagp17 | 77-85 | SLYNTVATL | | HLA-A*0201 | 173 |
| | | | | (SEQ. ID NO.:101) | | |
| HIV-1 | gp160 | 315-329 | RGPGRAFVTI | | HLA-A*0201 | 174 |
| | | | | (SEQ. ID NO.:102) | | |
| HIV-1 | gp41 | 768-778 | RLRDLLLIVTR | | HLA-A3 | 175. 178 |
| | | | | (SEQ. ID NO.:103) | | |
| HIV-1 | nef | 73-82 | QVPLRPMTYK | | HLA-A3 | 176 |
| | | | | (SEQ. ID NO.:104) | | |
| HIV-1 | gp120 | 36-45 | TVYYGVPVWK | | HLA-A3 | 177 |
| | | | | (SEQ. ID NO.:105) | | |
| HIV-1 | gagp17 | 20-29 | RLRPGGKKK | | HLA-A3 | 177 |
| | | | | (SEQ. ID NO.:106) | | |
| HIV-1 | gp120 | 38-46 | VYYGVPVWK | | HLA-A3 | 179 |
| | | | | (SEQ. ID NO.:107) | | |
| HIV-1 | nef | 74-82 | VPLRPMTYK | | HLA-a*1101 | 114 |
| | | | | (SEQ. ID NO.:108) | | |
| HIV-1 | gagp24 | 325-333 | AIFQSSMTK | | HLA-A*1101 | 114 |
| | | | | (SEQ. ID NO.:109) | | |
| HIV-1 | nef | 73-82 | QVPLRPMTYK | | HLA-A*1101 | 180 |
| | | | | (SEQ. ID NO.:104) | | |
| HIV-1 | nef | 83-94 | AAVDLSHFLKEK | | HLA-A*1101 | 159 |
| | | | | (SEQ. ID NO.:110) | | |
| HIV-1 | gagp24 | 349-359 | ACQGVGGPGGHK | | HLA-A*1101 | 181 |
| | | | | (SEQ. ID NO.:111) | | |
| HIV-1 | gagp24 | 203-212 | ETINEEAAEW | | HLA-A25 | 182 |
| | | | | (SEQ. ID NO.:112) | | |
| HIV-1 | nef | 128-137 | TPGPGVRYPL | | HLA-B7 | 159 |
| | | | | (SEQ. ID NO.:113) | | |
| HIV-1 | gagp17 | 24-31 | GGKKKYKL | | HLA-B8 | 183 |
| | | | | (SEQ. ID NO.:114) | | |
| HIV-1 | gp120 | 2-10 | RVKEKYQHL | | HLA-B8 | 181 |
| | | | | (SEQ. ID NO.:115) | | |
| HIV-1 | gagp24 | 298-306 | DRFYKTLRA | | HLA-B14 | 173 |
| | | | | (SEQ. ID NO.:116) | | |
| HIV-1 | NEF | 132-147 | GVRYPLTFGWCYKLVP | | HLA-B18 | 159 |
| | | | | (SEQ. ID NO.:117) | | |
| HIV-1 | gagp24 | 265-24 | KRWIILGLNK | | HLA-B*2705 | 184, 153 |
| | | | | (SEQ. ID NO.:118) | | |
| HIV-1 | nef | 190-198 | AFHHVAREL | | HLA-B*5201 | 170 |
| | | | | (SEQ. ID NO.:97) | | |
| EBV | EBNA-6 | 335-343 | KEHVIQNAF | | HLA-B44 | 121 |
| | | | | (SEQ. ID NO.: 119) | | |
| EBV | EBNA-6 | 130-139 | EENLLDFVRF | | HLA-B*4403 | 122 |
| | | | | (SEQ. ID NO.:120) | | |
| EBV | EBNA-2 | 42-51 | DTPLIPLTIF | | HLA-B51 | 121 |
| | | | | (SEQ. ID NO.:121) | | |
| EBV | EBNA-6 | 213-222 | QNGALATNTF | | HLA-B62 | 112 |
| | | | | (SEQ. ID NO.: 122) | | |
| EBV | EBNA-3 | 603-611 | RLRAEAGVK | | HLA-A3 | 123 |
| | | | | (SEQ. ID NO.:123) | | |
| HBV | sAg | 348-357 | GLSPTVWLSV | | HLA-A*0201 | 124 |
| | | | | (SEQ. ID NO.:124) | | |
| HBV | SAg | 335-343 | WLSLLVPFV | | HLA-A*0201 | 124 |
| | | | | (SEQ. ID NO.:125) | | |
| HBV | cAg | 18-27 | FLPSDFFPSV | | HLA-A*0201 | 125. 126, 127 |
| | | | | (SEQ. ID NO.:126) | | |
| HBV | cAg | 18-27 | FLPSDFFPSV | | HLA-A*0202 | 127 |
| | | | | (SEQ. ID NO.:126) | | |
| HBV | cAg | 18-27 | FLPSDFFPSV | | HLA-A*0205 | 127 |
| | | | | (SEQ. ID NO.:126) | | |
| HBV | cAg | 18-27 | FLPSDFFPSV | | HLA-A*0206 | 127 |
| | | | | (SEQ. ID NO.:126) | | |
| HBV | pol | 575-583 | FLLSLGIHL | | HLA-A*0201 | 128 |
| | | | | (SEQ. ID NO.:127) | | |
| HBV | pol | 816-824 | SLYADSPSV | | HLA-A*0201 | 128 |
| | | | | (SEQ. ID NO.:128) | | |
| HBV | pol | 455-463 | GLSRYVARL | | HLA-A*0201 | 128 |
| | | | | (SEQ. ID NO.:129) | | |
| HBV | env | 338-347 | LLVPFVQWFV | | HLA-A*0201 | 129 |
| | | | | (SEQ. ID NO.:130) | | |
| HBV | pol | 642-650 | ALMPLYACI | | HLA-A*0201 | 129 |
| | | | | (SEQ. ID NO.:131) | | |
| HBV | env | 378-387 | LLPIFFCLWV | | HLA-A*0201 | 129 |
| | | | | (SEQ. ID NO.:132) | | |
| HBV | pol | 538-546 | YMDDVVLGA | | HLA-A*0201 | 129 |
| | | | | (SEQ. ID NO.:133) | | |
| HBV | env | 250-258 | LLLCLIFLL | | HLA-A*0201 | 130 |
| | | | | (SEQ. ID NO.:134) | | |
| HBV | env | 260-269 | LLDYQGMLPV | | FILA-A*0201 | 130 |
| | | | | (SEQ. ID NO.:135) | | |
| HBV | env | 370-379 | SIVSPFIPLL | | HLA-A*0201 | 130 |
| | | | | (SEQ. ID NO.:136) | | |
| HBV | env | 183-191 | FLLTRILTI | | HLA-A*0201 | 130 |
| | | | | (SEQ. ID NO.:137) | | |
| HBV | cAg | 88-96 | YVNVNMGLK | | HLA-A*1101 | 131 |
| | | | | (SEQ. ID NO.:138) | | |
| HBV | cAg | 141-151 | STLPETTVVRR | | HLA-A*3101 | 132 |
| | | | | (SEQ. ID NO.:139) | | |
| HBV | cAg | 141-151 | STLPETTVVRR | | HLA-A*6801 | 132 |
| | | | | (SEQ. ID NO.:139) | | |
| HBV | cAg | 18-27 | FLPSDFFPSV | | HLA-A*6801 | 127 |
| | | | | (SEQ. ID NO.:126) | | |
| HBV | sAg | 28-39 | IPQSLDSWWTSL | | H2-L^{d} | 133 |
| | | | | (SEQ. ID NO.:140) | | |
| HBV | cAg | 93-100 | MGLKFRQL | | H2-K^{b} | 134 |
| | | | | (SEQ. ID NO.:141) | | |
| HBV | preS | 141-149 | STBXQSGXQ | | HLA-A*0201 | 135 |
| | | | | (SEQ. ID NO.: 142) | | |
| HCMV | gp B | 618-628 | FIAGNSAYEYV | | HLA-A*0201 | 124 |
| | | | | (SEQ. ID NO.:143) | | |
| HCMV | E1 | 978-989 | SDEEEAIVAYTL | | HLA-B18 | 136 |
| | | | | (SEQ. ID NO.:144) | | |
| HCMV | pp65 | 397-411 | DDVWTSGSDSDEELV | | HLA-b35 | 137 |
| | | | | (SEQ. ID NO.:145) | | |
| HCMV | pp65 | 123-131 | IPSINVHHY | | HLA-B*3501 | 136 |
| | | | | (SEQ. ID NO.:146) | | |
| HCMV | pp65 | 495-504 | NLVPMVATVO | | HLA-A*0201 | 137 |
| | | | | (SEQ. ID NO.:147) | | |
| HCMV | pp65 | 415-429 | RKTPRVTGGGAMAGA | | HLA-87 | 137 |
| | | | | (SEQ. ID NO.:148) | | |
| HCV | MP | 17-25 | DLMGYIPLV | | HLA-A*0201 | 138 |
| | | | | (SEQ. ID NO.:149) | | |
| HCV | MP | 63-72 | LLALLSCLTV | | HLA-A*0201 | 139 |
| | | | | (SEQ. ID NO.:150) | | |
| HCV | MP | 105-112 | ILHTPGCV | | HLA-A*0201 | 139 |
| | | | | (SEQ. ID NO.:151) | | |
| HCV | env E | 66-75 | QLRRHIDLLV | | HLA-A*0201 | 139 |
| | | | | (SEQ. ID NO.:152) | | |
| HCV | env E | 88-96 | DLCGSVFLV | | HLA-A*0201 | 139 |
| | | | | (SEQ. ID NO.:153) | | |
| HCV | env E | 172-180 | SMVGNWAKV | | HLA-A*0201 | 139 |
| | | | | (SEQ. ID NO.:154) | | |
| HCV | NS1 | 308-316 | HLHQNIVDV | | HLA-A*0201 | 139 |
| | | | | (SEQ. ID NO.:155) | | |
| HCV | NS1 | 340-348 | FLLLADARV | | HLA-A*0201 | 139 |
| | | | | (SEQ. ID NO.:156) | | |
| HCV | NS2 | 234-246 | GLRDLAVAVEPVV | | HLA-A*0201 | 139 |
| | | | | (SEQ. ID NO.:157) | | |
| HCV | NS1 | 18-28 | SLLAPGAKQNV | | HLA-A*0201 | 139 |
| | | | | (SEQ. ID NO.:158) | | |
| HCV | NS1 | 19-28 | LLAPGAKQNV | | HLA-A*0201 | 139 |
| | | | | (SEQ. ID NO.:159) | | |
| HCV | NS4 | 192-201 | LLFNILGGWV | | HLA-A*0201 | 129 |
| | | | | (SEQ. ID NO.:160) | | |
| HCV | NS3 | 579-587 | YLVAYQATV | | HLA-A*0201 | 129 |
| | | | | (SEQ. ID NO.:161) | | |
| HCV | core protein | 34-43 | YLLPRRGPRL | | HLA-A*0201 | 129 |
| | | | | (SEQ. ID NO.:162) | | |
| HCV | MP | 63-72 | LLALLSCLTI | | HLA-A*0201 | 129 |
| | | | | (SEQ. ID NO.:163) | | |
| HCV | NS4 | 174-182 | SLMAFTAAV | | HLA-A*0201 | 140 |
| | | | | (SEQ. ID NO.: 164) | | |
| HCV | NS3 | 67-75 | CINGVCWTV | | HLA-A*0201 | 140 |
| | | | | (SEQ. ID NO.: 165) | | |
| HCV | NS3 | 163-171 | LLCPAGHAV | | HLA-A*0201 | 141 |
| | | | | (SEQ. ID NO.: 166) | | |
| HCV | NS5 | 239-247 | ILDSFDPLV | | HLA-A*0201 | 141 |
| | | | | (SEQ. ID NO.:167) | | |
| HCV | NS4A | 236-244 | ILAGYGAGV | | HLA-A*0201 | 142 |
| | | | | (SEQ. ID NO.:168) | | |
| HCV | NS5 | 714-722 | GLQDCTMLV | | HLA-A*0201 | 142 |
| | | | | (SEQ. ID NO.: 169) | | |
| HCV | NS3 | 281-290 | TGAPVTYSTY | | HLA-A*0201 | 143 |
| | | | | (SEQ. ID NO.: 170) | | |
| HCV | NS4A | 149-157 | HMWNFISGI | | HLA-A*0201 | 144 |
| | | | | (SEQ. ID NO.:171) | | |
| HCV | NS5 | 575-583 | RVCEKMALY | | HLA-A*0201-A3 | 145 |
| | | | | (SEQ. ID NO.:172) | | |
| HCV | NS1 | 238-246 | TINYTIFK | | HLA-A*1101 | 145 |
| | | | | (SEQ. ID NO.:173) | | |
| HCV | NS2 | 109-116 | YISWCLWW | | HLA-A23 | 145 |
| | | | | (SEQ. ID NO.:174) | | |
| HCV | core protein | 40-48 | GPRLGVRAT | | HLA-B7 | 145 |
| | | | | (SEQ. ID NO.:175) | | |
| HIV-1 | gp120 | 380-388 | SFNCGGEFF | | HLA-Cw*0401 | 185 |
| | | | | (SEQ. ID NO.:176) | | |
| HIV-1 | RT | 206-214 | TEMEKEGKI | | H2-K^{k} | 186 |
| | | | | (SEQ. ID NO.:177) | | |
| HIV-1 | p17 | 18-26 | KIRLRPGGK | | HLA-A*0301 | 187 |
| | | | | (SEQ. ID NO.:178) | | |
| HIV-1 | p17 | 20-29 | RLRPGGKKKY | | HLA-A*0301 | 188 |
| | | | | (SEQ. ID NO.:179) | | |
| HIV-1 | RT | 325-333 | AIFQSSMTK | | HLA-A*0301 | 188 |
| | | | | (SEQ. ID NO.:180) | | |
| HIV-1 | p17 | 84-92 | TLYCVHQRI | | HLA-A11 | 188 |
| | | | | (SEQ. ID NO.:181) | | |
| HIV-1 | RT | 508-517 | IYQEPFKNLK | | HLA-A11 | 188 |
| | | | | (SEQ. ID NO.:182) | | |
| HIV-1 | p17 | 28-36 | KYKLKHIVW | | HLA-A24 | 188 |
| | | | | (SEQ. ID NO.:183) | | |
| HIV-1 | gp120 | 53-62 | LFCASDAKAY | | HLA-A24 | 189 |
| | | | | (SEQ. ID NO.:184) | | |
| HIV-1 | gagp24 | 145-155 | QAISPRTLNAW | | HLA-A25 | 188 |
| | | | | (SEQ. ID NO.:185) | | |
| HIV-1 | gagp24 | 167-175 | EVIPMFSAL | | HLA-A26 | 188 |
| | | | | (SEQ. ID NO.:186) | | |
| HIV-1 | RT | 593-603 | ETFYVDGAANR | | HLA-A26 | 188 |
| | | | | (SEQ. ID NO.:187) | | |
| HIV-1 | gp41 | 775-785 | RLRDLLLIVTR | | HLA-A31 | 190 |
| | | | | (SEQ. ID NO.:188) | | |
| HIV-1 | RT | 559-568 | PIQKETWETW | | HLA-A32 | 187 |
| | | | | (SEQ. ID NO.:189) | | |
| HIV-1 | gp120 | 419-427 | RIKQIINMW | | HLA-A32 | 187 |
| | | | | (SEQ. ID NO.:190) | | |
| HIV-1 | RT | 71-79 | ITLWQRPLV | | HLA-A*6802 | 188 |
| | | | | (SEQ. ID NO.:191) | | |
| HIV-1 | RT | 85-93 | DTVLEEMNL | | HLA-A*6802 | 188 |
| | | | | (SEQ. ID NO.:192) | | |
| HIV-1 | RT | 71-79 | ITLWQRPLV | | HLA-A*7401 | 188 |
| | | | | (SEQ. ID NO.:193) | | |
| HIV-1 | gag p24 | 148-156 | SPRTLNAWV | | HLA-B7 | 188 |
| | | | | (SEQ. ID NO.:194) | | |
| HIV-1 | gagp24 | 179-187 | ATPQDLNTM | | HLA-B7 | 188 |
| | | | | (SEQ. ID NO.:195) | | |
| HIV-1 | gp120 | 303-312 | RPNNNTRKSI | | HLA-B7 | 188 |
| | | | | (SEQ. ID NO.:196) | | |
| HIV-1 | gp41 | 843-851 | IPRRIRQGL | | HLA-B7 | 188 |
| | | | | (SEQ. ID NO.:197) | | |
| HIV-1 | p17 | 74-82 | ELRSLYNTV | | HLA-B8 | 188 |
| | | | | (SEQ. ID NO.:198) | | |
| HIV-1 | nef | 13-20 | WPTVRERM | | HLA-B8 | 188 |
| | | | | (SEQ. ID NO.:199) | | |
| HIV-1 | nef | 90-97 | FLKEKGGL | | HLA-B8 | 188 |
| | | | | (SEQ. ID NO.:200) | | |
| HIV-1 | gag p24 | 183-191 | DLNTMLNTV | | HLA-B14 | 191 |
| | | | | (SEQ. ID NO.:568) | | |
| HIV-1 | P17 | 18-27 | KIRLRPGGKK | | HLA-B27 | 188 |
| | | | | (SEQ. ID NO.:201) | | |
| HIV-1 | p17 | 19-27 | IRLRPGGKK | | HLA-B27 | 188 |
| | | | | (SEQ. ID NO.:202) | | |
| HIV-1 | gp41 | 791-799 | GRRGWEALKY | | HLA-B27 | 188 |
| | | | | (SEQ. ID NO.:203) | | |
| HIV-1 | nef | 73-82 | QVPLRPMTYK | | HLA-B27 | 188 |
| | | | | (SEQ. ID NO.:204) | | |
| HIV-1 | GP41 | 590-597 | RYLKDQQL | | HLA-B27 | 192 |
| | | | | (SEQ. ID NO.:205) | | |
| HIV-1 | nef | 105-114 | RRQDILDLWI | | HLA-B*2705 | 188 |
| | | | | (SEQ. ID NO.:206) | | |
| HIV-1 | nef | 134-141 | RYPLTFGW | | HLA-B*2705 | 188 |
| | | | | (SEQ. ID NO.:207) | | |
| HIV-1 | p17 | 36-44 | WASRELERF | | HLA-B35 | 188 |
| | | | | (SEQ. ID NO.:208) | | |
| HIV-1 | GAG P24 | 262-270 | TVLDVGDAY | | HLA-B35 | 188 |
| | | | | (SEQ. ID NO.:209) | | |
| HIV-1 | gp120 | 42-52 | VPVWKEATTTL | | HLA-B35 | 188 |
| | | | | (SEQ. ID NO.:210) | | |
| HIV-1 | P17 | 36-44 | NSSKVSQNY | | HLA-B35 | 193 |
| | | | | (SEQ. ID NO.:221) | | |
| HIV-1 | gag p24 | 254-262 | PPIPVGDIY | | HLA-B35 | 193 |
| | | | | (SEQ. ID NO.:212) | | |
| HIV-1 | RT | 342-350 | HPDIVIYQY | | HLA-B35 | 193 |
| | | | | (SEQ. ID NO.:213) | | |
| HIV-1 | gp41 | 611-619 | TAVPWNASW | | HLA-B35 | 194 |
| | | | | (SEQ. ID NO.:214) | | |
| HIV-1 | gag | 245-253 | NPVPVGNIY | | HLA-B35 | 193 |
| | | | | (SEQ. ID NO.:215) | | |
| HIV-1 | nef | 120-128 | YFPDWQNYT | | HLA-B37 | 188 |
| | | | | (SEQ. ID NO.:216) | | |
| HIV-1 | gag p24 | 193-201 | GHQAAMQML | | HLA-B42 | 188 |
| | | | | (SEQ. ID NO.:217) | | |
| HIV-1 | p17 | 20-29 | RLRPGGKKKY | | HLA-B42 | 188 |
| | | | | (SEQ. ID NO.:218) | | |
| HIV-1 | RT | 438-446 | YPGIKVRQL | | HLA-B42 | 188 |
| | | | | (SEQ. ID NO.:219) | | |
| HIV-1 | RT | 591-600 | GAETFYVDGA | | HLA-B45 | 188 |
| | | | | (SEQ. ID NO.:220) | | |
| HIV-1 | gag p24 | 325-333 | NANPDCKTI | | HLA-B51 | 188 |
| | | | | (SEQ. ID NO.:221) | | |
| HIV-1 | gag p24 | 275-282 | RMYSPTSI | | HLA-B52 | 188 |
| | | | | (SEQ. ID NO.:222) | | |
| HIV-1 | gp120 | 42-51 | VPVWKEATTT | | HLA-B*5501 | 192 |
| | | | | (SEQ. ID NO.:223) | | |
| HIV-1 | gag p24 | 147-155 | ISPRTLNAW | | HLA-B57 | 188 |
| | | | | (SEQ. ID NO.:224) | | |
| HIV-1 | gag p24 | 240-249 | TSTLQEQIGW | | HLA-B57 | 188 |
| | | | | (SEQ. ID NO.:225) | | |
| HIV-1 | gag p24 | 162-172 | KAFSPEVIPMF | | HLA-B57 | 188 |
| | | | | (SEQ. ID NO.:226) | | |
| HIV-1 | gag p24 | 311-319 | QASQEVKNW | | HLA-B57 | 188 |
| | | | | (SEQ. ID NO.:227) | | |
| HIV-1 | gag p24 | 311-319 | QASQDVKNW | | HLA-B57 | 188 |
| | | | | (SEQ. ID NO.:228) | | |
| HIV-1 | nef | 116-125 | HTQGYFPDWQ | | HLA-B57 | 188 |
| | | | | (SEQ. ID NO.:229) | | |
| HIV-1 | nef | 120-128 | YFPDWQNYT | | HLA-B57 | 188 |
| | | | | (SEQ. ID NO.:230) | | |
| HIV-1 | gag p24 | 240-249 | TSTLQEQIGW | | HLA-B58 | 188 |
| | | | | (SEQ. ID NO.:231) | | |
| HIV-1 | p17 | 20-29 | RLRPGGKKKY | | HLA-B62 | 188 |
| | | | | (SEQ. ID NO.:232) | | |
| HIV-1 | p24 | 268-277 | LGLNKIVRMY | | HLA-B62 | 188 |
| | | | | (SEQ. ID NO.:233) | | |
| HIV-1 | RT | 415-426 | LVGKLNWASQIY | | HLA-B62 | 188 |
| | | | | (SEQ. ID NO.:234) | | |
| HIV-1 | RT | 476-485 | ILKEPVHGVY | | HLA-B62 | 188 |
| | | | | (SEQ. ID NO.:235) | | |
| HIV-1 | nef | 117-127 | TQGYFPDWQNY | | HLA-B62 | 188 |
| | | | | (SEQ. ID NO.:236) | | |
| HIV-1 | nef | 84-91 | AVDLSHFL | | HLA-B62 | 188 |
| | | | | (SEQ. ID NO.:237) | | |
| HIV-1 | gag p24 | 168-175 | VIPMFSAL | | HLA-Cw*0102 | 188 |
| | | | | (SEQ. ID NO.:238) | | |
| HIV-1 | gp120 | 376-384 | FNCGGEFFY | | HLA-A29 | 196 |
| | | | | (SEQ. ID NO.:239) | | |
| HIV-1 | gp 120 | 375-383 | SFNCGGEFF | | HLA-B15 | 196 |
| | | | | (SEQ. ID NO.:240) | | |
| HIV-1 | nef | 136-145 | PLTFGWCYKL | | HLA-A*0201 | 197 |
| | | | | (SEQ. ID NO.:241) | | |
| HIV-1 | nef | 180-189 | VLEWRFDSRL | | HLA-A*0201 | 197 |
| | | | | (SEQ. ID NO.:242) | | |
| HIV- 1 | nef | 68-77 | FPVTPQVPLR | | HLA-B7 | 197 |
| | | | | (SEQ. ID NO.:243) | | |
| HIV-1 | nef | 128-137 | TPGPGVRYPL | | HLA-B7 | 197 |
| | | | | (SEQ. ID NO.:244) | | |
| HIV-1 | gag p24 | 308-316 | QASQEVKNW | | HLA-Cw*0401 | 521 |
| | | | | (SEQ. ID NO.:245) | | |
| HIV-1 IIIB | RT | 273-282 | VPLDEDFRKY | | HLA-B35 | 181 |
| | | | | (SEQ. ID NO.:246) | | |
| HIV-1 IIIB | RT | 25-33 | NPDIVIYQY | | HLA-B35 | 181 |
| | | | | (SEQ. ID NO.:247) | | |
| HIV-1 IIIB | gp41 | 557-565 | RAIEAQAHL | | HLA-B51 | 181 |
| | | | | (SEQ. ID NO.:248) | | |
| HIV-1 IIIB | RT | 231-238 | TAFTIPSI | | HLA-B51 | 181 |
| | | | | (SEQ. ID NO.:249) | | |
| HIV-1 IIIB | p24 | 215-223 | VHPVHAGPIA | | HLA-B*5501 | 181 |
| | | | | (SEQ. ID NO.:250) | | |
| HIV-1 IIIB | gp120 | 156-165 | NCSFNISTSI | | HLA-Cw8 | 181 |
| | | | | (SEQ. ID NO.:251) | | |
| HIV-1 IIIB | gp120 | 241-249 | CTNVSTVQC | | HLA-Cw8 | 181 |
| | | | | (SEQ. ID NO.:252) | | |
| HIV-I_{5F2} | gp120 | 312-320 | IGPGRAFHT | | H2-D^{d} | 198 |
| | | | | (SEQ. ID NO.:253) | | |
| HIV-I_{5F2} | pol | 25-33 | NPDIVIYQY | | HLA-B*3501 | 199 |
| | | | | (SEQ. ID NO.:254) | | |
| HIV-1_{5F2} | pol | 432-441 | EPIVGAETFY | | HLA-B*3501 | 199 |
| | | | | (SEQ. ID NO.:255) | | |
| HIV-1_{5F2} | pol | 432-440 | EPIVGAETF | | HLA-B*3501 | 199 |
| | | | | (SEQ. ID NO.:256) | | |
| HIV-1_{5F2} | pol | 6-14 | SPAIFQSSM | | HLA-B*3501 | 199 |
| | | | | (SEQ. ID NO.:257) | | |
| HIV-1_{5F2} | pol | 59-68 | VPLDKDFRKY | | HLA-B*3501 | 199 |
| | | | | (SEQ. ID NO.:258) | | |
| HIV-1_{5F2} | pol | 6-14 | IPLTEEAEL | | HLA-B*3501 | 199 |
| | | | | (SEQ. ID NO.:259) | | |
| HIV-1_{5F2} | nef | 69-79 | RPQVPLRPMTY | | HLA-B*3501 | 199 |
| | | | | (SEQ. ID NO.:260) | | |
| HIV-1_{5F2} | nef | 66-74 | FPVRPQVPL | | HLA-B*3501 | 199 |
| | | | | (SEQ. ID NO.:261) | | |
| HIV-1_{5F2} | env | 10-18 | DPNPQEVVL | | HLA-B*3501 | 199 |
| | | | | (SEQ. ID NO.:262) | | |
| HIV-1_{5F2} | env | 7-15 | RPIVSTQLL | | HLA-B*3501 | 199 |
| | | | | (SEQ. ID NO.:263) | | |
| HIV-1_{5F2} | pol | 6-14 | IPLTEEAEL | | HLA-B51 | 199 |
| | | | | (SEQ. ID NO.:264) | | |
| HIV-1_{5F2} | env | 10-18 | DPNPQEVVL | | HLA-B51 | 199 |
| | | | | (SEQ. ID NO.:265) | | |
| HIV-1_{5F2} | gagp24 | 199-207 | AMQMLKETI | | H2-K^{d} | 198 |
| | | | | (SEQ. ID NO.:266) | | |
| HIV-2 | gagp24 | 182-190 | TPYDINQML | | HLA-B*5301 | 200 |
| | | | | (SEQ. ID NO.:267) | | |
| HIV-2 | gag | 260-269 | RRWIQLGLQKV | | HLA-B*2703 | 188 |
| | | | | (SEQ. ID NO.:268) | | |
| HIV-1_{5F2} | gp41 | 593-607 | GIWGCSGKLICTTAV | | HLA-B17 | 201 |
| | | | | (SEQ. ID NO.:269) | | |
| HIV-1_{5F2} | gp41 | 753-767 | ALIWEDLRSLCLFSY | | HLA-B22 | 201 |
| | | | | (SEQ. ID NO.:270) | | |
| HPV 6b | E7 | 21-30 | GLHCYEQLV | | HLA-A*0201 | 202 |
| | | | | (SEQ. ID NO.:271) | | |
| HPV 6b | E7 | 47-55 | PLKQHFQIV | | HLA-A*0201 | 202 |
| | | | | (SEQ. ID NO.:272) | | |
| HPV11 | E7 | 4-12 | RLVTLKDIV | | HLA-A*0201 | 202 |
| | | | | (SEQ. ID NO.:273) | | |
| HPV16 | E7 | 86-94 | TLGIVCPIC | | HLA-A*0201 | 129 |
| | | | | (SEQ. ID NO.:274) | | |
| HPV16 | E7 | 85-93 | GTLGIVCPI | | HLA-A*0201 | 129 |
| | | | | (SEQ. ID NO.:275) | | |
| HPV16 | E7 | 12-20 | MLDLQPETT | | HLA-A*0201 | 129 |
| | | | | (SEQ. ID NO.:276) | | |
| HPV16 | E7 | 11-20 | YMLDLQPETT | | HLA-A*0201 | 203 |
| | | | | (SEQ. ID NO.:277) | | |
| HPV16 | E6 | 15-22 | RPRKLPQL | | HLA-B7 | 204 |
| | | | | (SEQ. ID NO.:278) | | |
| HPV16e6 | 15-22 | 49-57 | RAHYNIVTF | | HW-D^{b} | 205 |
| | | | | (SEQ. ID NO.:279) | | |
| HSV | gp B | 498-505 | SSIEFARL | | H2-K^{b} | 206 |
| | | | | (SEQ. ID NO.:280) | | |
| HSV-1 | gp C | 480-488 | GIGIGVLAA | | HLA-A*0201 | 104 |
| | | | | (SEQ. ID NO.:281) | | |
| HSV-1 | ICP27 | 448-456 | DYATLGVGV | | H2-K^{d} | 207 |
| | | | | (SEQ. ID NO.:282) | | |
| HSV-1 | ICP27 | 322-332 | LYRTFAGNPRA | | H2-K^{d} | 207 |
| | | | | (SEQ. ID NO.:283) | | |
| HSV-1 | UL39 | 822-829 | QTFDFGRL | | H2-K^{b} | 208 |
| | | | | (SEQ. ID NO.:284) | | |
| HSV-2 | gp C | 446-454 | GAGIGVAVL | | HLA-A*0201 | 104 |
| | | | | (SEQ. ID NO.:285) | | |
| HTLV-1 | TAX | 11-19 | LLFGYPVYV | | HLA-A*0201 | 209 |
| | | | | (SEQ. ID NO.:286) | | |
| Influenza | MP | 58-66 | GILGFVFTL | | HLA-A*0201 | 68, 169, 209, |
| | | | | (SEQ. ID NO.:287) | | 210, 211 |
| Influenza | MP | 59-68 | ILGFVFTLTV | | HLA-A*0201 | 168, 212, 213 |
| | | | | (SEQ. ID NO.:288) | | |
| Influenza | NP | 265-273 | ILRGSVAHK | | HLA-A3 | 214 |
| | | | | (SEQ. ID NO.:289) | | |
| Influenza | NP | 91-99 | KTGGPIYKR | | HLA-A*6801 | 215,216 |
| | | | | (SEQ. ID NO.:290) | | |
| Influenza | NP | 380-388 | ELRSRYWAI | | HLA-B8 | 217 |
| | | | | (SEQ. ID NO.:291) | | |
| Influenza | NP | 381-388 | LRSRYWAI | | HLA-B*2702 | 218 |
| | | | | (SEQ. ID NO.:292) | | |
| Influenza | NP | 339-347 | EDLRVLSFI | | HLA-B*3701 | 219 |
| | | | | (SEQ. ID NO.:293) | | |
| Influenza | NS1 | 158-166 | GEISPLPSL | | HLA-B44 | 220 |
| | | | | (SEQ. ID NO.:294) | | |
| Influenza | NP | 338-346 | FEDLRVLSF | | HLA-B44 | 220 |
| | | | | (SEQ. ID NO.:295) | | |
| Influenza | NS1 | 158-166 | GEISPLPSL | | HLA-B*4402 | 220 |
| | | | | (SEQ. ID NO.:294) | | |
| Influenza | NP | 338-346 | FEDLRVLSF | | HLA-B*4402 | 220 |
| | | | | (SEQ. ID NO.:295) | | |
| Influenza | PB1 | 591-599 | VSDGGPNLY | | HLA-A1 | 214, 29 |
| | | | | (SEQ. ID NO.:296) | | |
| Influenza A | NP | 44-52 | CTELKLSDY | | HLA-A1 | 29 |
| | | | | (SEQ. ID NO.:297) | | |
| Influenza | NS1 | 122-130 | AIMDKNIIL | | HLA-A*0201 | 221 |
| | | | | (SEQ. ID NO.:298) | | |
| Influenza A | NS1 | 123-132 | IMDKNIILKA | | HLA-A*0201 | 221 |
| | | | | (SEQ. ID NO.:299) | | |
| Influenza A | NP | 383-391 | SRYWAIRTR | | HLA-B*2705 | 160, 184 |
| | | | | (SEQ. ID NO.:300) | | |
| Influenza A | NP | 147-155 | TYQRTRALV | | H2-K^{d} | 222,223 |
| | | | | (SEQ. ID NO.:301) | | |
| Influenza A | HA | 210-219 | TYVSVSTSTL | | H2-K^{d} | 224,225 |
| | | | | (SEQ. ID NO.:302) | | |
| Influenza A | HA | 518-526 | IYSTVASSL | | H2-K^{d} | 224 |
| | | | | (SEQ. ID NO.:303) | | |
| Influenza A | HA | 259-266 | FEANGNLI | | H2-K^{k} | 226, 227, 228 |
| | | | | (SEQ. ID NO.:304) | | |
| Influenza A | HA | 10-18 | IEGGWTGMI | | H2-K^{k} | 226, 227, 228 |
| | | | | (SEQ. ID NO.:305) | | |
| Influenza A | NP | 50-57 | SDYEGRLI | | H2-K^{k} | 229, 230 |
| | | | | (SEQ. ID NO.:306) | | |
| Influenza a | NS1 | 152-160 | EEGAIVGEI | | H2-K^{k} | 231 |
| | | | | (SEQ. ID NO.:307) | | |
| Influenza A34 | NP | 366-374 | ASNENMETM | | H2-D^{b} | 168, 222, 219 |
| | | | | (SEQ. ID NO.:308) | | |
| Influenza A68 | NP | 366-374 | ASNENMDAM | | H2-D^{b} | 232 |
| | | | | (SEQ. ID NO.:309) | | |
| Influenza B | NP | 85-94 | KLGEFYNQMM | | HLA-A*0201 | 233 |
| | | | | (SEQ. ID NO.:310) | | |
| Influenza B | NP | 85-94 | KAGEFYNQMM | | HLA-A*0201 | 234 |
| | | | | (SEQ. ID NO.:311) | | |
| Influenza JAP | HA | 204-212 | LYQNVGTYV | | H2-K^{d} | 235 |
| | | | | (SEQ. ID NO.:312) | | |
| Influenza JAP | HA | 210-219 | TYVSVGTSTL | | H2-K^{d} | 225 |
| | | | | (SEQ. ID NO.:313) | | |
| Influenza JAP | HA | 523-531 | VYQILAIYA | | H2-K^{d} | 235 |
| | | | | (SEQ. ID NO.:314) | | |
| Influenza JAP | HA | 529-537 | IYATVAGSL | | H2-K^{d} | 235 |
| | | | | (SEQ. ID NO.:315) | | |
| Influenza JAP | HA | 210-219 | TYVSVGTSTI (L>I) | | H2-K^{d} | 236 |
| | | | | (SEQ. ID NO.:316) | | |
| Influenza JAP | HA | 255-262 | FESTGNLI | | H2-K^{k} | 237 |
| | | | | (SEQ. ID NO.:317) | | |
| JHMV | cAg | 318-326 | APTAGAFFF | | H2-L^{d} | 238 |
| | | | | (SEQ. ID NO.:318) | | |
| LCMV | NP | 118-126 | RPQASGVYM | | H2-L^{d} | 239, 240 |
| | | | | (SEQ. ID NO.:319) | | |
| LCMV | NP | 396-404 | FQPQNGQFI | | H2-D^{b} | 241 |
| | | | | (SEQ. ID NO.:320) | | |
| LCMV | GP | 276-286 | SGVENPGGYCL | | H2-D^{b} | 242 |
| | | | | (SEQ. ID NO.:321) | | |
| LCMV | GP | 33-42 | KAVYNFATCG | | H2-D^{b} | 243, 244 |
| | | | | (SEQ. ID NO.:322) | | |
| MCMV | pp89 | 168-176 | YPHFMPTNL | | H2-L^{d} | 245 |
| | | | | (SEQ. ID NO.:323) | | |
| MHV | spike protein | 510-518 | CLSWNGPHL | | H2-D^{b} | 248 |
| | | | | (SEQ. ID NO.:324) | | |
| MMTV | env gp 36 | 474-482 | SFAVATTAL | | H2-K^{d} | 246 |
| | | | | (SEQ. ID NO.:325) | | |
| MMTV | gag p27 | 425-433 | SYETFISRL | | H2-K^{d} | 246 |
| | | | | (SEQ. ID NO.:326) | | |
| MMTV | env gp73 | 544-551 | ANYDFICV | | H2-K^{b} | 247 |
| | | | | (SEQ. ID NO.:327) | | |
| MuLV | env p15E | 574-581 | KSPWFTTL | | H2-K^{b} | 249,250 |
| | | | | (SEQ. ID NO.:328) | | |
| MuLV | env gp70 | 189-196 | SSWDFITV | | H2-K^{b} | 251, Sijts et al. |
| | | | | (SEQ. ID NO.:329) | | submitted |
| MuLV | gag 75K | 75-83 | CCLCLTVFL | | H2-D^{b} | 252 |
| | | | | (SEQ. ID NO.:330) | | |
| MuLV | env gp70 | 423-431 | SPSYVYHQF | | H2-L^{d} | 253 |
| | | | | (SEQ. ID NO.:331) | | |
| MV | F protein | 437-447 | SRRYPDAVYLH | | HLA-B*27 | 254 |
| | | | | (SEQ. ID NO.:332) | | |
| MV | F protein | 438-446 | RRYPDAVYL | | HLA-B*2705 | 255 |
| | | | | (SEQ. ID NO.:333) | | |
| MV | NP | 281-289 | YPALGLHEF | | H2-L^{d} | 256 |
| | | | | (SEQ. ID NO.:334) | | |
| MV | HA | 343-351 | DPVIDRLYL | | H2-L^{d} | 257 |
| | | | | (SEQ. ID NO.:335) | | |
| MV | HA | 544-552 | SPGRSFSYF | | H2-L^{d} | 257 |
| | | | | (SEQ. ID NO.:336) | | |
| Poliovirus | VP1 | 111-118 | TYKDTVQL | | H2-k^{d} | 258 |
| | | | | (SEQ. ID NO.:337) | | |
| Poliovirus | VP1 | 208-217 | FYDGFSKVPL | | H2-K^{d} | 258 |
| | | | | (SEQ. ID NO.:338) | | |
| Pseudorabies | G111 | 455-463 | IAGIGILAI | | HLA-A*0201 | 104 |
| virus gp | | | | (SEQ. ID NO.:339) | | |
| Rabiesvirus | NS | 197-205 | VEAEIAHQI | | H2-K^{k} | 227, 227 |
| | | | | (SEQ. ID NO.:340) | | |
| Rotavirus | VP7 | 33-40 | IIYRFLLI | | H2-K^{b} | 259 |
| | | | | (SEQ. ID NO.:341) | | |
| Rotavirus | VP6 | 376-384 | VGPVFPPGM | | H2-K^{b} | 260 |
| | | | | (SEQ. ID NO.:342) | | |
| Rotavirus | VP3 | 585-593 | YSGYIFRDL | | H2-K^{b} | 260 |
| | | | | (SEQ. ID NO.:343) | | |
| RSV | M2 | 82-90 | SYIGSINNI | | H2-K^{d} | 261 |
| | | | | (SEQ. ID NO.:344) | | |
| SIV | gagp11C | 179-190 | EGCTPYDINQML | | Mamu-A*01 | 266 |
| | | | | (SEQ. ID NO.:345) | | |
| SV | NP | 324-332 | FAPGNYPAL | | H2-D^{b} | 262 |
| | | | | (SEQ. ID NO.:346) | | |
| SV | NP | 324-332 | FAPGNYPAL | | H2-K^{b} | 263, 264, 265 |
| | | | | (SEQ. ID NO.:346) | | |
| SV40 | T | 404-411 | VVYDFLKC | | H2-K^{b} | 267 |
| | | | | (SEQ. ID NO.:347) | | |
| SV40 | T | 206-215 | SAINNYAQKL | | H2-D^{b} | 268,269 |
| | | | | (SEQ. ID NO.:348) | | |
| SV40 | T | 223-231 | CKGVNKEYL | | H2-D^{b} | 268, 269 |
| | | | | (SEQ. ID NO.:349) | | |
| SV40 | T | 489-497 | QGINNLDNL | | H2-D^{b} | 268, 269 |
| | | | | (SEQ. ID NO.:350) | | |
| SV40 | T | 492-500 | NNLDNLRDY(L) | | H2-D^{b} | 270 |
| | | (501) | | (SEQ. ID NO.:351) | | |
| SV40 | T | 560-568 | SEFLLEKRI | | H2-K^{k} | 271 |
| | | | | (SEQ. ID NO.:352) | | |
| VSV | NP | 52-59 | RGYVYQGL | | H2-K^{b} | 272 |
| | | | | (SEQ. ID NO.:353) | | |

Table II sets forth antigens identified from various protein sources. The Table is extracted from Table 4.2 in the Rammensee book with the references in Table II being the same as the references in the Rammensee Table 4.2.

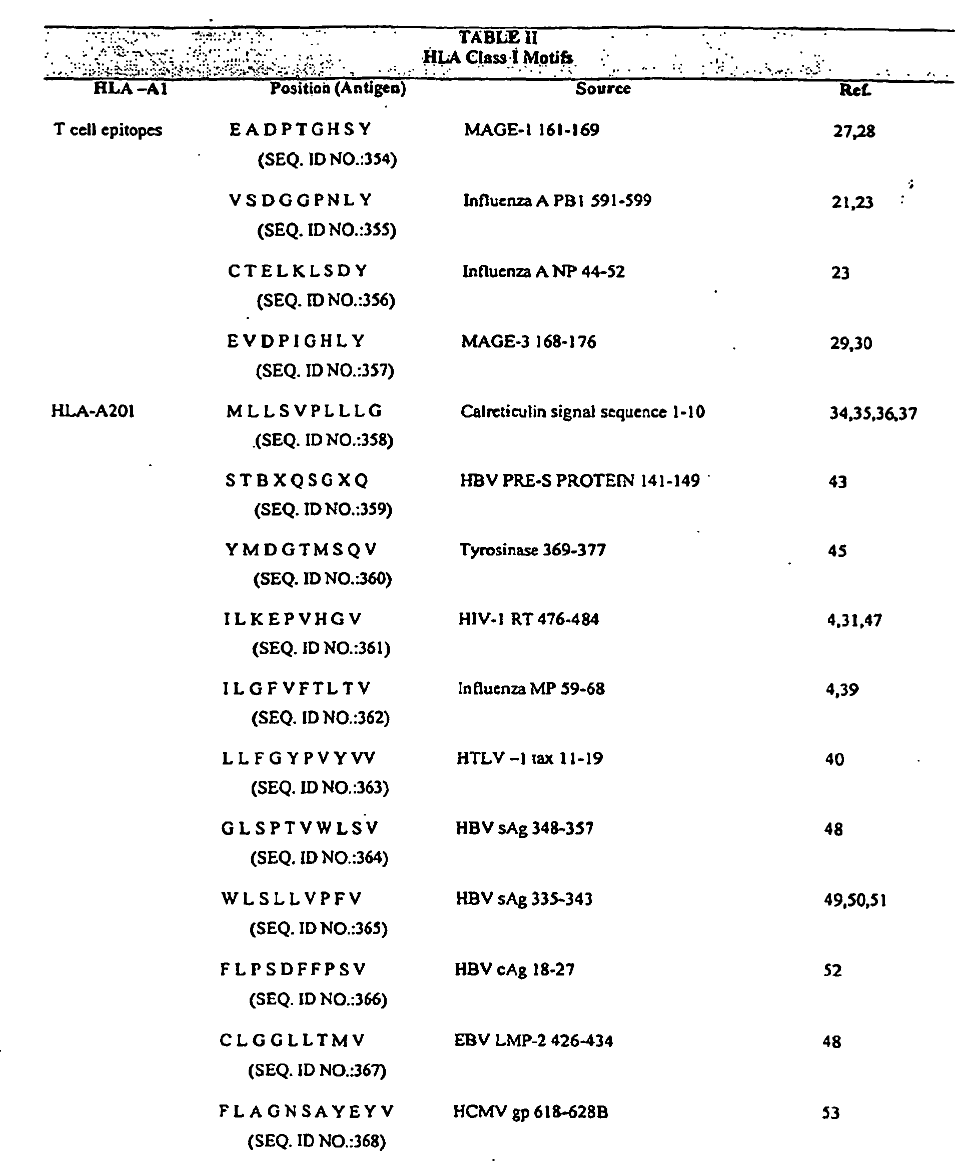

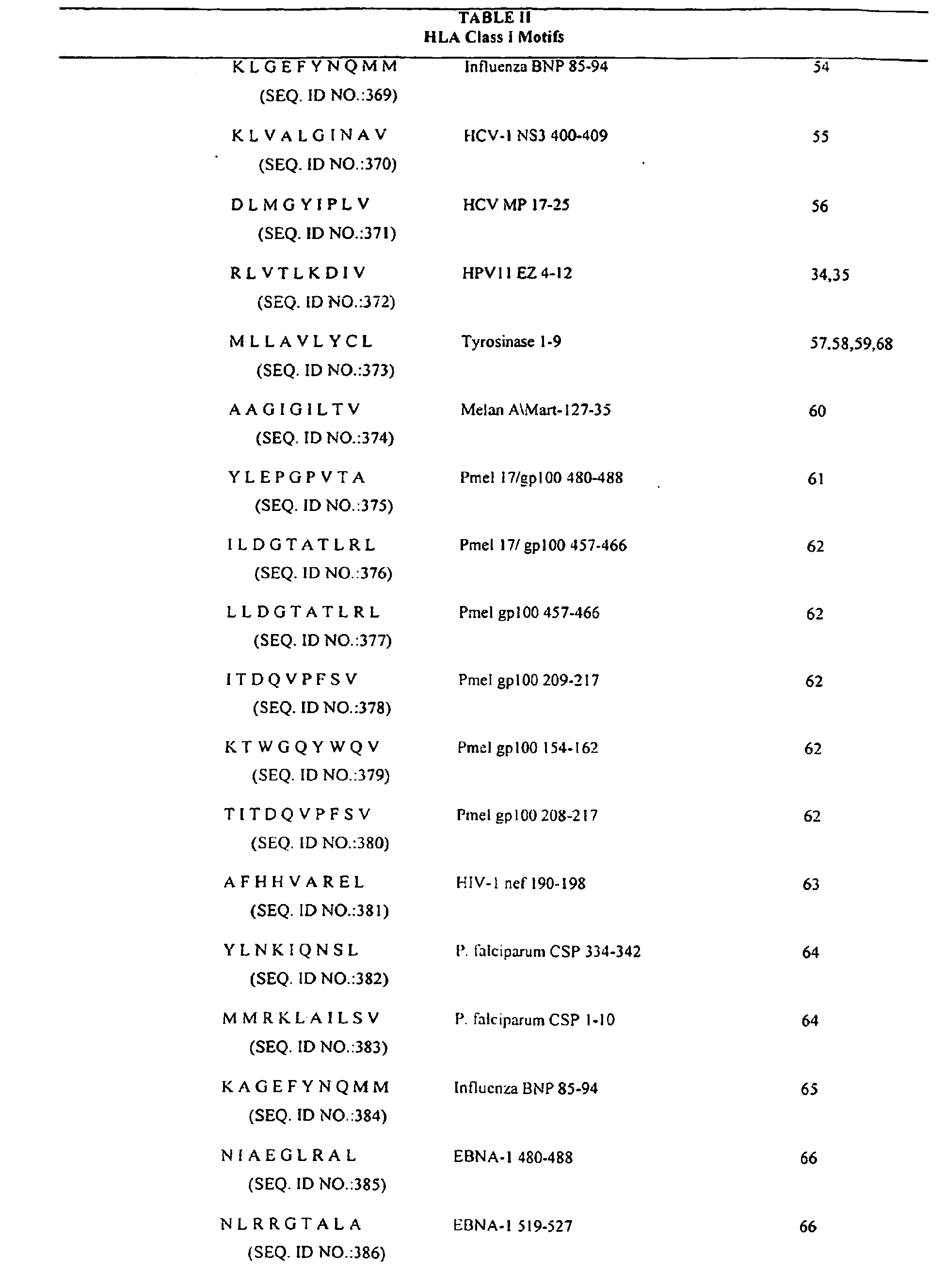

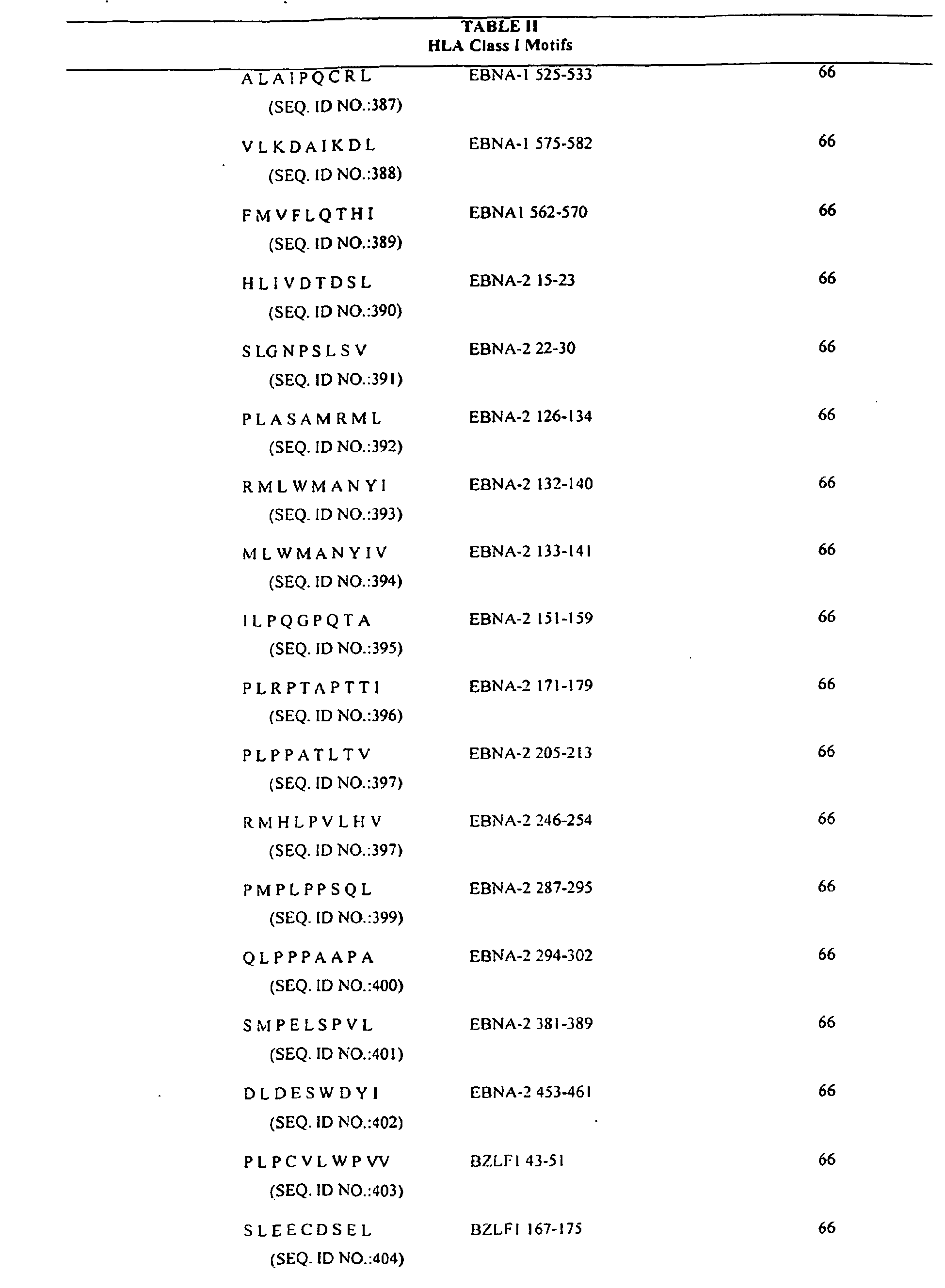

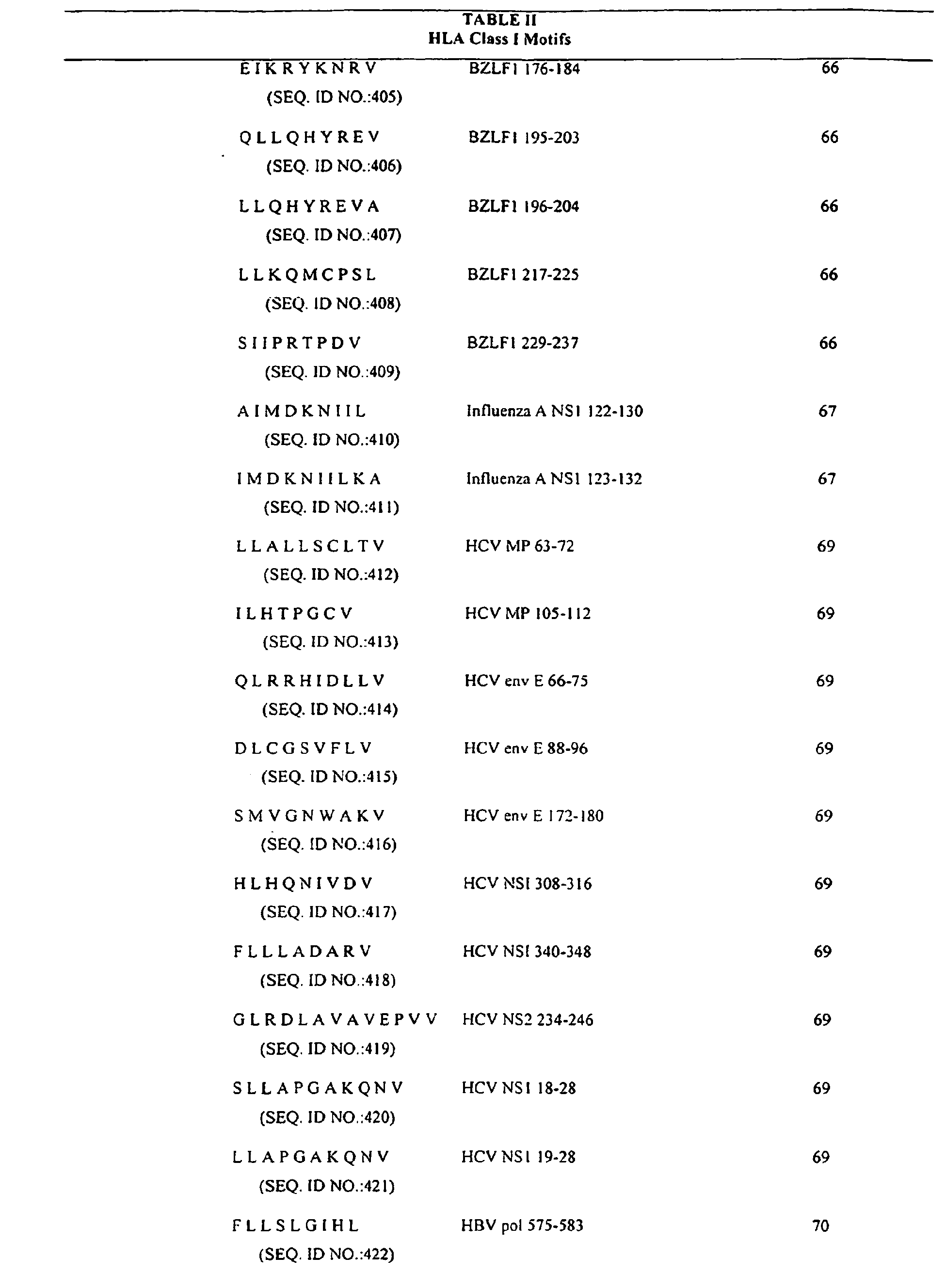

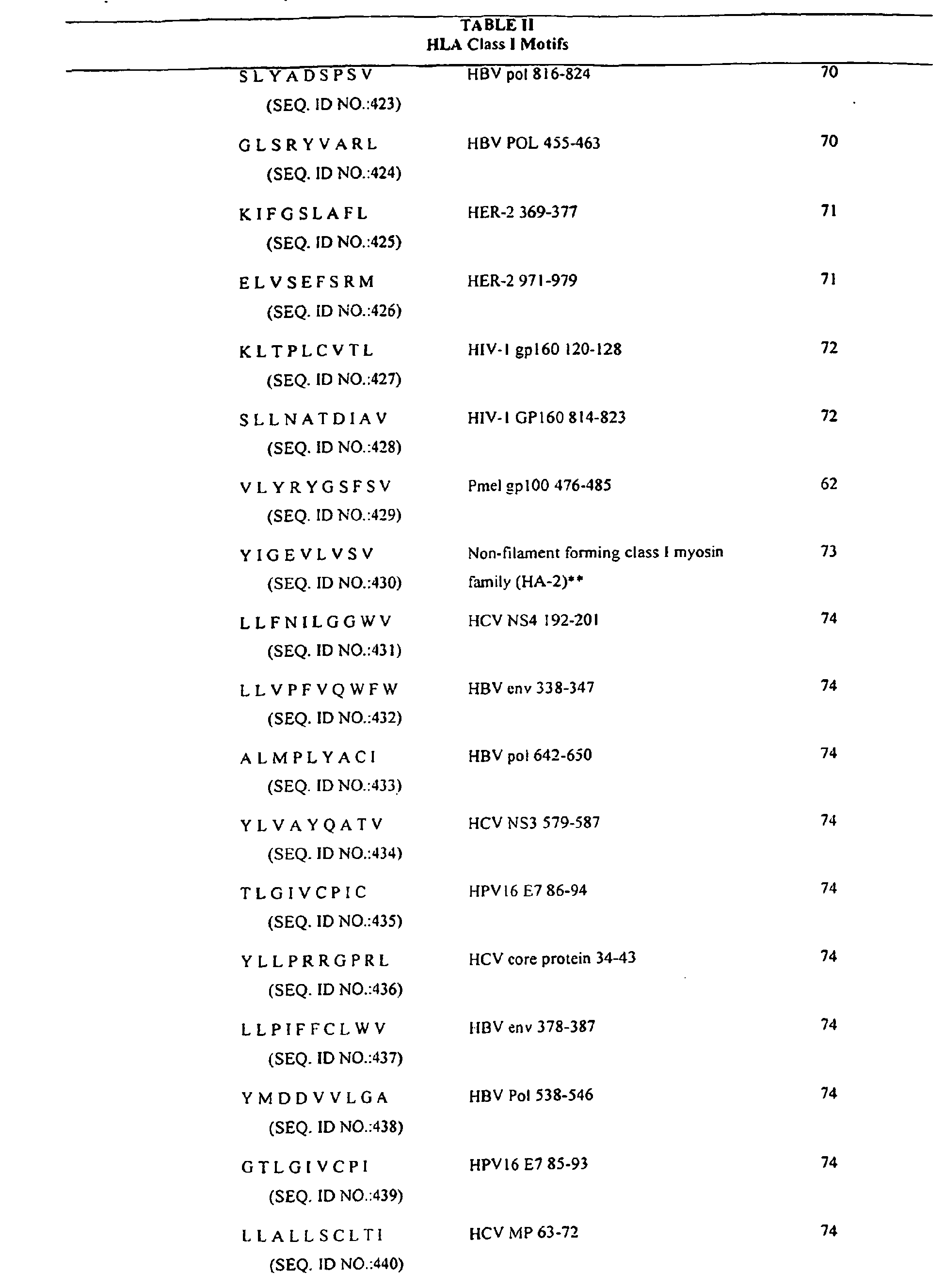

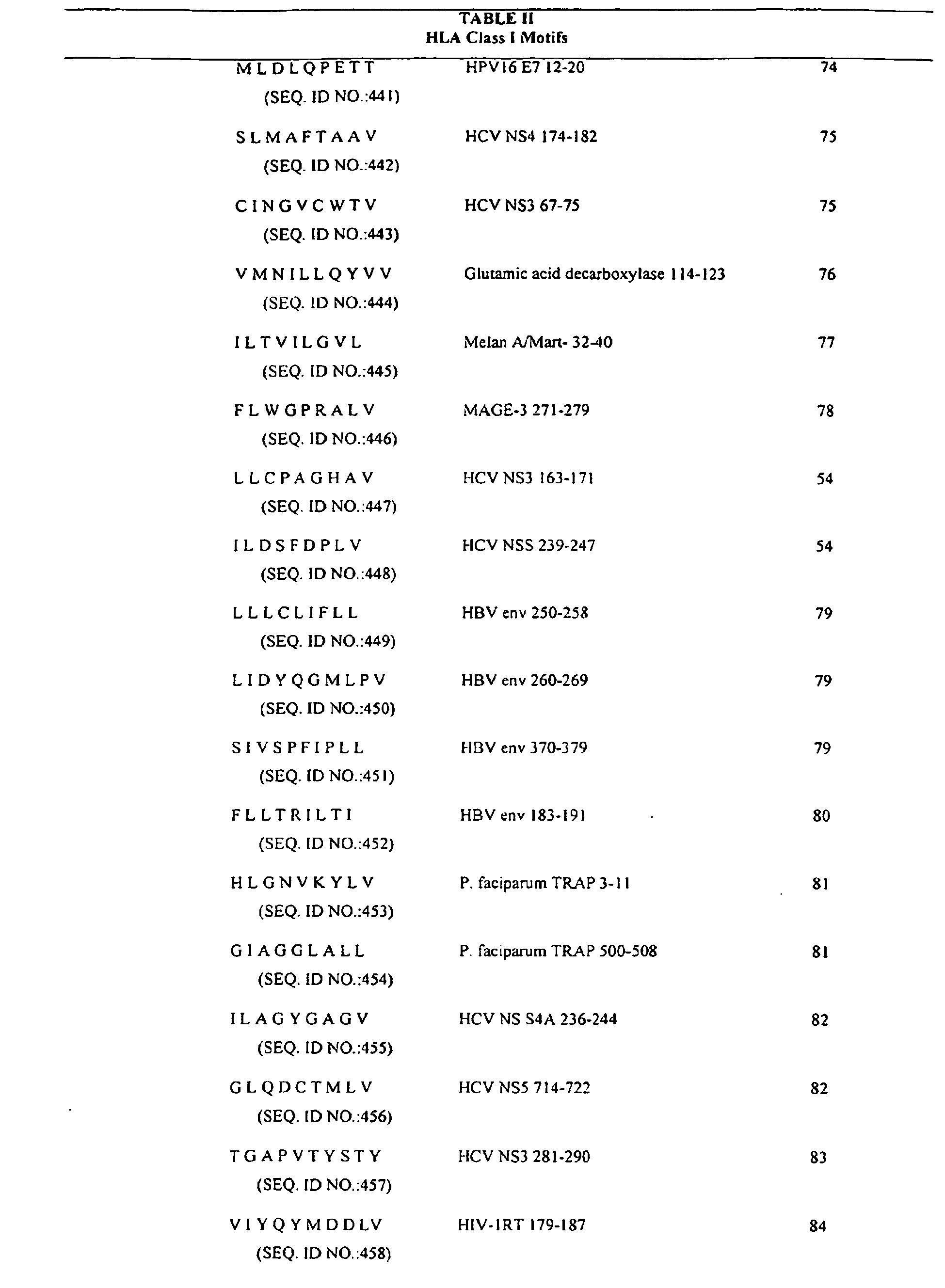

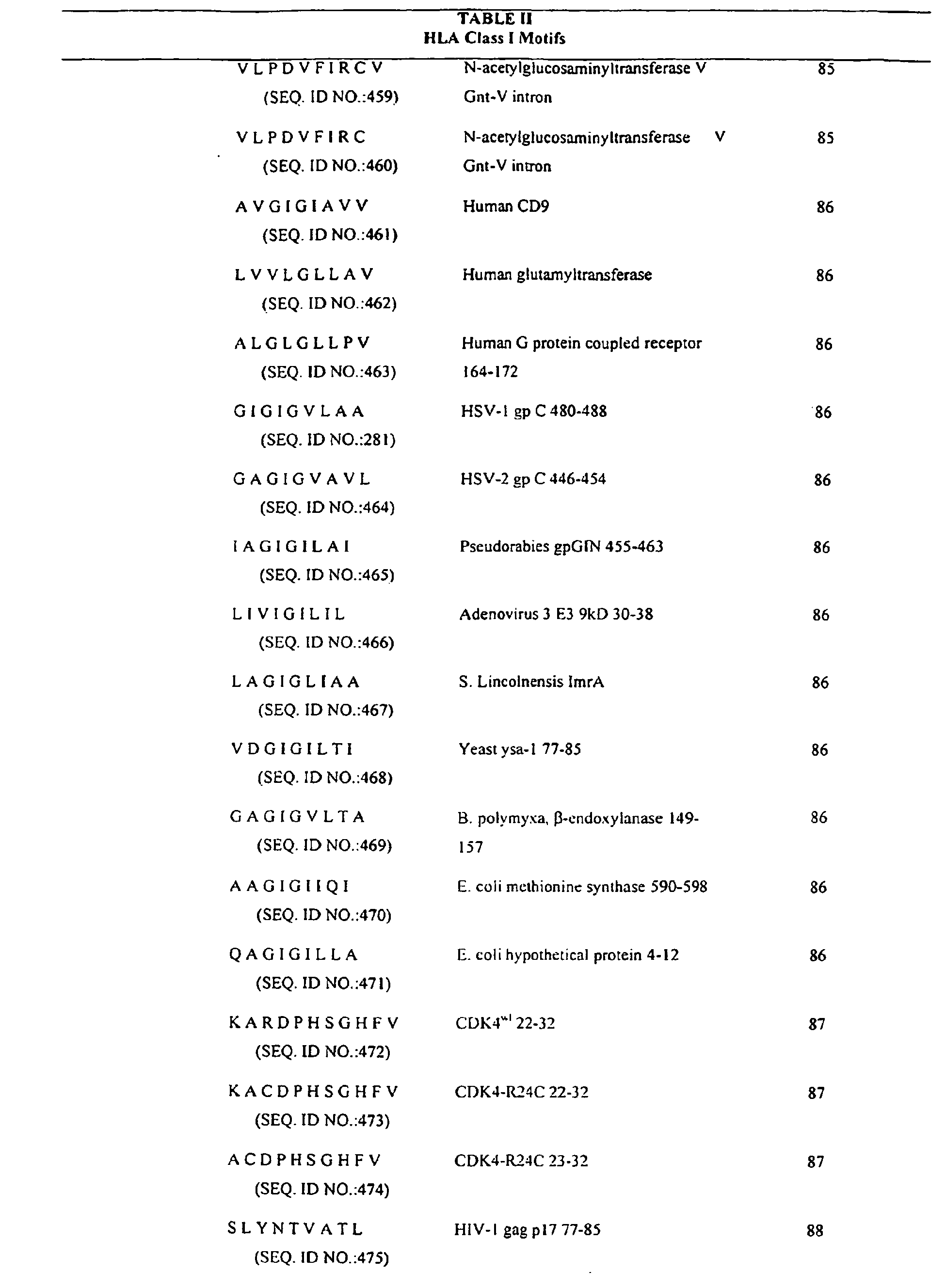

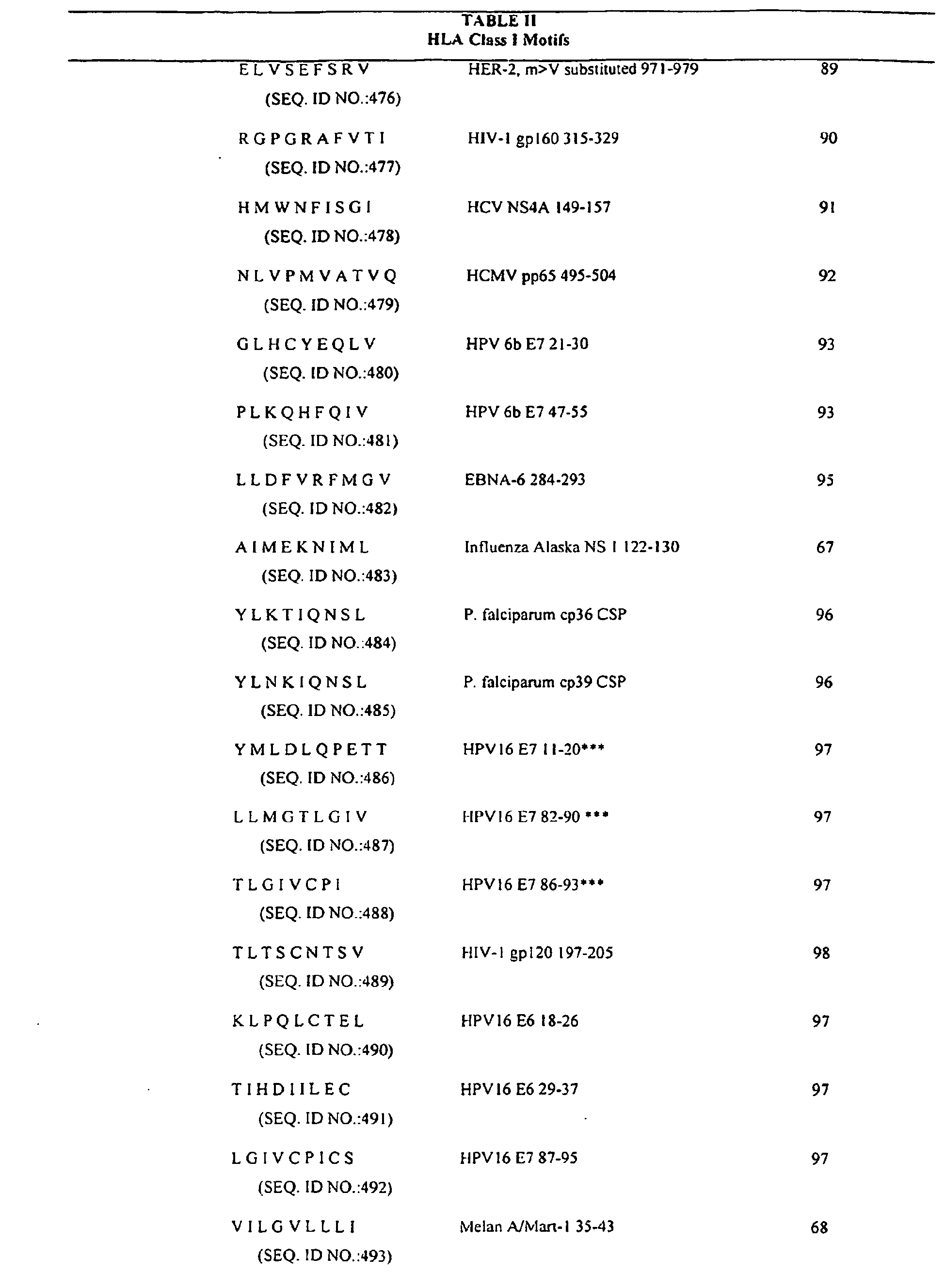

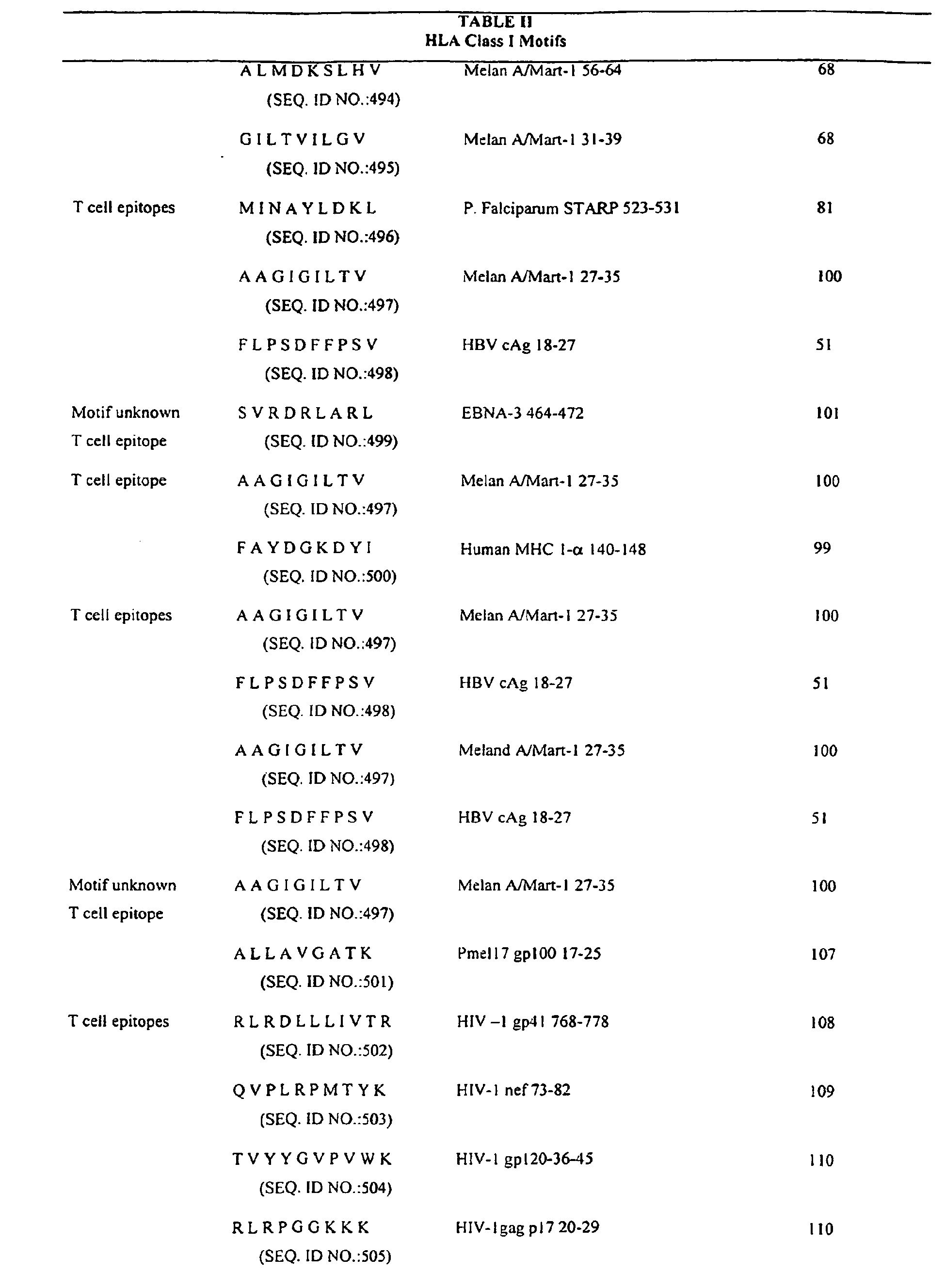

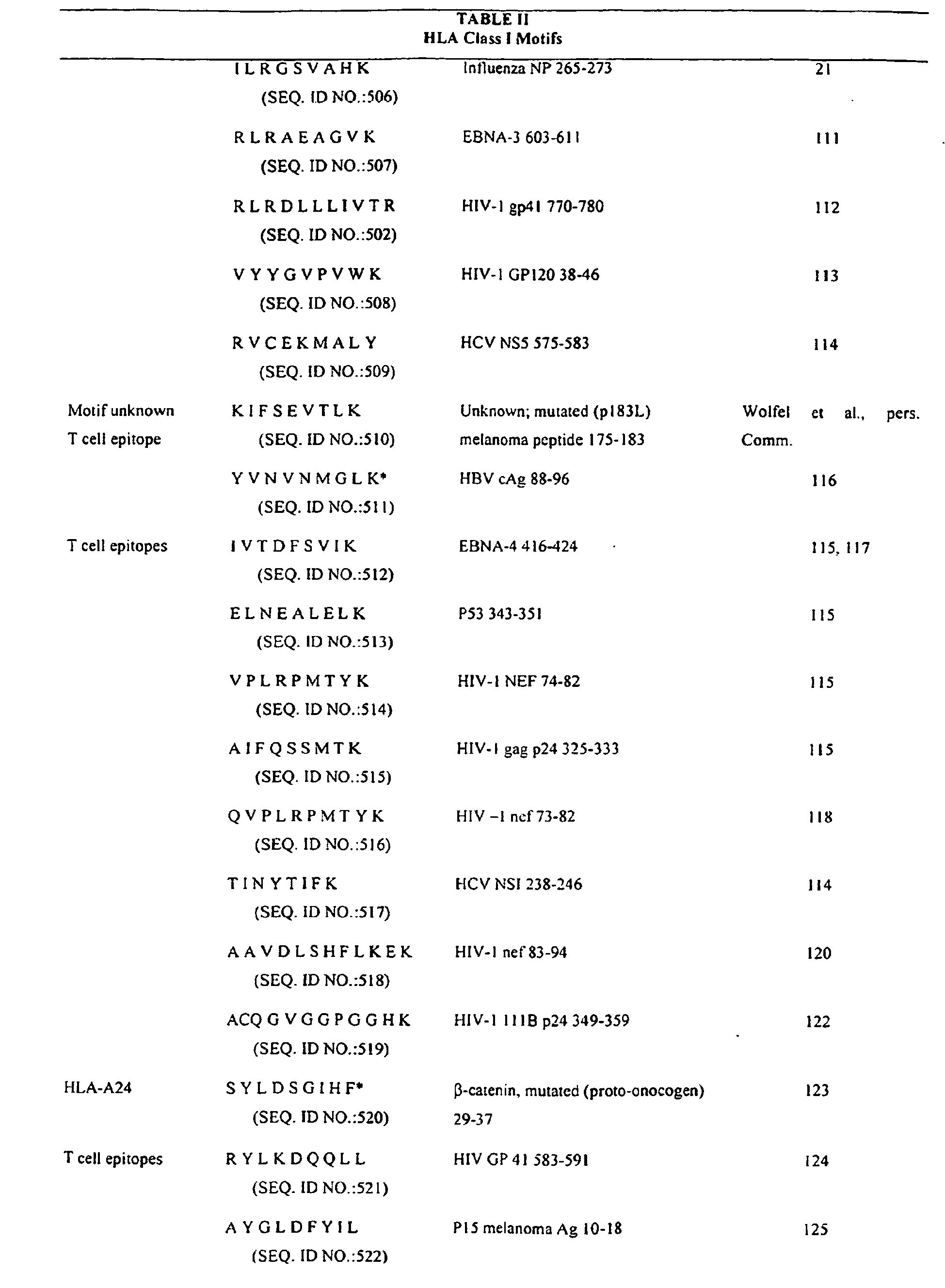

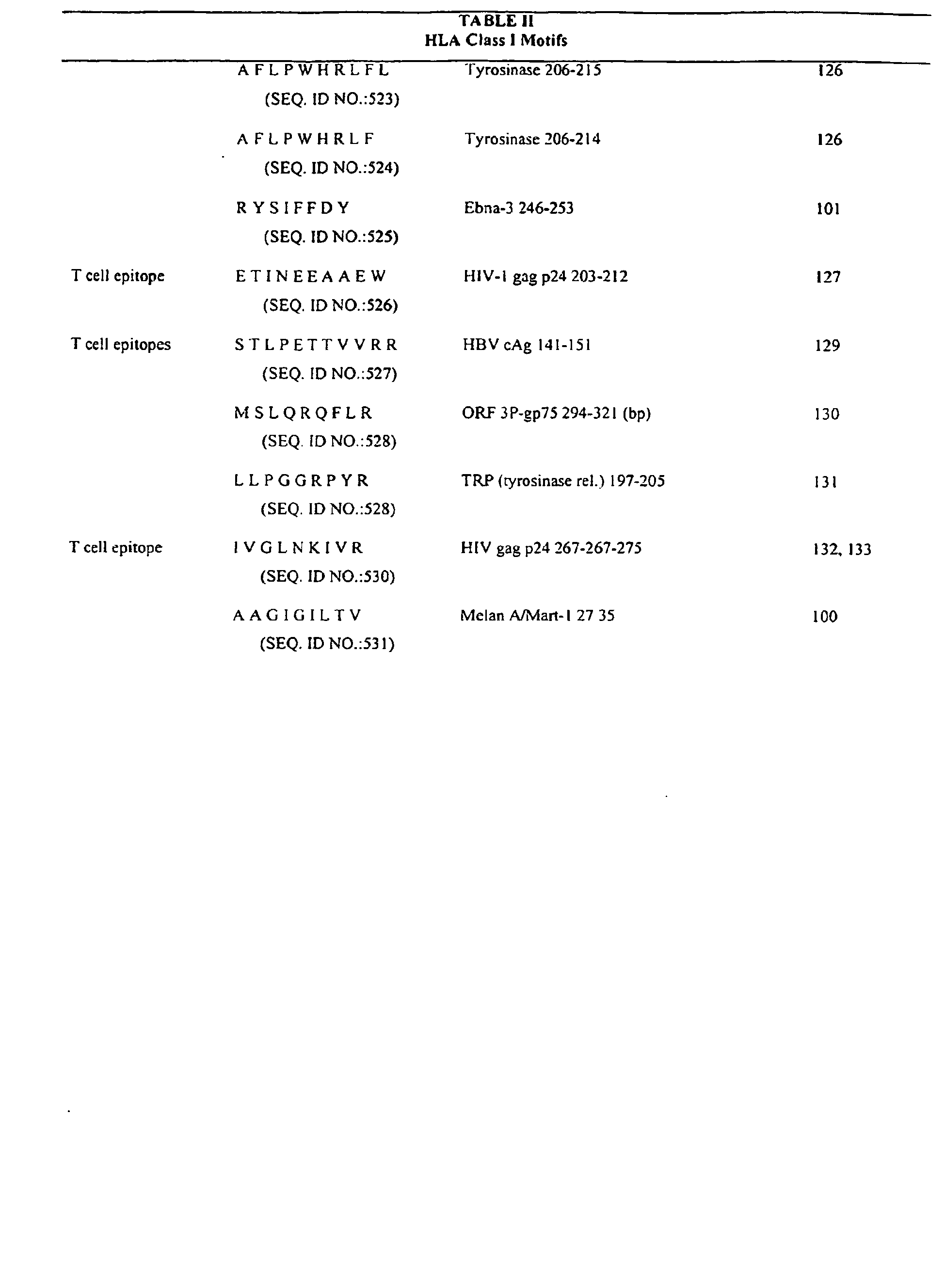

Table III sets forth additional antigens useful in the invention that are available from the Ludwig Cancer Institute. The Table refers to patents in which the identified antigens can be found and as such are incorporated herein by reference. TRA refers to the tumor-related antigen and the LUD No. refers to the Ludwig Institute number.

| **Table III** | | | | | |
|---|---|---|---|---|---|
| | **LUD** | **Patent** | | | |
| **TRA** | **No.** | **No.** | **Date Patent Issued** | **Peptide (Antigen)** | **HLA** |
| MAGE-4 | 5293 | 5,405,940 | 11 April 1995 | EVDPASNTY | HLA-A1 |
| | | | | (SEQ. ID NO.:532) | |
| MAGE-41 | 5293 | 5,405,940 | 11 April 1995 | EVDPTSNTY | HLA-A1 |
| | | | | (SEQ ID NO:533) | |
| MAGE-5 | 5293 | 5,405,940 | 11 April 1995 | EADPTSNTY | HLA-A1 |
| | | | | (SEQ ID NO:534) | |
| MAGE-51 | 5293 | 5,405,940 | 11 April 1995 | EADPTSNTY | HLA-A |
| | | | | (SEQ ID NO:534) | |
| MAGE-6 | 5294 | 5,405,940 | 11 April 1995 | EVDPIGHVY | HLA-A1 |
| | | | | (SEQ ID NO:535) | |
| | 5299.2 | 5,487,974 | 30 January 1996 | MLLAVLYCLL | HLA-A2 |
| | | | | (SEQ ID NO:536) | |
| | 5360 | 5,530,096 | 25 June 1996 | MLLAVLYCL | HLA-B44 |
| | | | | (SEQ ID NO:537) | |
| Tyrosinase | 5360.1 | 5,519,117 | 21 May 1996 | SEIWRDIDFA | HLA-B44 |
| | | | | (SEQ ID NO:538) | |
| | | | | SFIWRDIDF | |
| | | | | (SEQ ID NO:539) | |
| Tyrosinase | 5431 | 5,774,316 | 28 April 1998 | XEIWRDIDF | HLA-B44 |
| | | | | (SEQ ID NO:540) | |
| MAGE-2 | 5340 | 5,554,724 | 10 September 1996 | STLVEVTLGEV | HLA-A2 |
| | | | | (SEQ ID NO:541) | |
| | | | | LVEVTLGEV | |
| | | | | (SEG ID NO:542) | |
| | | | | VIFSKASEYL | |
| | | | | (SEQ ID NO:543) | |
| | | | | IIVLAIIAI | |
| | | | | (SEQ ID NO:544) | |
| | | | | KIWEELSMLEV | |
| | | | | (SEQ ID NO:545) | |
| | | | | LIETSYVKV | |
| | | | | (SEQ ID NO:546) | |
| | 5327 | 5,585,461 | 17 December 1996 | FLWGPRALV | HLA-A2 |
| | | | | (SEQ ID NO:547) | |
| | | | | TLVEVTLGEV | |
| | | | | (SEQ ID NO:548) | |
| | | | | ALVETSYVKV | |
| | | | | (SEQ ID NO:549) | |
| MAGE-3 | 5344 | 5,554,506 | 10 September 1996 | KIWEELSVL | HLA-A2 |
| | | | | (SEQ ID NO:550) | |
| MAGE-3 | 5393 | 5,405,940 | 11 April 1995 | EVDPIGHLY | HLA-A1 |
| | | | | (SEQ ID NO:551) | |
| MAGE | 5293 | 5,405,940 | 11 April 1995 | EXDX₅Y | HLA-A1 |
| | | | | (SEQ. ID NO.:552) | |
| | | | | (but not EADPTGHSY) | |
| | | | | (SEQ. ID NO.:553) | |
| | | | | E (A/V) D X₅ Y | |
| | | | | (SEQ. ID NO.:554) | |
| | | | | E (A/V) D P X₄ Y | |
| | | | | (SEQ. ID NO.:555) | |
| | | | | E (A/V) D P (I/A/T) X₃ Y | |
| | | | | (SEQ. ID NO.:556). | |
| | | | | E (A/V) D P (I/A/T) (G/S) X₂ Y | |
| | | | | (SEQ. ID NO.:557) | |
| | | | | E (A/V) D P (I/A/T) (G/S) (H/N) X Y | |
| | | | | (SEQ. ID NO.:558) | |
| | | | | E (A/V) D P (I/A/T) (G/S) (H/N) (L/T/V) Y | |
| | | | | (SEQ. ID NO.:559) | |
| MAGE-1 | 5361 | 5,558.995 | 24 September 1996 | ELHSAYGEPRKLLTQD | HLA-C |
| | | | | (SEQ ID NO:560) | Clone 10 |
| | | | | EHSAYGEPRKLL | |
| | | | | (SEQ ID NO:561) | |
| | | | | SAYGEPRKL | |
| | | | | (SEQ ID NO:562) | |
| MAGE-1 | 5253.4 | TBA | TBA | EADPTGHSY | HLA-A1 |
| | | | | (SEQ ID NO:563) | |
| BAGE | 5310.1 | TBA | TBA | MAARAVFLALSAQLLQARLMKE | HLA-C |
| | | | | (SEQ ID NO:564) | Clone 10 |
| | | | | MAARAVFLALSAQLLQ | HLA-C |
| | | | | (SEQ ID NO:565) | Clone 10 |
| | | | | AARAVFLAL | HLA-C |
| | | | | (SEQ ID NO:566) | Clone 10 |
| GAGE | 5323.2 | 5,648,226 | 15 July 1997 | YRPRPRRY | HLA-CW6 |
| | | | | (SEQ. ID NO.:567) | |

Preferred peptide antigens are those of tumor associated antigens (TAA) and chronic infections. Particularly preferred peptide antigens are derived from tyrosinase, gp100 or Melan A for the treatment of melanoma.

The peptide antigens of this invention are readily prepared using standard peptide synthesis means known in the art. Generally they can be prepared commercially by one of numerous companies that do chemical synthesis. An example is American Peptides, Inc., where the distributor is CLINALFA AG (Laufelfingen, Switzerland). The antigens are prepared in accordance with GMP standards. Purity is assessed by analytical HPLC. The product is characterized by amino-acid analysis and tested for sterility and the absence of pyrogens.

In delivering an appropriate antigen, e.g., a polypeptide, to the animal's system it may be delivered directly as the polypeptide, or it may be delivered indirectly, e.g., using a DNA construct or vector, or a recombinant virus that codes for the desired antigen. Any vector driving expression in a professional antigen presenting cell is suitable for this purpose. In the indirect delivery, the antigen is expressed in the cell, to be presented by the MHC Class I on the surface of the cell to stimulate the CTL response.

Antigens may be used alone or may be delivered in combination with other antigens or with other compounds such as cytokines that are known to enhance immune stimulation of CTL responses, such as, GM-CSF, IL-12, IL-2, TNF, IFN, IL-18, IL-3, IL-4, IL-8, IL-9, IL-13, IL-10, IL-14, IL-15, G-CSF, IFN alpha, IFN beta, IFN gamma, TGF alpha, TGF beta, and the like. The cytokines are known in the art and are readily available in the literature or commercially. Many animal and human tumors have been shown to produce cytokines such as IL-4, IL-10, TGF-β that are potent modulators of the immune response and that protect tumors from immune-mediated destruction. The production of IL-4, IL-10 or TGF-B by the tumors may achieve this protective effect by suppressing the induction of cellular immunity, including the elaboration of CTL responses. Alternatively, cytokines that support CTL responses can be exogenously added to help in the balance between induction of anti-tumor cell mediated and non-tumor-destructive humoral responses. Several such exogenous cytokines show utility in experimental mouse vaccination models which are known to enhance CTL responses, including GM-CSF, IFN and IL-2. An effective exogenous cytokine that may be used is GM-CSF. GM-CSF is reported to enhance the expression of the so called "co-stimulatory" molecules, such as B7-1 or B7-2 on antigen presenting cells (APC), which are important players in the symphony of interactions that occur during stimulation of CTL by APC. Moreover, GM-CSF is known to induce activation of APC and to facilitate growth and differentiation of APC, thereby making these important CTL stimulating cells available both in greater numbers and potency.

### Delivery of the Antigen

This invention is based in part on the observation that a CTL response is not sustained using standard vaccine techniques. While not wanting to be bound by any particular theory, it is thought that T cells do not have a functional memory that is long-lived. Antibody-mediated B-cell memory, on the other hand, appears to have a long-lived effector memory. Thus, delivering an antigen that produces a CTL response must be done over time to keep the patient's immune system appropriately stimulated to attack the target cells. While it has been suggested that antigens and adjuvants can be prepared as biodegradable microspheres or liposomes, none of these preparations have thus far provided a CTL response that is useful for attacking cancer cells or pathogens on a long term basis. The delivery must be sustained over the desired period of time at a level sufficient to maintain the antigen level to obtain the desired response and that it must be delivered from a reservoir having fluid antigen composition that is introduced so that it reaches the animal's lymphatic system.

Ultimately antigen must find its way into the lymphatic system in order to efficiently stimulate CTL. However, delivery of antigen according to the invention can involve infusion into various compartments of the body, including but not limited to subcutaneous, intravenous, intraperitoneal and intralymphatic, the latter being preferred. Each of these various points of infusion results in antigen uptake into the lymphatic system. The relative amounts of antigen needed to induce a beneficial CTL response varies according to the different sites of infusion. In general, direct infusion of antigen into the lymph system is deemed to be the most efficient means of inducing a CTL response, but that the material difference between the various routes is not necessarily relevant in terms of the quantity of antigen needed, or, in terms of the operating parameters of the invention. The pump systems of the invention are capable of delivering material quantities of antigen in a range that makes the invention suitable for inducing CTL response through delivery to all compartments of the body. CTL stimulation based on delivery of antigen via various routes will be variable, based on the properties of different antigens, which will reflect factors that influence antigen behavior in the body and its rate of equilibration to (or longevity in) the lymph, such an antigen stability in the body fluid, solubility of antigen in body fluid, binding affinity for HLA and potency as a stimulator of CTL.

It is most efficient, and therefore, preferred, that the introduction is done as directly as possible to the lymphatic system to avoid the destruction of the antigen by metabolism in the body. When introduction of a fluid antigen composition occurs subcutaneously, larger quantities of antigen are needed to assure enough antigen reaches the lymphatic system. Such subcutaneous injection is contemplated by this invention if it can be justified by factors such as cost, stability of the antigen, how quickly the antigen gets to the lymph system, how well it equilibrates with the lymph, and other factors that the attending doctor or specialist will recognize. Subcutaneous delivery will generally require 100 to 1000 times more antigen than direct delivery to the lymph system. It is preferable, therefore, that the antigen composition is introduced through a device for local administration to the lymphatic system, e.g. the spleen, a lymph node, or a lymph vessel. The device for local administration may be positioned outside the patient or implanted into the patient. In either case, the device will have a reservoir to hold the fluid antigen-containing composition, a pump to transfer the composition, and a transmission channel leading from the reservoir to be directed to the preferred region of administration in the patient's body. In either case it is preferably portable.

For the device positioned outside the patient's body (the external device), there are numerous devices used for delivering insulin to diabetic patients that are useful in this invention. Generally these are comprised of a reservoir for holding the antigen composition (instead of insulin), a programmable pump to pump the composition out of the reservoir, a transmission channel or line for transmitting the composition, and a means to introduce the composition into the animal's body to ultimately reach the lymphatic system.

The pump employed may be a roller/peristaltic pump, a syringe pump, a piston/valve pump, a gas pressure pump, or the like that has a power source (generally a battery for portability) that is programmable to deliver the desired level of antigen composition to the patient's body and the lymphatic system. A further discussion of the operation of these pumps may be found "Insulin Pump Therapy" by E. Austenst and T. Stahl, Walter de Gruyter, Berlin, New York (1990), at Chapter 3. A list of pumps available at that time that are useful for this invention are given in Table IV. More recent versions of these pumps are available from the manufacturers shown.

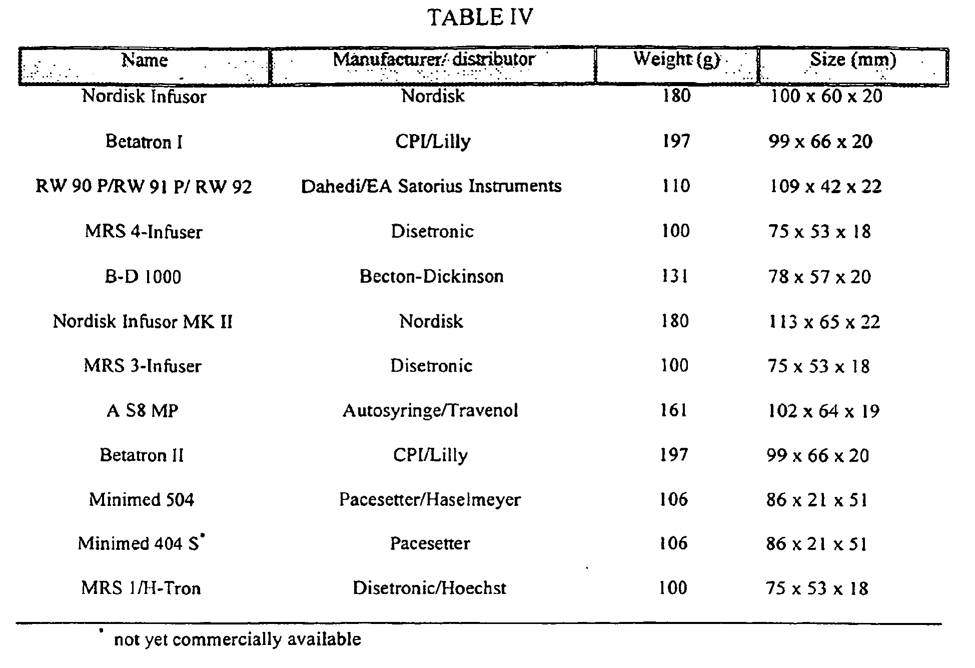

Particularly useful pumps are the Disetronic H-Tron V100 Insulin Pump from Disetronic Medical Systems, Burgdorf, Switzerland and the Minimed 507 Insulin Pump from MiniMed Inc., 12744 San Fernando Road, Sylmar, California 91342. The MiniMed is particularly useful in that it allows programming a bolus without looking at the pump through a series of audio tones (settable in either 0.5 or 1.0 unit increments) and allows programming a bolus for delivery over an extended period of time - from 30 minutes to 4 hours. It provides up to 12 basal rates (or profiles) that can be programmed per 24 hours from 0.0 -25 units/hour in 0.1 unit increments. The device allows for the temporary increase or decrease of a set basal rate from 30 minutes to 24 hours in 30 minute increments. Other features relating to safety, time display, memory, etc. are available from the manufacturer.

The reservoir for the antigen composition should be large enough for delivery of the desired amount of antigen over time and is easily refillable or replaceable without requiring the user to reinsert the means for introducing the antigen composition to the lymph system.

In preparing the antigen compositions of this invention, a composition (preferably aqueous) is prepared to be compatible with the lymph system and is physiologically acceptable to the animal being treated. In preparing the antigen compositions useful in this invention one considers the physicochemical properties of the antigen such as the isoelectric point, molecular weight, glycosylation or other post-translational modification, and overall amino acid composition. These properties along with any known behavior of the drug in different solutions (e.g. different buffers, cofactors, etc.) as well as its *in vivo* behavior will help guide the choice of formulation components. One parameter that impacts all the major degradation pathways is the solution pH. Thus, the initial formulations also assess the pH dependence of the degradation reactions and the mechanism for degradation can often be determined from the pH dependence to determine the stability of the protein in each solution. Rapid screening methods usually involve the use of accelerated stability at elevated temperatures (e.g. 40° C) using techniques known in the art.

In general the antigen compositions useful in this invention will be prepared suitable for parenteral injection, in very small quantities. As such a composition must be free of contamination and have a pH compatible with the lymph system. However, because very small quantities of the antigenic composition will be delivered it need not be the same pH as blood or lymph, and it need not be aqueous-based. For antigens that are less soluble a suitable cosolvent or surfactant may be used, such as dimethyl sulfoxide (DMSO) or PLURONIC brand surfactants. The pH range that is compatible is from about 6.7 - 7.3 and can be prepared using water for injection to meet USP specifications (see Remington: The Science and Practice of Pharmacy, Nineteenth Edition; Chapters 86-88). Generally, a standard saline solution that is buffered with a physiologically acceptable weak acid and its base conjugate, e.g., a phosphate or citrate buffering system, will be the basis of the antigen composition. In some cases, a small amount of an antioxidant may be useful to stabilize the composition and prevent oxidation. Factors to consider in preparing the antigen compositions may be found in the 1994 American Chemical Society book entitled *"Formulation and Delivery of Proteins and Peptides"* (Acs Symposium Series, No. 567) by Jeffery L. Cleland and Robert Langer (Editor)).

Generally the amount of the antigen in the antigen composition will vary from patient to patient and from antigen to antigen, depending on such factors as the activity of the antigen in inducing a response and the flow rate of the lymph through the patient's system. In general the antigen composition may be delivered at a rate of from about 1 to about 500 microliters/hour or about 24 to about 12000 microliters/day. The concentration of the antigen is such that about 0.1 micrograms to about 10,000 micrograms of the antigen will be delivered during 24 hours. The flow rate is based on the knowledge that each minute approximately about 100 to about 1000 microliters of lymph fluid flows through an adult inguinal lymph node. The objective is to maximize local concentration of vaccine formulation in the lymph system. A certain amount of empirical investigation on patients will be necessary to determine the most efficacious level of infusion for a given vaccine preparation in humans.

To introduce the antigen composition into the lymphatic system of the patient, the composition is preferably directed to a lymph vessel, lymph node, the spleen, or other appropriate portion of the lymph system. Preferably, the composition is directed to a lymph node such as an inguinal or axillary node by inserting a catheter or needle to the node and maintaining the catheter or needle throughout the delivery. Suitable needles or catheters are available made of metal or plastic (e.g. polyurethane, polyvinyl chloride [PVC], TEFLON, polyethylene, and the like). In inserting the catheter or needle into the inguinal node for example, the inguinal node is punctured under ultrasonographic control using a Vialon™ Insyte-W™ cannula and catheter of 24G_{3/4} (Becton Dickinson, USA) which is fixed using Tegaderm transparent dressing (Tegaderm™ 1624, 3M, St. Paul, MN 55144, USA). This procedure is generally done by an experienced radiologist. The location of the catheter tip inside the inguinal lymph node is confirmed by injection of a minimal volume of saline, which immediately and visibly increases the size of the lymph node. The latter procedure allows confirmation that the tip is inside the node and can be performed to ensure that the tip does not slip out of the lymph node can be repeated on various days after implantation of the catheter. In case the tip did in fact slip out of location inside the lymph node, a new catheter can be implanted.

In another embodiment, the antigen is delivered to the lymphatic system through an article of manufacture that is implanted in the animal, preferably at or near a site of a lymphatic organ. The article will include a pump that can deliver the antigen at a controlled rate over a pre-determined period of time and is suitable for use in the host. Several devices are known in the art for the delivery of agents (such as drugs) in humans or animals and these can be used or adapted for use in the present invention.

The implantable device will be similar to the external device discussed above in that it comprises a reservoir of a physiologically-acceptable, aqueous, antigen-containing composition that is capable of inducing a CTL response in an animal, a pump positioned in association with the reservoir to deliver the composition at a defined rate, a transmission channel to discharge the composition from the reservoir, and optionally a delivery line connected to the transmission channel, which delivery line is of a size suitable for positioning in the animal and for delivery of the composition in a manner that reaches the lymphatic system of the animal.

Preferably the pump in the implantable device is an osmotic pump of the type used in the ALZET® model device or the DUROS™ model device pioneered by Alza Corporation, Palo Alto, CA or in a device made by Pharmetrix and exemplified in U.S. patent 4,838,862. The osmotic pump utilizes the osmotic effect using a membrane permeable to water but impermeable to a solute. Osmotic pressure built up in a device is used to deliver a composition at a controlled rate over time. A review by Giancarlo Santus and Richard Baker of "Osmotic Drug Delivery: A Review of the Patent Literature" in the Journal of Controlled Release 35 (1995) 1-21, provides useful guidelines for the type of osmotic pumps that are useful in this invention. The osmotic pump forces the composition through a discharge orifice to discharge the composition. Optionally a delivery line connects to the discharge orifice to position the line suitably for delivery to the lymphatic system of the animal. Patents that describe devices useful in this invention include the following U.S. patents: (A) 3,604,417 assigned to American Cyanamid; (B) 4,838,862; 4,898,582; 5,135,498; 5,169,390; and 5,257,987 all assigned to Pharmetrix, (C) 4,340,048; 4,474,575; 4,552,651; 4,619,652; 4,753,651; 3,732,865; 3,760,804; 3,760,805; 3,929,132; 3,995,632; 4,034,756; 4,350,271; 4,455,145; 5,017,381; 5,023,088; 5,030,216; 5,034,229; 5,037,420; 5,057,318; 5,059,423; 5,110,596; 5,110,597; 5,135,523; 5,137,727; 5,174,999; 5,209,746; 5,221,278; 5,223,265; 3,760,984; 3,987,790; 3,995,631; 4,203,440; 4,286,067; 4,300,558; 4,304,232; 4,340,054; 4,367,741; 4,450,198; 4,855,141; 4,865,598; 4,865,845; 4,872,873; 4,929,233; 4,963,141; 4,976,966, all assigned to Alza Corp. Osmotic pumps have been used for example to deliver Hyptophan to albino mice (Peters and Buhr (1984) Neurochem. Int. 6(5): 685 - 691 and to deliver IL-2 to mice (US-A-4908433).

A basic osmotic pump device incorporates a housing containing a chamber for storing the antigen containing composition to be delivered, separated from a compartment containing an osmotic salt material by a barrier that is moveable under pressure such as a piston or a flexible impermeable membrane. The compartment containing the osmotic salt is separated from osmotic fluid by a semipermeable membrane. In some embodiments, a fluid barrier, such as a foil sheet, isolates the osmotic salt chamber from the osmotic fluid, keeping the pump inactivated until removal of the barrier immediately prior to use. Other osmotic pump devices use body fluid as the osmotic fluid. In these devices a semipermeable membrane separates the osmotic salt compartment from body fluids, and the pump is activated once inserted into the body under exposure to body fluids. In either case, volumetric expansion of the osmotic salt compartment drives the expulsion of the stored antigen from the compartment and into the surrounding environment of the body. These pumps have been highly successful at achieving steady-state pumping and delivery of agents. The pumps are of a small size that can be inserted into a patient, with flexible consideration as to location. This is important in the case of CTL vaccines, since the inventor has determined that efficient induction of CTL responses is contingent on the antigen or antigen expression system being delivered into the lymphatic system, in order to ultimately achieve antigen delivery into a lymphatic organ such as the spleen. Antigen delivered into a lymph node is 100-1000 times more efficient at inducing CTL responses compared with conventional subcutaneous delivery. A modification to the osmotic pump incorporates a microcatheter attachment (i.e., the optional delivery line) at its discharge end, such that when the pump is implanted proximal to a lymphatic organ, such as a lymph node, the catheter can be inserted into the organ to facilitate delivery of the vaccine directly into the lymphatic system.

Prior to the administration of the antigen using any of the above vehicles, methods may be used to assist in the determination of the optimum location for the antigen delivery. For example, when using the osmotic pump, radiography may be used to image a patient's lymphatic flow, to determine where relatively high lymphatic drainage occurs, in order to decide upon an insertion position for the osmotic pump that maximizes delivery into the lymphatic system. Since each patient has unique lymphatic drainage profiles, imaging would be conducted for each individual prior to insertion of osmotic pump for delivery of antigen. When using direct cannulation of the lymphatic vessel, such as in the use of osmotic or insulin pumps to deliver antigen, ultrasound can be used to position the needle directly into the lymphatic vessel and to monitor its positioning over the period of treatment.

The following non-limiting examples are illustrative of the present invention.

### EXAMPLES

### Materials and Methods For Examples 1-5

Mice: The generation of T cell receptor transgenic mice (TCR+ mice) in which approx. 90% of the CD8+ T cells express a TCR recognizing the immunodominant LCMV-glycoprotein epitope (gp-peptide aa33-41, p33:KAVYNFATC-SEQ ID NO:569) presented on H-2D^{b}, has been described in detail. All experimental mice were between 8 and 12 weeks of age and bred and held under strict pathogen free conditions at the Institut Für Labortierkunde at the University of Zurich.

Viruses: LCMV (Armstrong strain) was originally obtained from Dr. M.B.A. Oldstone, Scripps Clinics and Research Foundation, LaJolla, San Diego, CA. Seed virus was grown on BHK cells and plaqued on MC57 cells using an immunological focus assay, as described previously.

### Osmotic pump: ALZA model #1007b.

*In vivo* protection assays for specific CTL activity: The *in vivo* assay for the detection of CTL activity by challenge infections with LCMV has been described in detail previously (Oehen et al. 1991). Briefly, mice are intravenously challenged with 2X10³ pfu of LCMV (Armstrong). After 4 days the titer of LCMV is determined using the above mentioned immunological focus assay.

**Primary ex vivo cytotoxicity against LCMV-gp**: Mice were injected intravenously with 10µg of p33. After 36 hours spleen single cell suspensions were coincubated for 5h with ⁵¹Cr-labeled syngeneic EL-4 (H-2^{b}) target cells, that were either pulsed with p33 or left unpulsed. Specific lysis was calculated as [(experimental ⁵¹Cr release - spontaneous ⁵¹Cr release) / (total ⁵¹Cr release - spontaneous ⁵¹Cr release) X 100%].

**LCMV induced foot pad swelling reaction**: Mice were infected with LCMV (Armstrong) by intradermal injection into the hind footpad (5000 pfu in 30:1). Footpad thickness was measured daily with a spring loaded caliper. Footpad swelling is calculated as (measured thickness -thickness before injection) / (thickness before injection).

### Example 1

### Continuous release of peptide antigen using osmotic pump induces potent CTL response in C57BL/6 Mice

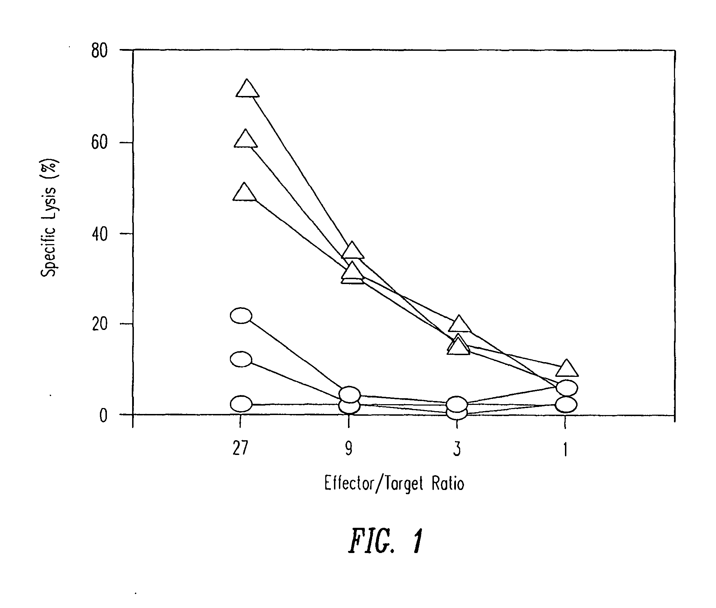

C57BL16 mice were either intravenously injected with a single dose of 50µg p33 (including 500 ng GM-CSF) (circles) or were implanted with a micro-osmotic pump releasing a mixture of 50µg of p33 and 500 ng GM-CSF over a time period of 7 days (triangles), or were left naive (data not shown). After 7 days mice were sacrificed to prepare single cell suspensions from the spleen. Spleen cells were restimulated in vitro for 5 days by p33 pulsed in the presence of low amounts of IL-2. Specific cytotoxicity was measured using ⁵¹Cr-labeled EL-4 target cells pulsed with p33. Specific lysis of EL-4 target cells without p33 was less than 16% for all effectors. The results are shown in Figure 1.

### Example 2

### Continuous release of antigen induces CTL immunity against virus in C57BL/6 mice

C57BL/6 mice were either intravenously injected with a single dose of 50µg p33 (including 500 ng GM-CSF. Pharmingen) or were implanted with a microsomotic pump releasing a mixture of 50µg of p33 and 500 ng GM-CSF over a time period of 7 days, or were left naive. After 7 days specific CTL activity was assessed *in vivo* using anti-viral protection assays. C57BL/6 mice were intravenously challenged with LCMV Armstrong strain (2x10³ p.f.u.). After 4 days mice were sacrificed and LCMV titers were determined in spleens using an immunological focus assay. Mice implanted with osmotic pump showed significantly lower virus titers indicating active CTL immunity against the virus (Table V).

**TABLE V**

| **C57BL/6 Mice** | **Virus Titer (log**_{**10**}**)** |
|---|---|
| Single injection | 4.2 |
| Single injection | 4.6 |
| Single injection | 4.0 |
| Pump delivered | 2.2 |
| Pump delivered | 1.8 |
| Pump delivered | 2.0 |
| Unprimed | 4.8 |
| Unprimed | 3.8 |
| Unprimed | 4.4 |

### Example 3

### Continuous release of antigen maintains potent CTL effectors in TCR Transgenic Mice

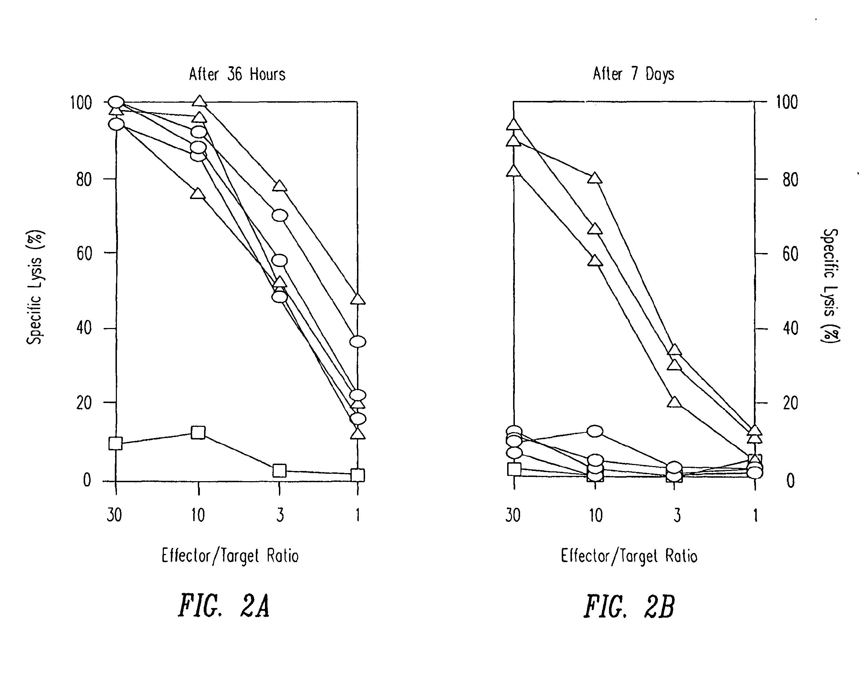

TCR transgenic mice were either intravenously injected with a single dose of 50µg p33 (circles) or were implanted with a microsomotic pump releasing a mixture of 50µg of p33 (triangles), or left naive (squares). After 36 hours mice were sacrificed to prepare single cell suspensions from the spleen which were assayed ex vivo for p33-specific cytotoxicity using ⁵¹Cr-labeled EL-4 target cells pulsed with p33. Similarly mice were either intravenously injected with a single dose of 50µg p33 (circles) or were implanted with a micro-osmotic pump releasing a mixture of 50µg of p33 over a time period of 7 days (triangles), or were left naïve (squares). After 7 days mice were sacrificed to prepare single cell suspensions from the spleen to assay ex vivo p33-specific cytotoxicity using ⁵¹Cr-labeled EL-4 target cells pulsed with p33. Specific lysis of EL-4 target cells without p33 was less than 18% for all effectors. The results are shown in Figures 2A and 2B.

### Continuous release of antigen maintains protective CTL response against virus infection.

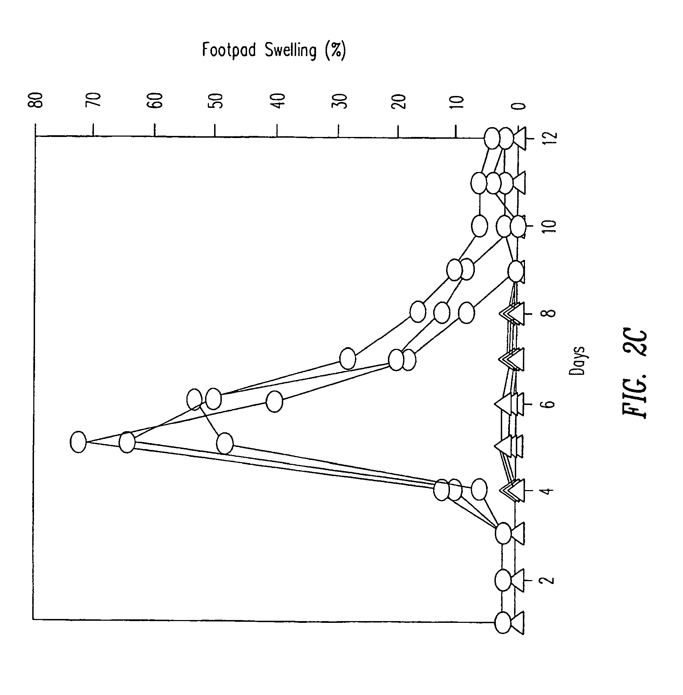

After 7 days TCR transgenic mice were challenged by intradermal LCMV injection into their hind foot pads (2x10³ pfu in 30µl). The absence of a foot pad swelling reaction, as observed in mice with an implanted pump (triangles), indicates that at the time point of injection there was active CTL immunity inhibiting local replication of the virus in the foot pad. In contrast, foot pad swelling, as observed in mice injected with the peptide as a single bolus (circles) and naive control mice (data not shown), indicated that LCMV successfully replicated in the foot pad in the absence of protective CTL. The results are shown in Figure 2C.

### Example 4

### Direct delivery of antigen into lymphatic organ dramatically increases efficiency of CTL induction

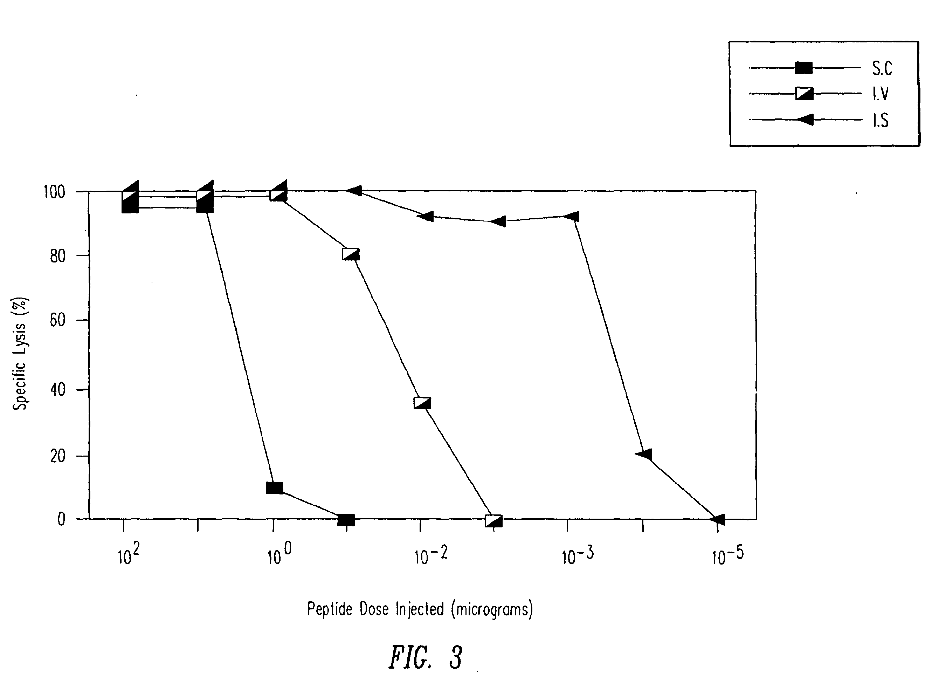

TCR transgenic mice were injected with graded doses of gp-peptide p33 either subcutaneously (S.C.), intravenously (I.V.) or directly into the spleen (I.S.) via a small abdominal incision. The efficiency of CTL induction was assessed by measuring gp-specific CTL activity 24 hours after injection. CTL activity is known to peak one day after injection of peptide. Mice were sacrificed to prepare single cell suspensions from draining lymph nodes or from spleen to assay ex vivo p33-specific cytotoxicity using ⁵¹Cr-labeled EL-4 target cells pulsed with p33. Specific lysis of EL-4 target cells without p33 was less than 12% for all effectors. The results are shown in Figure 3.

### Example 5

### Dendritic Cells Purified from Mice Receiving Intrasplenic Injection of Peptide Potently Stimulate CTL

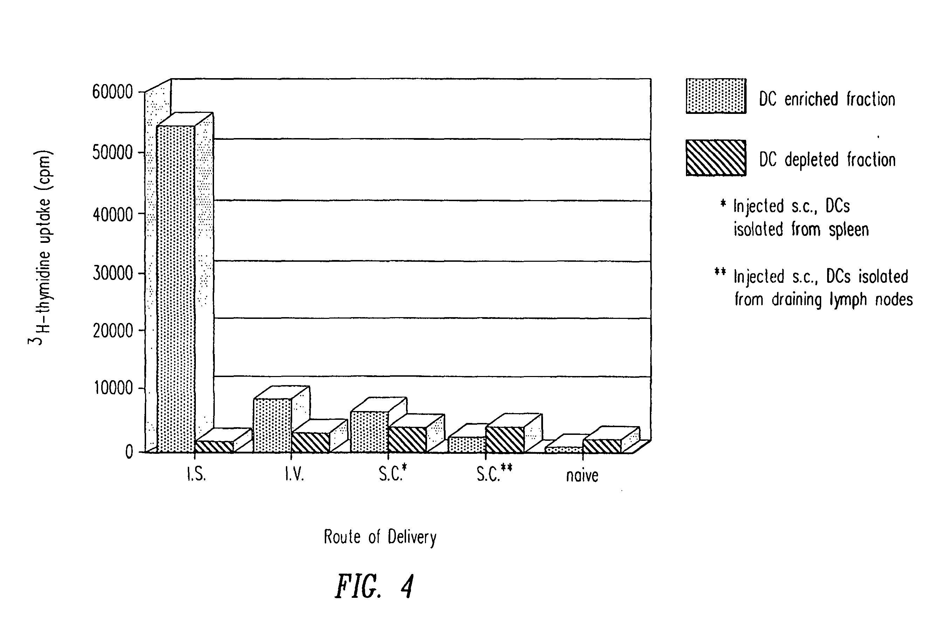

The effect of directing peptide delivery into lymphatic system was assessed. Peptide p33 was injected either i.v., s.c. or directly into the spleen of wild-type C57BL/6 mice. After 2 hours, DCs were isolated from the spleen of animals injected either i.s. or i.v., and additionally from the regional draining lymph nodes of animals injected s.c. Cells isolated from these tissues were sorted for DCs using magnetic beads coupled with a monoclonal antibody recognizing the integrin-alpha chain, a marker specific for DCs in spleen and lymph nodes. The positively and the negatively sorted cell fractions were compared regarding their capacity to *in vitro* stimulate naive CD8+ T cells from TCR transgenic mice specific for LCMV-gp. Only when peptide had been directly injected into the spleen, the DC containing cell fraction stimulated CTL to proliferate, as measured by ³H-thymidine uptake. This indicated that CTL induction after direct injection of peptide into lymphatic organs reflected efficient loading of DCs with peptide. In contrast, the fraction depleted for DC did not induce proliferation and DCs isolated from lymphoid organs of i.v. and s.c injected mice were not effective stimulators. The results are shown in Figure 4.

While the present invention has been described with reference to what are presently considered to be the preferred examples, it is to be understood that the invention is not limited to the disclosed examples.

## Claims

1. A device for delivering an antigen and sustaining an induced CTL response or inducing and sustaining a CTL response in an animal, which device comprises:
a reservoir filled with a fluid physiologically-acceptable, peptide antigen-containing composition free of conventional adjuvants that comprises an adequate amount of said antigen that is capable of providing an antigen level in the animal that is sufficient to sustain an induced CTL response or to induce and sustain a CTL response in said animal against said antigen;
a pump connected to the reservoir to deliver the composition at a defined rate; and
a transmission line to discharge the composition from the reservoir, wherein the transmission line is capable of positioning in the animal and can deliver the composition in a manner that reaches the lymphatic system of the animal.

2. The device according to claim 1, wherein the reservoir is removable from the device and is refillable with said physiologically acceptable, antigen-containing composition that is capable of inducing and sustaining a CTL response in an animal.

3. The device according to claim 1 or 2, wherein the composition comprises only one antigen.

4. The device according to claim 1 or 2, wherein the composition comprises more than one antigen.

5. The device according to any of the preceding claims, wherein the composition further comprises a cytokine capable of enhancing a CTL response.

6. The device according to claim 5, wherein the cytokine is selected from the group consisting of GM-CSF, IL-12, IL-2, TNF, IL-18, IL-3, IL-8, IL-9, IL-13, IL-10, IL-14, IL-15, G-CSF, IFN alpha, IFN beta, IFN gamma, TGF alpha and TGF beta.

7. The device according to any of the preceding claims, wherein the antigen is selected from the group consisting of a differentiation antigen, a tumor-specific multilineage antigen, an embryonic antigen, an oncogene antigen, a mutated tumor-suppressor gene antigen and a viral antigen.

8. The device according to claim 7, wherein the antigen is selected from the group consisting of MART-1/MelanA (MART-1), gp100 (Pmel 17), tyrosinase, TRP-1, TRP-2, MAGE-1, MAGE-3, BAGE, GAGE-1, GAGE-2, p15(58), CEA, p53, Ras, HER-2/neu, BCR-ABL, E2A-PRL, H4-RET, IGH-IGK, MYL-RAR, EBVA, (HPV) antigens E6 and E7, TSP-180, MAGE-4, MAGE-5, MAGE-6, RAGE, NY-ESO, p185erbB2, p18erbB-3, c-met, nm-23H1, PSA, TAG-72, CA19-9, CA 72-4, CAM 17.1, NuMa, K-ras, H-ras, β-Catenin, CDK4, Mum-1, p15 and p16.

9. The device according to any of the preceding claims, wherein said transmission line comprises a delivery line that is of a size suitable for positioning in the animal and for delivery of the composition in a manner that reaches the lymphatic system of the animal.

10. The device according to claim 9, wherein the device is an external device and the delivery line is a catheter that is long enough for delivery to the animal subcutaneously or lymphatically.

11. The device according to claim 10, wherein said catheter is long enough for delivery directly to the lymphatic system of the animal.

12. The device according to claim 11, wherein the delivery to the lymphatic system is through an axillary or inguinal node.

13. The device according to any of the preceding claims, wherein the position permitting delivery of the composition is a lymph node or a lymph vessel.

14. The device according to claim 13, wherein the position permitting delivery of the composition is an area of high lymphatic drainage.

15. The device according to claim 14, wherein the area of high lymphatic drainage is determined by imaging lymphatic flow of the animal.

16. The device according to any of the preceding claims, wherein the device is portable.

17. The device according to claim 16, wherein the device is of a size suitable for portably attaching to a human.

18. The device according to any of the preceding claims, wherein the pump is a roller/peristaltic pump, a syringe pump or a gas pressure pump.

19. The device according to any of the preceding claims, wherein the pump is battery operated.

20. The device according to any of the preceding claims, in combination with printed instructions for delivery of the antigen composition on a regular basis over time to maintain the antigen in the animal's lymphatic system at a level sufficient to maintain a CTL response in the animal.

21. A process for preparing a system useful for sustaining an induced CTL response or inducing and sustaining a CTL response in an animal capable of benefiting from such a response, which comprises:
placing a physiologically acceptable, aqueous, peptide antigen-containing composition free of conventional adjuvants that comprises an adequate amount of said antigen that is capable of providing an antigen level in said animal that is sufficient to sustain an induced CTL response or to induce and sustain a CTL response in said animal against said antigen in a reservoir of a device according to any of claims 1 to 20, said device having a pump for delivering the composition at a defined rate through a transmission line to the animal.

22. The process according to claim 21, wherein
a peptide antigen specific to a tumor or pathogen is matched to a tumor or pathogen in the animal, and
a physiologically acceptable composition of the matched peptide antigen is prepared before the composition is placed in the reservoir.

## Patentansprüche

1. Vorrichtung zum Zuführen eines Antigens und Aufrechterhalten einer induzierten CTL-Antwort oder zum Induzieren und Aufrechterhalten einer CTL-Antwort in einem Tier, umfassend:
einen Behälter, der mit einer flüssigen, physiologisch annehmbaren, Peptidantigenenthaltenden Zusammensetzung gefüllt ist, die frei von herkömmlichen Zusatzstoffen ist und eine ausreichende Menge des Antigens umfasst, welche geeignet ist, in dem Tier einen Antigen-Pegel bereitzustellen, der ausreicht, um in dem Tier gegen das Antigen eine induzierte CTL-Antwort aufrechtzuerhalten oder eine CTL-Antwort zu induzieren und aufrechtzuerhalten;
eine mit dem Behälter verbundene Pumpe zum Zuführen der Zusammensetzung in einer definierten Rate; und
eine Übertragungsleitung zum Ausleiten der Zusammensetzung aus dem Behälter, wobei die Übertragungsleitung geeignet ist, in das Tier eingebracht zu werden, und die Zusammensetzung in einer Weise zuführen kann, dass das lymphatische System des Tieres erreicht wird.

2. Vorrichtung nach Anspruch 1, wobei der Behälter von der Vorrichtung entfernbar und mit der physiologisch annehmbaren, Antigen-enthaltenden Zusammensetzung, die zum Induzieren und Aufrechterhalten einer CTL-Antwort in einem Tier geeignet ist, wiederbefüllbar ist.

3. Vorrichtung nach Anspruch 1 oder 2, wobei die Zusammensetzung nur ein Antigen umfasst.

4. Vorrichtung nach Anspruch 1 oder 2, wobei die Zusammensetzung mehr als ein Antigen umfasst.

5. Vorrichtung nach einem der vorhergehenden Ansprüche, wobei die Zusammensetzung ferner ein zum Verstärken einer CTL-Antwort geeignetes Zytokin umfasst.

6. Vorrichtung nach Anspruch 5, wobei das Zytokin ausgewählt ist aus der Gruppe bestehend aus GM-CSF, IL-12, IL-2, TNF, IL-18, IL-3, IL-8, IL-9, IL-13, IL-10, IL-14, IL-15, G-CSF, IFN alpha, IFN beta, IFN gamma, TGF alpha und TGF beta.

7. Vorrichtung nach einem der vorhergehenden Ansprüche, wobei das Antigen ausgewählt ist aus der Gruppe bestehend aus einem Differenzierungsantigen, einem Tumorspezifischen Multilineage-Antigen, einem embryonalen Antigen, einem Onkogen-Antigen, einem mutierten Tumorsuppressorgen-Antigen und einem viralen Antigen.

8. Vorrichtung nach Anspruch 7, wobei das Antigen ausgewählt ist aus der Gruppe bestehend aus MART-1/MelanA (MART-1), gp100 (Pmel 17), Tyrosinase, TRP-1, TRP-2, MAGE-1, MAGE-3, BAGE, GAGE-1, GAGE-2, p15(58), CEA, p53, Ras, HER-2/neu, BCR-ABL, E2A-PRL, H4-RET, IGH-IGK, MYL-RAR, EBVA, (HPV-) Antigene E6 und E7, TSP-180, MAGE-4, MAGE-5, MAGE-6, RAGE, NY-ESO, p185erbB2, p18erbB-3, c-met, nm-23H1, PSA, TAG-72, CA19-9, CA 72-4, CAM 17.1, NuMa, K-ras, H-ras, β-Catenin, CDK4, Mum-1, p15 und p16.

9. Vorrichtung nach einem der vorhergehenden Ansprüche, wobei die Übertragungsleitung eine Zuführungsleitung umfasst, deren Größe geeignet ist, in das Tier eingebracht zu werden und die Zusammensetzung in einer Weise zuzuführen, dass das lymphatische System des Tieres erreicht wird.

10. Vorrichtung nach Anspruch 9, wobei die Vorrichtung eine externe Vorrichtung und die Zuführungsleitung ein Katheter ist, der lang genug ist, um dem Tier subkutan oder lymphatisch zugeführt zu werden.

11. Vorrichtung nach Anspruch 10, wobei der Katheter lang genug ist, um direkt in das lymphatische System des Tieres zugeführt zu werden.

12. Vorrichtung nach Anspruch 11, wobei das Zuführen in das lymphatische System durch einen Achsel- oder Leistenknoten erfolgt.

13. Vorrichtung nach einem der vorhergehenden Ansprüche, wobei der Ort, der ein Zuführen der Zusammensetzung ermöglicht, ein Lymphknoten oder ein Lymphgefäß ist.

14. Vorrichtung nach Anspruch 13, wobei der Ort, der ein Zuführen der Zusammensetzung ermöglicht, ein Gebiet von hoher lymphatischer Drainage ist.

15. Vorrichtung nach Anspruch 14, wobei das Gebiet von hoher lymphatischer Drainage durch das Abbilden des lymphatischen Flusses des Tieres ermittelt wird.

16. Vorrichtung nach einem der vorhergehenden Ansprüche, wobei die Vorrichtung tragbar ist.

17. Vorrichtung nach Anspruch 16, wobei die Vorrichtung eine Größe aufweist, die zum tragbaren Befestigen an einem Menschen geeignet ist.

18. Vorrichtung nach einem der vorhergehenden Ansprüche, wobei die Pumpe eine Rollkolbenpumpe / peristaltische Pumpe, eine Spritzenpumpe oder eine Gasdruckpumpe ist.

19. Vorrichtung nach einem der vorhergehenden Ansprüche, wobei die Pumpe batteriebetrieben ist.

20. Vorrichtung nach einem der vorhergehenden Ansprüche, zusammen mit schriftlichen Anleitungen zum Zuführen der Antigenzusammensetzung auf einer regelmäßigen Basis im Zeitverlauf zum Erhalten des Antigens im lymphatischen System des Tieres auf einem Pegel, der zum Erhalten der CTL-Antwort in dem Tier ausreicht.

21. Verfahren zum Herstellen eines Systems, das verwendbar ist, um in einem Tier eine induzierte CTL-Antwort aufrechtzuerhalten oder eine CTL-Antwort zu induzieren und aufrechtzuerhalten, wobei das Tier geeignet ist, von einer solchen Antwort zu profitieren, umfassend:
das Einbringen einer physiologisch annehmbaren, wässrigen, Peptidantigenenthaltenden Zusammensetzung, die frei von herkömmlichen Zusatzstoffen ist und eine ausreichende Menge des Antigens umfasst, welche geeignet ist, in dem Tier einen Antigen-Pegel bereitzustellen, der ausreicht, um in dem Tier gegen das Antigen eine induzierte CTL-Antwort aufrechtzuerhalten oder eine CTL-Antwort zu induzieren und aufrechtzuerhalten, in einen Behälter einer Vorrichtung nach einem der Ansprüche 1 bis 20, wobei die Vorrichtung eine Pumpe zum Zuführen der Zusammensetzung in einer definierten Rate durch eine Übertragungsleitung in das Tier aufweist.

22. Verfahren nach Anspruch 21, wobei
ein für einen Tumor oder ein Pathogen spezifisches Peptidantigen an einen Tumor oder ein Pathogen in dem Tier angeglichen wird, und
eine physiologisch annehmbare Zusammensetzung des angeglichenen Peptidantigens hergestellt wird, bevor die Zusammensetzung in den Behälter gegeben wird.

## Revendications

1. Dispositif pour délivrer un antigène et maintenir une réaction CTL (lymphocyte T cytotoxique) induite ou pour induire et maintenir une réaction CTL chez un animal, ledit dispositif comprenant :
un réservoir rempli d'une composition liquide, physiologiquement acceptable, contenant de l'antigène peptide libre d'adjuvants conventionnels qui comprend une quantité adéquate dudit antigène qui est capable d'assurer un niveau d'antigène chez l'animal suffisant pour maintenir une réaction CTL induite ou pour induire et maintenir une réaction CTL chez ledit animal contre ledit antigène ;
une pompe raccordée au réservoir pour délivrer la composition selon un taux défini ; et
une ligne de transmission pour évacuer la composition du réservoir, la ligne de transmission étant capable de se positionner dans l'animal et pouvant délivrer la composition de manière à atteindre le système lymphatique de l'animal.

2. Dispositif selon la revendication 1, dans lequel le réservoir est amovible du dispositif et peut être de nouveau rempli de ladite composition physiologiquement acceptable contenant de l'antigène, capable d'induire et de maintenir une réaction CTL chez un animal.

3. Dispositif selon la revendication 1 ou 2, dans lequel la composition ne comprend qu'un seul antigène.

4. Dispositif selon la revendication 1 ou 2, dans lequel la composition comprend plus d'un antigène.

5. Dispositif selon l'une quelconque des revendications précédentes, dans lequel la composition comprend, en outre, une cytokine capable d'augmenter une réaction CTL.

6. Dispositif selon la revendication 5, dans lequel la cytokine est choisie dans le groupe consistant en GM-CSF, IL-12, IL-2, TNF, IL-18, IL-3, IL-8, IL-9, IL-13, IL-10, IL-14, IL-15, G-CSF, IFN alpha, IFN bêta, IFN gamma, TGF alpha et TGF bêta.

7. Dispositif selon l'une quelconque des revendications précédentes, dans lequel l'antigène est choisi dans le groupe consistant en un antigène différentiation, un antigène multilignage spécifique d'une tumeur, un antigène embryonnaire, un antigène oncogène, un antigène de gène suspresseur de tumeur muté et un antigène viral.

8. Dispositif selon la revendication 7, dans lequel l'antigène est choisi dans le groupe consistant en MART-1/MelanA (MART-1), gp100 (Pmel 17), tyrosinase, TRP-1, TRP-2, MAGE-1, MAGE-3, BAGE, GAGE-1, GAGE-2, p15(58), CEA, p53, Ras, HER-2/neu, BCR-ABL, E2A-PRL, H4-RET, IGH-IGK, MYL-RAR, EBVA, (HPV) antigènes E6 et E7, TSP-180, MAGE-4, MAGE-5, MAGE-6, RAGE, NY-ESO, p185erbB2, p18erbB-3, c-met, nm-23H1, PSA, TAG-72, CA19-9, CA 72-4, CAM 17.1, NuMa, K-ras, H-ras, β-Catenin, CDK4, Mum-1, p15 et p 16.

9. Dispositif selon l'une quelconque des revendications précédentes, dans lequel ladite ligne de transmission comprend une ligne de fourniture dont la taille est adaptée au positionnement dans l'animal et pour délivrer la composition de manière à atteindre le système lymphatique de l'animal.

10. Dispositif selon la revendication 9, dans lequel le dispositif est un dispositif externe et la ligne de fourniture est un cathéter d'une longueur suffisante pour une fourniture à l'animal de façon sous-cutanée ou lymphatique.

11. Dispositif selon la revendication 10, dans lequel ledit cathéter est d'une longueur suffisante pour une fourniture directe au système lymphatique de l'animal.

12. Dispositif selon la revendication 11, dans lequel la fourniture au système lymphatique s'effectue par un ganglion axillaire ou inguinal.

13. Dispositif selon l'une quelconque des revendications précédentes, dans lequel la position permettant la fourniture de la composition est un ganglion lymphatique ou un vaisseau lymphatique.

14. Dispositif selon la revendication 13, dans lequel la position permettant la fourniture de la composition est une zone de fort drainage lymphatique.

15. Dispositif selon la revendication 14, dans lequel la zone de fort drainage lymphatique est déterminée en imageant le flux lymphatique de l'animal.

16. Dispositif selon l'une quelconque des revendications précédentes, où le dispositif est portable.

17. Dispositif selon la revendication 16, où le dispositif est de taille adaptée pour être attaché de façon portative à un être humain.

18. Dispositif selon l'une quelconque des revendications précédentes, dans lequel la pompe est une pompe péristaltique / à galets, une pompe seringue ou une pompe à pression de gaz.

19. Dispositif selon l'une quelconque des revendications précédentes, dans lequel la pompe fonctionne grâce à une batterie.

20. Dispositif selon l'une quelconque des revendications précédentes, combiné avec des instructions écrites pour la fourniture de la composition d'antigène sur une base régulière dans le temps pour maintenir l'antigène dans le système lymphatique de l'animal à un niveau suffisant pour maintenir une réaction CTL chez l'animal.

21. Procédé pour préparer un système utile pour maintenir une réaction CTL induite ou pour induire et maintenir une réaction CTL chez un animal apte à bénéficier d'une telle réaction, qui comprend :
le placement d'une composition physiologiquement acceptable, aqueuse, contenant de l'antigène peptide, libre d'adjuvants conventionnels, qui comprend une quantité adéquate dudit antigène capable de fournir un niveau d'antigène chez ledit animal suffisant pour maintenir une réaction CTL induite ou pour induire et maintenir une réaction CTL chez ledit animal contre ledit antigène, dans un réservoir d'un dispositif selon l'une quelconque des revendications 1 à 20, ledit dispositif possédant une pompe pour fournir la composition à un taux défini au travers d'une ligne de transmission à l'animal.

22. Procédé selon la revendication 21, dans lequel
un antigène peptide spécifique à une tumeur ou un pathogène est ajusté à une tumeur ou un pathogène dans l'animal et
une composition physiologiquement acceptable de l'antigène peptide ajusté est préparée avant que la composition soit placée dans le réservoir.


### SEQUENCE LISTING

<110> Kuendig,.Thomas M.
   Simard, John J. L.
<120> A Method of Inducing a CTL Response
<130> CTLI-001/02WO
<140> Not yet assigned
   <141> 1998-07-10
<150> 08/988,320US
   <151> 1997-12-10
<150> 2,209,815CA
   <151> 1997-07-10
<160> 569
<170> PatentIn Ver. 2.0
<210> 1
   <211> 9
   <212> PRT
   <213> Adenovirus 3
<400> 1
   Leu Ile Val Ile Gly Ile Leu Ile Leu
   1 5
<210> 2
   <211> 10
   <212> PRT
   <213> Adenovirus 5
<400> 2
   Ser Gly Pro Ser Asn Thr Pro Pro Glu Ile
   1 5 10
<210> 3
   <211> 9
   <212> PRT
   <213> Adenovirus 5
<400> 3
   Val Asn Ile Arg Asn Cys Cys Tyr Ile
   1 5
<210> 4
   <211> 10
   <212> PRT
   <213> Adenovirus 5
<400> 4
   Ser Gly Pro Ser Asn Ile Pro Pro Glu Ile
   1 5 10
<210> 5
   <211> 9
   <212> PRT
   <213> CSFV
<400> 5
   Glu Asn Ala Leu Leu Val Ala Leu Phe
   1 5
<210> 6
   <211> 9
   <212> PRT
   <213> Dengue virus 4
<400> 6
   Thr Pro Glu Gly Ile Ile Pro Thr Leu
   1 5
<210> 7
   <211> 9
   <212> PRT
   <213> EBV
<400> 7
   Cys Leu Gly Gly Leu Leu Thr Met Val
   1 5
<210> 8
   <211> 9
   <212> PRT
   <213> EBV
<400> 8
   Asn Ile Ala Glu Gly Leu Arg Ala Leu
   1 5
<210> 9
   <211> 9
   <212> PRT
   <213> EBV
<400> 9
   Asn Leu Arg Arg Gly Thr Ala Leu Ala
   1 5
<210> 10
   <211> 9
   <212> PRT
   <213> EBV
<400> 10
   Ala Leu Ala Ile Pro Gln Cys Arg Leu
   1 5
<210> 11
   <211> 9
   <212> PRT
   <213> EBV
<400> 11
   Val Leu Lys Asp Ala Ile Lys Asp Leu
   1 5
<210> 12
   <211> 9
   <212> PRT
   <213> EBV
<400> 12
   Phe Met Val Phe Leu Gln Thr His Ile
   1 5
<210> 13
   <211> 9
   <212> PRT
   <213> EBV
<400> 13
   His Leu Ile Val Asp Thr Asp Ser Leu
   1 5
<210> 14
   <211> 9
   <212> PRT
   <213> EBV
<400> 14
   Ser Leu Gly Asn Pro Ser Leu Ser Val
   1 5
<210> 15
   <211> 9
   <212> PRT
   <213> EBV
<400> 15
   Pro Leu Ala Ser Ala Met Arg Met Leu
   1 5
<210> 16
   <211> 9
   <212> PRT
   <213> EBV
<400> 16
   Arg Met Leu Trp Met Ala Asn Tyr Ile
   1 5
<210> 17
   <211> 9
   <212> PRT
   <213> EBV
<400> 17
   Met Leu Trp Met Ala Asn Tyr Ile Val
   1 5
<210> 18
   <211> 9
   <212> PRT
   <213> EBV
<400> 18
   Ile Leu Pro Gln Gly Pro Gln Thr Ala
   1 5
<210> 19
   <211> 9
   <212> PRT
   <213> EBV
<400> 19
   Pro Leu Arg Pro Thr Ala Pro Thr Ile
   1 5
<210> 20
   <211> 9
   <212> PRT
   <213> EBV
<400> 20
   Pro Leu Pro Pro Ala Thr Leu Thr Val
   1 5
<210> 21
   <211> 9
   <212> PRT
   <213> EBV
<400> 21
   Arg Met His Leu Pro Val Leu His Val
   1 5
<210> 22
   <211> 9
   <212> PRT
   <213> EBV
<400> 22
   Pro Met Pro Leu Pro Pro Ser Gln Leu
   1 5
<210> 23
   <211> 9
   <212> PRT
   <213> EBV
<400> 23
   Gln Leu Pro Pro Pro Ala Ala Pro Ala
   1 5
<210> 24
   <211> 9
   <212> PRT
   <213> EBV
<400> 24
   Ser Met Pro Glu Leu Ser Pro Val Leu
   1 5
<210> 25
   <211> 9
   <212> PRT
   <213> EBV
<400> 25
   Asp Leu Asp Glu Ser Trp Asp Tyr Ile
   1 5
<210> 26
   <211> 9
   <212> PRT
   <213> EBV
<400> 26
   Pro Leu Pro Cys Val Leu Trp Pro Val
   1 5
<210> 27
   <211> 9
   <212> PRT
   <213> EBV
<400> 27
   Ser Leu Glu Glu Cys Asp Ser Glu Leu
   1 5
<210> 28
   <211> 9
   <212> PRT
   <213> EBV
<400> 28
   Glu Ile Lys Arg Tyr Lys Asn Arg Val
   1 5
<210> 29
   <211> 9
   <212> PRT
   <213> EBV
<400> 29
   Gln Leu Leu Gln His Tyr Arg Glu Val
   1 5
<210> 30
   <211> 9
   <212> PRT
   <213> HCV-1
<400> 30
   Leu Leu Gln His Tyr Arg Glu Val Ala
   1 5
<210> 31
   <211> 9
   <212> PRT
   <213> EBV
<400> 31
   Leu Leu Lys Gln Met Cys Pro Ser Leu
   1 5
<210> 32
   <211> 9
   <212> PRT
   <213> EBV
<400> 32
   Ser Ile Ile Pro Arg Thr Pro Asp Val
   1 5
<210> 33
   <211> 10
   <212> PRT
   <213> EBV
<400> 33
   Leu Leu Asp Phe Val Arg Phe Met Gly Val
   1 5 10
<210> 34
   <211> 9
   <212> PRT
   <213> EBV
<400> 34
   Ser Val Arg Asp Arg Leu Ala Arg Leu
   1 5
<210> 35
   <211> 9
   <212> PRT
   <213> EBV
<400> 35
   Ile Val Thr Asp Phe Ser Val Ile Lys
   1 5
<210> 36
   <211> 10
   <212> PRT
   <213> EBV
<400> 36
   Ala Val Phe Asp Arg Lys Ser Asp Ala Lys
   1 5 10
<210> 37
   <211> 8
   <212> PRT
   <213> EBV
<400> 37
   Arg Tyr Ser Ile Phe Phe Asp Tyr
   1 5
<210> 38
   <211> 9
   <212> PRT
   <213> EBV
<400> 38
   Gln Pro Arg Ala Pro Ile Arg Pro Ile
   1 5
<210> 39
   <211> 9
   <212> PRT
   <213> EBV
<400> 39
   Arg Pro Pro Ile Phe Ile Arg Arg Leu
   1 5
<210> 40
   <211> 9
   <212> PRT
   <213> EBV
<400> 40
   Glu Pro Asp Val Pro Pro Gly Ala Ile
   1 5
<210> 41
   <211> 9
   <212> PRT
   <213> EBV
<400> 41
   Ile Pro Gln Cys Arg Leu Thr Pro Leu
   1 5
<210> 42
   <211> 9
   <212> PRT
   <213> EBV
<400> 42
   Gly Pro Gly Pro Gln Pro Gly Pro Leu
   1 5
<210> 43
   <211> 9
   <212> PRT
   <213> EBV
<400> 43
   Gln Pro Gly Pro Leu Glu Arg Ser Ile
   1 5
<210> 44
   <211> 9
   <212> PRT
   <213> EBV
<400> 44
   Arg Pro Gln Lys Arg Pro Ser Cys Ile
   1 5
<210> 45
   <211> 9
   <212> PRT
   <213> EBV
<400> 45
   Pro Pro Thr Pro Leu Leu Thr Val Leu
   1 5
<210> 46
   <211> 9
   <212> PRT
   <213> EBV
<400> 46
   Thr Pro Ser Pro Pro Arg Met His Leu
   1 5
<210> 47
   <211> 9
   <212> PRT
   <213> EBV
<400> 47
   Pro Pro Arg Met His Leu Pro Val Leu
   1 5
<210> 48
   <211> 9
   <212> PRT
   <213> EBV
<400> 48
   Val Pro Asp Gln Ser Met His Pro Leu
   1 5
<210> 49
   <211> 9
   <212> PRT
   <213> EBV
<400> 49
   Pro Pro Ser Ile Asp Pro Ala Asp Leu
   1 5
<210> 50
   <211> 9
   <212> PRT
   <213> EBV
<400> 50
   Leu Pro Cys Val Leu Trp Pro Val Leu
   1 5
<210> 51
   <211> 10
   <212> PRT
   <213> EBV
<400> 51
   Cys Pro Ser Leu Asp Val Asp Ser Ile Ile
   1 5 10
<210> 52
   <211> 9
   <212> PRT
   <213> EBV
<400> 52
   Thr Pro Asp Val Leu His Glu Asp Leu
   1 5
<210> 53
   <211> 9
   <212> PRT
   <213> EBV
<400> 53
   Phe Leu Arg Gly Arg Ala Tyr Gly Leu
   1 5
<210> 54
   <211> 9
   <212> PRT
   <213> EBV
<400> 54
   Gln Ala Lys Trp Arg Leu Gln Thr Leu
   1 5
<210> 55
   <211> 9
   <212> PRT
   <213> EBV
<400> 55
   Ala Tyr Pro Leu His Glu Gln His Gly
   1 5
<210> 56
   <211> 9
   <212> PRT
   <213> EBV
<400> 56
   Tyr Ile Lys Ser Phe Val Ser Asp Ala
   1 5
<210> 57
   <211> 9
   <212> PRT
   <213> EBV
<400> 57
   Arg Arg Arg Trp Arg Arg Leu Thr Val
   1 5
<210> 58
   <211> 9
   <212> PRT
   <213> Hepatitis C virus
<400> 58
   Arg Arg Ile Tyr Asp Leu Ile Glu Leu
   1 5
<210> 59
   <211> 9
   <212> PRT
   <213> EBV
<400> 59
   Tyr Pro Leu His Glu Gln His Gly Met
   1 5
<210> 60
   <211> 9
   <212> PRT
   <213> Hepatitis C virus
<400> 60
   His Ser Lys Lys Lys Cys Asp Glu Leu
   1 5
<210> 61
   <211> 8
   <212> PRT
   <213> Hepatitis C virus
<400> 61
   Ala Ser Arg Cys Trp Val Ala Met
   1 5
<210> 62
   <211> 9
   <212> PRT
   <213> Hepatitis C virus
<400> 62
   Gly Gln Ile Val Gly Gly Val Tyr Leu
   1 5
<210> 63
   <211> 10
   <212> PRT
   <213> Hepatitis C virus
<400> 63
   Arg Pro Leu Thr Asp Phe Asp Gln Gly Trp
   1 5 10
<210> 64
   <211> 10
   <212> PRT
   <213> Hepatitis C virus
<400> 64
   Leu Met Gly Tyr Ile Pro Leu Val Gly Ala
   1 5 10
<210> 65
   <211> 10
   <212> PRT
   <213> Hepatitis C virus
<400> 65
   Ala Asp Leu Met Gly Tyr Ile Pro Leu Val
   1 5 10
<210> 66
   <211> 16
   <212> PRT
   <213> Hepatitis C virus
<400> 66
   Met Ser Tyr Ser Trp Thr Gly Ala Leu Val Thr Pro Cys Ala Glu Glu
   1 5 10 15
<210> 67
   <211> 9
   <212> PRT
   <213> Hepatitis C virus
<400> 67
   Lys His Pro Asp Ala Thr Tyr Ser Arg
   1 5
<210> 68
   <211> 10
   <212> PRT
   <213> Hepatitis C virus
<400> 68
   Lys Leu Val Ala Leu Gly Ile Asn Ala Val
   1 5 10
<210> 69
   <211> 9
   <212> PRT
   <213> Hepatitis C virus
<400> 69
   Gly Asp Phe Asp Ser Val Ile Asp Cys
   1 5
<210> 70
   <211> 9
   <212> PRT
   <213> Hepatitis C virus
<400> 70
   Gly Asn Ala Ser Arg Cys Trp Val Ala
   1 5
<210> 71
   <211> 9
   <212> PRT
   <213> Hepatitis C virus
<400> 71
   Thr Arg Pro Pro Leu Gly Asn Trp Phe
   1               5
<210> 72
   <211> 9
   <212> PRT
   <213> Hepatitis C virus
<400> 72
   Val Pro His Pro Asn Ile Glu Glu Val
   1               5
<210> 73
   <211> 9
   <212> PRT
   <213> Hepatitis C virus
<400> 73
   Tyr Thr Gly Asp Phe Asp Ser Val Ile
   1               5
<210> 74
   <211> 8
   <212> PRT
   <213> Hepatitis C virus
<400> 74
   Ser Trp Ala Ile Lys Trp Glu Tyr
   1               5
<210> 75
   <211> 9
   <212> PRT
   <213> Hepatitis C virus
<400> 75
   Lys His Pro Asp Ala Thr Tyr Ser Arg
   1               5
<210> 76
   <211> 9
   <212> PRT
   <213> Hepatitis C virus
<400> 76
   Gly Asp Phe Asp Ser Val Ile Asp Cys
   1 5
<210> 77
   <211> 9
   <212> PRT
   <213> Human immunodeficiency virus
<400> 77
   Arg Tyr Leu Lys Asp Gln Gln Leu Leu
   1 5
<210> 78
   <211> 9
   <212> PRT
   <213> Human immunodeficiency virus
<400> 78
   Ile Val Gly Leu Asn Lys Ile Val Arg
   1 5
<210> 79
   <211> 9
   <212> PRT
   <213> Human immunodeficiency virus
<400> 79
   Glu Ile Tyr Lys Arg Trp Ile Ile Leu
   1 5
<210> 80
   <211> 9
   <212> PRT
   <213> Human immunodeficiency virus
<400> 80
   Gly Glu Ile Tyr Lys Arg Trp Ile Ile
   1 5
<210> 81
   <211> 9
   <212> PRT
   <213> Human immunodeficiency virus
<400> 81
   Glu Ile Lys Asp Thr Lys Glu Ala Leu
   1 5
<210> 82
   <211> 8
   <212> PRT
   <213> Human immunodeficiency virus
<400> 82
   Tyr Leu Lys Asp Gln Gln Leu Leu
   1 5
<210> 83
   <211> 11
   <212> PRT
   <213> Human immunodeficiency virus
<400> 83
   Ile Leu Gly Leu Asn Lys Ile Val Arg Met Tyr
   1 5 10
<210> 84
   <211> 9
   <212> PRT
   <213> Human immunodeficiency virus
<400> 84
   Glu Arg Tyr Leu Lys Asp Gln Gln Leu
   1 5
<210> 85
   <211> 11
   <212> PRT
   <213> Human immunodeficiency virus
<400> 85
   Tyr His Thr Gln Gly Tyr Phe Pro Gln Trp Gln
   1 5 10
<210> 86
   <211> 12
   <212> PRT
   <213> Human immunodeficiency virus
<400> 86
   Thr Gln Gly Tyr Phe Pro Gln Trp Gln Asn Tyr Thr
   1 5 10
<210> 87
   <211> 9
   <212> PRT
   <213> Human immunodeficiency virus
<400> 87
   Gly Arg Ala Phe Val Thr Ile Gly Lys
   1 5
<210> 88
   <211> 9
   <212> PRT
   <213> Human immunodeficiency virus
<400> 88
   Lys Arg Trp Ile Ile Leu Gly Leu Asn
   1 5
<210> 89
   <211> 10
   <212> PRT
   <213> Human immunodeficiency virus
<400> 89
   Gln Val Pro Leu Arg Pro Met Thr Tyr Lys
   1 5 10
<210> 90
   <211> 9
   <212> PRT
   <213> Human immunodeficiency virus
<400> 90
   Thr Gln Gly Tyr Phe Pro Gln Trp Gln
   1 5
<210> 91
   <211> 9
   <212> PRT
   <213> Human immunodeficiency virus
<400> 91
   His Gln Ala Ile Ser Pro Arg Thr Leu
   1 5
<210> 92
   <211> 12
   <212> PRT
   <213> Human immunodeficiency virus
<400> 92
   Gln Met Val His Gln Ala Ile Ser Pro Arg Thr Leu
   1 5 10
<210> 93
   <211> 10
   <212> PRT
   <213> Human immunodeficiency virus
<400> 93
   Met Tyr Ala Pro Pro Ile Gly Gly Gln Ile
   1 5 10
<210> 94
   <211> 10
   <212> PRT
   <213> Human immunodeficiency virus
<400> 94
   Arg Gly Pro Gly Arg Ala Phe Val Thr Ile
   1 5 10
<210> 95
   <211> 9
   <212> PRT
   <213> Human immunodeficiency virus
<400> 95
   Met Pro Gly Arg Ala Phe Val Thr Ile
   1 5
<210> 96
   <211> 9
   <212> PRT
   <213> Human immunodeficiency virus type 1
<400> 96
   Ile Leu Lys Glu Pro Val His Gly Val
   1 5
<210> 97
   <211> 9
   <212> PRT
   <213> Human immunodeficiency virus type 1
<400> 97
   Ala Phe His His Val Ala Arg Glu Leu
   1 5
<210> 98
   <211> 9
   <212> PRT
   <213> Human immunodeficiency virus type 1
<400> 98
   Lys Leu Thr Pro Leu Cys Val Thr Leu
   1 5
<210> 99
   <211> 10
   <212> PRT
   <213> Human immunodeficiency virus type 1
<400> 99
   Ser Leu Leu Asn Ala Thr Asp Ile Ala Val
   1 5 10
<210> 100
   <211> 9
   <212> PRT
   <213> Human immunodeficiency virus type 1
<400> 100
   Val Ile Tyr Gln Tyr Met Asp Asp Leu
   1 5
<210> 101
   <211> 9
   <212> PRT
   <213> Human immunodeficiency virus type 1
<400> 101
   Ser Leu Tyr Asn Thr Val Ala Thr Leu
   1 5
<210> 102
   <211> 10
   <212> PRT
   <213> Human immunodeficiency virus type 1
<400> 102
   Arg Gly Pro Gly Arg Ala Phe Val Thr Ile
   1 5 10
<210> 103
   <211> 11
   <212> PRT
   <213> Human immunodeficiency virus type 1
<400> 103
   Arg Leu Arg Asp Leu Leu Leu Ile Val Thr Arg
   1 5 10
<210> 104
   <211> 10
   <212> PRT
   <213> Human immunodeficiency virus type 1
<400> 104
   Gln Val Pro Leu Arg Pro Met Thr Tyr Lys
   1 5 10
<210> 105
   <211> 10
   <212> PRT
   <213> Human immunodeficiency virus type 1
<400> 105
   Thr Val Tyr Tyr Gly Val Pro Val Trp Lys
   1 5 10
<210> 106
   <211> 9
   <212> PRT
   <213> Human immunodeficiency virus type 1
<400> 106
   Arg Leu Arg Pro Gly Gly Lys Lys Lys
   1 5
<210> 107
   <211> 9
   <212> PRT
   <213> Human immunodeficiency virus type 1
<400> 107
   Val Tyr Tyr Gly Val Pro Val Trp Lys
   1 5
<210> 108
   <211> 9
   <212> PRT
   <213> Human immunodeficiency virus type 1
<400> 108
   Val Pro Leu Arg Pro Met Thr Tyr Lys
   1 5
<210> 109
   <211> 9
   <212> PRT
   <213> Human immunodeficiency virus type 1
<400> 109
   Ala Ile Phe Gln Ser Ser Met Thr Lys
   1 5
<210> 110
   <211> 12
   <212> PRT
   <213> Human immunodeficiency virus type 1
<400> 110
   Ala Ala Val Asp Leu Ser His Phe Leu Lys Glu Lys
   1 5 10
<210> 111
   <211> 12
   <212> PRT
   <213> Human immunodeficiency virus type 1
<400> 111
   Ala Cys Gln Gly Val Gly Gly Pro Gly Gly His Lys
   1 5 10
<210> 112
   <211> 10
   <212> PRT
   <213> Human immunodeficiency virus type 1
<400> 112
   Glu Thr Ile Asn Glu Glu Ala Ala Glu Trp
   1 5 10
<210> 113
   <211> 10
   <212> PRT
   <213> Human immunodeficiency virus type 1
<400> 113
   Thr Pro Gly Pro Gly Val Arg Tyr Pro Leu
   1 5 10
<210> 114
   <211> 8
   <212> PRT
   <213> Human immunodeficiency virus type 1
<400> 114
   Gly Gly Lys Lys Lys Tyr Lys Leu
   1 5
<210> 115
   <211> 9
   <212> PRT
   <213> Human immunodeficiency virus type 1
<400> 115
   Arg Val Lys Glu Lys Tyr Gln His Leu
   1 5
<210> 116
   <211> 9
   <212> PRT
   <213> Human immunodeficiency virus type 1
<400> 116
   Asp Arg Phe Tyr Lys Thr Leu Arg Ala
   1 5
<210> 117
   <211> 16
   <212> PRT
   <213> Human immunodeficiency virus type 1
<400> 117
   Gly Val Arg Tyr Pro Leu Thr Phe Gly Trp Cys Tyr Lys Leu Val Pro
   1 5 10 15
<210> 118
   <211> 10
   <212> PRT
   <213> Human immunodeficiency virus type 1
<400> 118
   Lys Arg Trp Ile Ile Leu Gly Leu Asn Lys
   1 5 10
<210> 119
   <211> 9
   <212> PRT
   <213> EBV
<400> 119
   Lys Glu His Val Ile Gln Asn Ala Phe
   1 5
<210> 120
   <211> 10
   <212> PRT
   <213> EBV
<400> 120
   Glu Glu Asn Leu Leu Asp Phe Val Arg Phe
   1 5 10
<210> 121
   <211> 10
   <212> PRT
   <213> EBV
<400> 121
   Asp Thr Pro Leu Ile Pro Leu Thr Ile Phe
   1 5 10
<210> 122
   <211> 10
   <212> PRT
   <213> EBV
<400> 122
   Gln Asn Gly Ala Leu Ala Ile Asn Thr Phe
   1 5 10
<210> 123
   <211> 9
   <212> PRT
   <213> EBV
<400> 123
   Arg Leu Arg Ala Glu Ala Gly Val Lys
   1 5
<210> 124
   <211> 10
   <212> PRT
   <213> Hepatitis B virus
<400> 124
   Gly Leu Ser Pro Thr Val Trp Leu Ser Val
   1 5 10
<210> 125
   <211> 9
   <212> PRT
   <213> Hepatitis B virus
<400> 125
   Trp Leu Ser Leu Leu Val Pro Phe Val
   1 5
<210> 126
   <211> 10
   <212> PRT
   <213> Hepatitis B virus
<400> 126
   Phe Leu Pro Ser Asp Phe Phe Pro Ser Val
   1 5 10
<210> 127
   <211> 9
   <212> PRT
   <213> Hepatitis B virus
<400> 127
   Phe Leu Leu Ser Leu Gly Ile His Leu
   1 5
<210> 128
   <211> 9
   <212> PRT
   <213> Hepatitis B virus
<400> 128
   Ser Leu Tyr Ala Asp Ser Pro Ser Val
   1 5
<210> 129
   <211> 9
   <212> PRT
   <213> Hepatitis B virus
<400> 129
   Gly Leu Ser Arg Tyr Val Ala Arg Leu
   1 5
<210> 130
   <211> 10
   <212> PRT
   <213> Hepatitis B virus
<400> 130
   Leu Leu Val Pro Phe Val Gln Trp Phe Val
   1 5 10
<210> 131
   <211> 9
   <212> PRT
   <213> Hepatitis B virus
<400> 131
   Ala Leu Met Pro Leu Tyr Ala Cys Ile
   1 5
<210> 132
   <211> 10
   <212> PRT
   <213> Hepatitis B virus
<400> 132
   Leu Leu Pro Ile Phe Phe Cys Leu Trp Val
   1 5 10
<210> 133
   <211> 9
   <212> PRT
   <213> Hepatitis B virus
<400> 133
   Tyr Met Asp Asp Val Val Leu Gly Ala
   1 5
<210> 134
   <211> 9
   <212> PRT
   <213> Hepatitis B virus
<400> 134
   Leu Leu Leu Cys Leu Ile Phe Leu Leu
   1 5
<210> 135
   <211> 10
   <212> PRT
   <213> Hepatitis B virus
<400> 135
   Leu Leu Asp Tyr Gln Gly Met Leu Pro Val
   1 5 10
<210> 136
   <211> 10
   <212> PRT
   <213> Hepatitis B virus
<400> 136
   Ser Ile Val Ser Pro Phe Ile Pro Leu Leu
   1 5 10
<210> 137
   <211> 9
   <212> PRT
   <213> Hepatitis B virus
<400> 137
   Phe Leu Leu Thr Arg Ile Leu Thr Ile
   1 5
<210> 138
   <211> 9
   <212> PRT
   <213> Hepatitis B virus
<400> 138
   Tyr Val Asn Val Asn Met Gly Leu Lys
   1 5
<210> 139
   <211> 11
   <212> PRT
   <213> Hepatitis B virus
<400> 139
   Ser Thr Leu Pro Glu Thr Thr Val Val Arg Arg
   1 5 10
<210> 140
   <211> 12
   <212> PRT
   <213> Hepatitis B virus
<400> 140
   Ile Pro Gln Ser Leu Asp Ser Trp Trp Thr Ser Leu
   1 5 10
<210> 141
   <211> 8
   <212> PRT
   <213> Hepatitis B virus
<400> 141
   Met Gly Leu Lys Phe Arg Gln Leu
   1 5
<210> 142
   <211> 9
   <212> PRT
   <213> Hepatitis B virus
<400> 142
   Ser Thr Asx Xaa Gln Ser Gly Xaa Gln
   1 5
<210> 143
   <211> 11
   <212> PRT
   <213> Human cytomegalovirus
<400> 143
   Phe Ile Ala Gly Asn Ser Ala Tyr Glu Tyr Val
   1 5 10
<210> 144
   <211> 12
   <212> PRT
   <213> Human cytomegalovirus
<400> 144
   Ser Asp Glu Glu Glu Ala Ile Val Ala Tyr Thr Leu
   1 5 10
<210> 145
   <211> 15
   <212> PRT
   <213> Human cytomegalovirus
<400> 145
   Asp Asp Val Trp Thr Ser Gly Ser Asp Ser Asp Glu Glu Leu Val
   1 5 10 15
<210> 146
   <211> 9
   <212> PRT
   <213> Human cytomegalovirus
<400> 146
   Ile Pro Ser Ile Asn Val His His Tyr
   1 5
<210> 147
   <211> 10
   <212> PRT
   <213> Human cytomegalovirus
<400> 147
   Asn Leu Val Pro Met Val Ala Thr Val Gln
   1 5 10
<210> 148
   <211> 15
   <212> PRT
   <213> Human cytomegalovirus
<400> 148
   Arg Lys Thr Pro Arg Val Thr Gly Gly Gly Ala Met Ala Gly Ala
   1 5 10 15
<210> 149
   <211> 9
   <212> PRT
   <213> Hepatitis C virus
<400> 149
   Asp Leu Met Gly Tyr Ile Pro Leu Val
   1 5
<210> 150
   <211> 10
   <212> PRT
   <213> Hepatitis C virus
<400> 150
   Leu Leu Ala Leu Leu Ser Cys Leu Thr Val
   1 5 10
<210> 151
   <211> 8
   <212> PRT
   <213> Hepatitis C virus
<400> 151
   Ile Leu His Thr Pro Gly Cys Val
   1 5
<210> 152
   <211> 10
   <212> PRT
   <213> Hepatitis C virus
<400> 152
   Gln Leu Arg Arg His Ile Asp Leu Leu Val
   1 5 10
<210> 153
   <211> 9
   <212> PRT
   <213> Hepatitis C virus
<400> 153
   Asp Leu Cys Gly Ser Val Phe Leu Val
   1 5
<210> 154
   <211> 9
   <212> PRT
   <213> Hepatitis C virus
<400> 154
   Ser Met Val Gly Asn Trp Ala Lys Val
   1 5
<210> 155
   <211> 9
   <212> PRT
   <213> Hepatitis C virus
<400> 155
   His Leu His Gln Asn Ile Val Asp Val
   1 5
<210> 156
   <211> 9
   <212> PRT
   <213> Hepatitis C virus
<400> 156
   Phe Leu Leu Leu Ala Asp Ala Arg Val
   1 5
<210> 157
   <211> 13
   <212> PRT
   <213> Hepatitis C virus
<400> 157
   Gly Leu Arg Asp Leu Ala Val Ala Val Glu Pro Val Val
   1 5 10
<210> 158
   <211> 11
   <212> PRT
   <213> Hepatitis C virus
<400> 158
   Ser Leu Leu Ala Pro Gly Ala Lys Gln Asn Val
   1 5 10
<210> 159
   <211> 10
   <212> PRT
   <213> Hepatitis C virus
<400> 159
   Leu Leu Ala Pro Gly Ala Lys Gln Asn Val
   1 5 10
<210> 160
   <211> 10
   <212> PRT
   <213> Hepatitis C virus
<400> 160
   Leu Leu Phe Asn Ile Leu Gly Gly Trp Val
   1 5 10
<210> 161
   <211> 9
   <212> PRT
   <213> Hepatitis C virus
<400> 161
   Tyr Leu Val Ala Tyr Gln Ala Thr Val
   1 5
<210> 162
   <211> 10
   <212> PRT
   <213> Hepatitis C virus
<400> 162
   Tyr Leu Leu Pro Arg Arg Gly Pro Arg Leu
   1 5 10
<210> 163
   <211> 10
   <212> PRT
   <213> Hepatitis C virus
<400> 163
   Leu Leu Ala Leu Leu Ser Cys Leu Thr Ile
   1 5 10
<210> 164
   <211> 9
   <212> PRT
   <213> Hepatitis C virus
<400> 164
   Ser Leu Met Ala Phe Thr Ala Ala Val
   1 5
<210> 165
   <211> 9
   <212> PRT
   <213> Hepatitis C virus
<400> 165
   Cys Ile Asn Gly Val Cys Trp Thr Val
   1 5
<210> 166
   <211> 9
   <212> PRT
   <213> Hepatitis C virus
<400> 166
   Leu Leu Cys Pro Ala Gly His Ala Val
   1 5
<210> 167
   <211> 9
   <212> PRT
   <213> Hepatitis C virus
<400> 167
   Ile Leu Asp Ser Phe Asp Pro Leu Val
   1 5
<210> 168
   <211> 9
   <212> PRT
   <213> Hepatitis C virus
<400> 168
   Ile Leu Ala Gly Tyr Gly Ala Gly Val
   1 5
<210> 169
   <211> 9
   <212> PRT
   <213> Hepatitis C virus
<400> 169
   Gly Leu Gln Asp Cys Thr Met Leu Val
   1 5
<210> 170
   <211> 10
   <212> PRT
   <213> Hepatitis C virus
<400> 170
   Thr Gly Ala Pro Val Thr Tyr Ser Thr Tyr
   1 5 10
<210> 171
   <211> 9
   <212> PRT
   <213> Hepatitis C virus
<400> 171
   His Met Trp Asn Phe Ile Ser Gly Ile
   1 5
<210> 172
   <211> 9
   <212> PRT
   <213> Hepatitis C virus
<400> 172
   Arg Val Cys Glu Lys Met Ala Leu Tyr
   1 5
<210> 173
   <211> 8
   <212> PRT
   <213> Hepatitis C virus
<400> 173
   Thr Ile Asn Tyr Thr Ile Phe Lys
   1 5
<210> 174
   <211> 8
   <212> PRT
   <213> Hepatitis C virus
<400> 174
   Tyr Ile Ser Trp Cys Leu Trp Trp
   1 5
<210> 175
   <211> 9
   <212> PRT
   <213> Hepatitis C virus
<400> 175
   Gly Pro Arg Leu Gly Val Arg Ala Thr
   1 5
<210> 176
   <211> 9
   <212> PRT
   <213> Human immunodeficiency virus type 1
<400> 176
   Ser Phe Asn Cys Gly Gly Glu Phe Phe
   1 5
<210> 177
   <211> 9
   <212> PRT
   <213> Human immunodeficiency virus type 1
<400> 177
   Thr Glu Met Glu Lys Glu Gly Lys Ile
   1 5
<210> 178
   <211> 9
   <212> PRT
   <213> Human immunodeficiency virus type 1
<400> 178
   Lys Ile Arg Leu Arg Pro Gly Gly Lys
   1 5
<210> 179
   <211> 10
   <212> PRT
   <213> Human immunodeficiency virus type 1
<400> 179
   Arg Leu Arg Pro Gly Gly Lys Lys Lys Tyr
   1 5 10
<210> 180
   <211> 9
   <212> PRT
   <213> Human immunodeficiency virus type 1
<400> 180
   Ala Ile Phe Gln Ser Ser Met Thr Lys
   1 5
<210> 181
   <211> 9
   <212> PRT
   <213> Human immunodeficiency virus type 1
<400> 181
   Thr Leu Tyr Cys Val His Gln Arg Ile
   1 5
<210> 182
   <211> 10
   <212> PRT
   <213> Human immunodeficiency virus type 1
<400> 182
   Ile Tyr Gln Glu Pro Phe Lys Asn Leu Lys
   1 5 10
<210> 183
   <211> 9
   <212> PRT
   <213> Human immunodeficiency virus type 1
<400> 183
   Lys Tyr Lys Leu Lys His Ile Val Trp
   1 5
<210> 184
   <211> 10
   <212> PRT
   <213> Human immunodeficiency virus type 1
<400> 184
   Leu Phe Cys Ala Ser Asp Ala Lys Ala Tyr
   1 5 10
<210> 185
   <211> 11
   <212> PRT
   <213> Human immunodeficiency virus type 1
<400> 185
   Gln Ala Ile Ser Pro Arg Thr Leu Asn Ala Trp
   1 5 10
<210> 186
   <211> 9
   <212> PRT
   <213> Human immunodeficiency virus type 1
<400> 186
   Glu Val Ile Pro Met Phe Ser Ala Leu
   1 5
<210> 187
   <211> 11
   <212> PRT
   <213> Human immunodeficiency virus type 1
<400> 187
   Glu Thr Phe Tyr Val Asp Gly Ala Ala Asn Arg
   1 5 10
<210> 188
   <211> 11
   <212> PRT
   <213> Human immunodeficiency virus type 1
<400> 188
   Arg Leu Arg Asp Leu Leu Leu Ile Val Thr Arg
   1 5 10
<210> 189
   <211> 10
   <212> PRT
   <213> Human immunodeficiency virus type 1
<400> 189
   Pro Ile Gln Lys Glu Thr Trp Glu Thr Trp
   1 5 10
<210> 190
   <211> 9
   <212> PRT
   <213> Human immunodeficiency virus type 1
<400> 190
   Arg Ile Lys Gln Ile Ile Asn Met Trp
   1 5
<210> 191
   <211> 9
   <212> PRT
   <213> Human immunodeficiency virus type 1
<400> 191
   Ile Thr Leu Trp Gln Arg Pro Leu Val
   1 5
<210> 192
   <211> 9
   <212> PRT
   <213> Human immunodeficiency virus type 1
<400> 192
   Asp Thr Val Leu Glu Glu Met Asn Leu
   1 5
<210> 193
   <211> 9
   <212> PRT
   <213> Human immunodeficiency virus type 1
<400> 193
   Ile Thr Leu Trp Gln Arg Pro Leu Val
   1 5
<210> 194
   <211> 9
   <212> PRT
   <213> Human immunodeficiency virus type 1
<400> 194
   Ser Pro Arg Thr Leu Asn Ala Trp Val
   1 5
<210> 195
   <211> 9
   <212> PRT
   <213> Human immunodeficiency virus type 1
<400> 195
   Ala Thr Pro Gln Asp Leu Asn Thr Met
   1 5
<210> 196
   <211> 10
   <212> PRT
   <213> Human immunodeficiency virus type 1
<400> 196
   Arg Pro Asn Asn Asn Thr Arg Lys Ser Ile
   1 5 10
<210> 197
   <211> 9
   <212> PRT
   <213> Human immunodeficiency virus type 1
<400> 197
   Ile Pro Arg Arg Ile Arg Gln Gly Leu
   1 5
<210> 198
   <211> 9
   <212> PRT
   <213> Human immunodeficiency virus type 1
<400> 198
   Glu Leu Arg Ser Leu Tyr Asn Thr Val
   1 5
<210> 199
   <211> 8
   <212> PRT
   <213> Human immunodeficiency virus type 1
<400> 199
   Trp Pro Thr Val Arg Glu Arg Met
   1 5
<210> 200
   <211> 8
   <212> PRT
   <213> Human immunodeficiency virus type 1
<400> 200
   Phe Leu Lys Glu Lys Gly Gly Leu
   1 5
<210> 201
   <211> 10
   <212> PRT
   <213> Human immunodeficiency virus type 1
<400> 201
   Lys Ile Arg Leu Arg Pro Gly Gly Lys Lys
   1 5 10
<210> 202
   <211> 9
   <212> PRT
   <213> Human immunodeficiency virus type 1
<400> 202
   Ile Arg Leu Arg Pro Gly Gly Lys Lys
   1 5
<210> 203
   <211> 10
   <212> PRT
   <213> Human immunodeficiency virus type 1
<400> 203
   Gly Arg Arg Gly Trp Glu Ala Leu Lys Tyr
   1 5 10
<210> 204
   <211> 10
   <212> PRT
   <213> Human immunodeficiency virus type 1
<400> 204
   Gln Val Pro Leu Arg Pro Met Thr Tyr Lys
   1 5 10
<210> 205
   <211> 8
   <212> PRT
   <213> Human immunodeficiency virus type 1
<400> 205
   Arg Tyr Leu Lys Asp Gln Gln Leu
   1 5
<210> 206
   <211> 10
   <212> PRT
   <213> Human immunodeficiency virus type 1
<400> 206
   Arg Arg Gln Asp Ile Leu Asp Leu Trp Ile
   1 5 10
<210> 207
   <211> 8
   <212> PRT
   <213> Human immunodeficiency virus type 1
<400> 207
   Arg Tyr Pro Leu Thr Phe Gly Trp
   1 5
<210> 208
   <211> 9
   <212> PRT
   <213> Human immunodeficiency virus type 1
<400> 208
   Trp Ala Ser Arg Glu Leu Glu Arg Phe
   1 5
<210> 209
   <211> 9
   <212> PRT
   <213> Human immunodeficiency virus type 1
<400> 209
   Thr Val Leu Asp Val Gly Asp Ala Tyr
   1 5
<210> 210
   <211> 11
   <212> PRT
   <213> Human immunodeficiency virus type 1
<400> 210
   Val Pro Val Trp Lys Glu Ala Thr Thr Thr Leu
   1 5 10
<210> 211
   <211> 9
   <212> PRT
   <213> Human immunodeficiency virus type 1
<400> 211
   Asn Ser Ser Lys Val Ser Gln Asn Tyr
   1 5
<210> 212
   <211> 9
   <212> PRT
   <213> Human immunodeficiency virus type 1
<400> 212
   Pro Pro Ile Pro Val Gly Asp Ile Tyr
   1 5
<210> 213
   <211> 9
   <212> PRT
   <213> Human immunodeficiency virus type 1
<400> 213
   His Pro Asp Ile Val Ile Tyr Gln Tyr
   1 5
<210> 214
   <211> 9
   <212> PRT
   <213> Human immunodeficiency virus type 1
<400> 214
   Thr Ala Val Pro Trp Asn Ala Ser Trp
   1 5
<210> 215
   <211> 9
   <212> PRT
   <213> Human immunodeficiency virus type 1
<400> 215
   Asn Pro Val Pro Val Gly Asn Ile Tyr
   1 5
<210> 216
   <211> 9
   <212> PRT
   <213> Human immunodeficiency virus type 1
<400> 216
   Tyr Phe Pro Asp Trp Gln Asn Tyr Thr
   1 5
<210> 217
   <211> 9
   <212> PRT
   <213> Human immunodeficiency virus type 1
<400> 217
   Gly His Gln Ala Ala Met Gln Met Leu
   1 5
<210> 218
   <211> 10
   <212> PRT
   <213> Human immunodeficiency virus type 1
<400> 218
   Arg Leu Arg Pro Gly Gly Lys Lys Lys Tyr
   1 5 10
<210> 219
   <211> 9
   <212> PRT
   <213> Human immunodeficiency virus type 1
<400> 219
   Tyr Pro Gly Ile Lys Val Arg Gln Leu
   1 5
<210> 220
   <211> 10
   <212> PRT
   <213> Human immunodeficiency virus type 1
<400> 220
   Gly Ala Glu Thr Phe Tyr Val Asp Gly Ala
   1 5 10
<210> 221
   <211> 9
   <212> PRT
   <213> Human immunodeficiency virus type 1
<400> 221
   Asn Ala Asn Pro Asp Cys Lys Thr Ile
   1 5
<210> 222
   <211> 8
   <212> PRT
   <213> Human immunodeficiency virus type 1
<400> 222
   Arg Met Tyr Ser Pro Thr Ser Ile
   1 5
<210> 223
   <211> 10
   <212> PRT
   <213> Human immunodeficiency virus type 1
<400> 223
   Val Pro Val Trp Lys Glu Ala Thr Thr Thr
   1 5 10
<210> 224
   <211> 9
   <212> PRT
   <213> Human immunodeficiency virus type 1
<400> 224
   Ile Ser Pro Arg Thr Leu Asn Ala Trp
   1 5
<210> 225
   <211> 10
   <212> PRT
   <213> Human immunodeficiency virus type 1
<400> 225
   Thr Ser Thr Leu Gln Glu Gln Ile Gly Trp
   1 5 10
<210> 226
   <211> 11
   <212> PRT
   <213> Human immunodeficiency virus type 1
<400> 226
   Lys Ala Phe Ser Pro Glu Val Ile Pro Met Phe
   1 5 10
<210> 227
   <211> 9
   <212> PRT
   <213> Human immunodeficiency virus type 1
<400> 227
   Gln Ala Ser Gln Glu Val Lys Asn Trp
   1 5
<210> 228
   <211> 9
   <212> PRT
   <213> Human immunodeficiency virus type 1
<400> 228
   Gln Ala Ser Gln Asp Val Lys Asn Trp
   1 5
<210> 229
   <211> 10
   <212> PRT
   <213> Human immunodeficiency virus type 1
<400> 229
   His Thr Gln Gly Tyr Phe Pro Asp Trp Gln
   1 5 10
<210> 230
   <211> 9
   <212> PRT
   <213> Human immunodeficiency virus type 1
<400> 230
   Tyr Phe Pro Asp Trp Gln Asn Tyr Thr
   1 5
<210> 231
   <211> 10
   <212> PRT
   <213> Human immunodeficiency virus type 1
<400> 231
   Thr Ser Thr Leu Gln Glu Gln Ile Gly Trp
   1 5 10
<210> 232
   <211> 10
   <212> PRT
   <213> Human immunodeficiency virus type 1
<400> 232
   Arg Leu Arg Pro Gly Gly Lys Lys Lys Tyr
   1 5 10
<210> 233
   <211> 10
   <212> PRT
   <213> Human immunodeficiency virus type 1
<400> 233
   Leu Gly Leu Asn Lys Ile Val Arg Met Tyr
   1 5 10
<210> 234
   <211> 12
   <212> PRT
   <213> Human immunodeficiency virus type 1
<400> 234
   Leu Val Gly Lys Leu Asn Trp Ala Ser Gln Ile Tyr
   1 5 10
<210> 235
   <211> 10
   <212> PRT
   <213> Human immunodeficiency virus type 1
<400> 235
   Ile Leu Lys Glu Pro Val His Gly Val Tyr
   1 5 10
<210> 236
   <211> 11
   <212> PRT
   <213> Human immunodeficiency virus type 1
<400> 236
   Thr Gln Gly Tyr Phe Pro Asp Trp Gln Asn Tyr
   1 5 10
<210> 237
   <211> 8
   <212> PRT
   <213> Human immunodeficiency virus type 1
<400> 237
   Ala Val Asp Leu Ser His Phe Leu
   1 5
<210> 238
   <211> 8
   <212> PRT
   <213> Human immunodeficiency virus type 1
<400> 238
   Val Ile Pro Met Phe Ser Ala Leu
   1 5
<210> 239
   <211> 9
   <212> PRT
   <213> Human immunodeficiency virus type 1
<400> 239
   Phe Asn Cys Gly Gly Glu Phe Phe Tyr
   1 5
<210> 240
   <211> 9
   <212> PRT
   <213> Human immunodeficiency virus type 1
<400> 240
   Ser Phe Asn Cys Gly Gly Glu Phe Phe
   1 5
<210> 241
   <211> 10
   <212> PRT
   <213> Human immunodeficiency virus type 1
<400> 241
   Pro Leu Thr Phe Gly Trp Cys Tyr Lys Leu
   1 5 10
<210> 242
   <211> 10
   <212> PRT
   <213> Human immunodeficiency virus type 1
<400> 242
   Val Leu Glu Trp Arg Phe Asp Ser Arg Leu
   1 5 10
<210> 243
   <211> 10
   <212> PRT
   <213> Human immunodeficiency virus type 1
<400> 243
   Phe Pro Val Thr Pro Gln Val Pro Leu Arg
   1 5 10
<210> 244
   <211> 10
   <212> PRT
   <213> Human immunodeficiency virus type 1
<400> 244
   Thr Pro Gly Pro Gly Val Arg Tyr Pro Leu
   1 5 10
<210> 245
   <211> 9
   <212> PRT
   <213> Human immunodeficiency virus type 1
<400> 245
   Gln Ala Ser Gln Glu Val Lys Asn Trp
   1 5
<210> 246
   <211> 10
   <212> PRT
   <213> Human immunodeficiency virus type 1
<400> 246
   Val Pro Leu Asp Glu Asp Phe Arg Lys Tyr
   1 5 10
<210> 247
   <211> 9
   <212> PRT
   <213> Human immunodeficiency virus
<400> 247
   Asn Pro Asp Ile Val Ile Tyr Gln Tyr
   1 5
<210> 248
   <211> 9
   <212> PRT
   <213> Human immunodeficiency virus
<400> 248
   Arg Ala Ile Glu Ala Gln Ala His Leu
   1 5
<210> 249
   <211> 8
   <212> PRT
   <213> Human immunodeficiency virus
<400> 249
   Thr Ala Phe Thr Ile Pro Ser Ile
   1 5
<210> 250
   <211> 10
   <212> PRT
   <213> Human immunodeficiency virus
<400> 250
   Val His Pro Val His Ala Gly Pro Ile Ala
   1 5 10
<210> 251
   <211> 10
   <212> PRT
   <213> Human immunodeficiency virus
<400> 251
   Asn Cys Ser Phe Asn Ile Ser Thr Ser Ile
   1 5 10
<210> 252
   <211> 9
   <212> PRT
   <213> Human immunodeficiency virus
<400> 252
   Cys Thr Asn Val Ser Thr Val Gln Cys
   1 5
<210> 253
   <211> 9
   <212> PRT
   <213> Human immunodeficiency virus
<400> 253
   Ile Gly Pro Gly Arg Ala Phe His Thr
   1 5
<210> 254
   <211> 9
   <212> PRT
   <213> Human immunodeficiency virus
<400> 254
   Asn Pro Asp Ile Val Ile Tyr Gln Tyr
   1 5
<210> 255
   <211> 10
   <212> PRT
   <213> Human immunodeficiency virus
<400> 255
   Glu Pro Ile Val Gly Ala Glu Thr Phe Tyr
   1 5 10
<210> 256
   <211> 9
   <212> PRT
   <213> Human immunodeficiency virus
<400> 256
   Glu Pro Ile Val Gly Ala Glu Thr Phe
   1 5
<210> 257
   <211> 9
   <212> PRT
   <213> Human immunodeficiency virus
<400> 257
   Ser Pro Ala Ile Phe Gln Ser Ser Met
   1 5
<210> 258
   <211> 10
   <212> PRT
   <213> Human immunodeficiency virus
<400> 258
   Val Pro Leu Asp Lys Asp Phe Arg Lys Tyr
   1 5 10
<210> 259
   <211> 9
   <212> PRT
   <213> Human immunodeficiency virus
<400> 259
   Ile Pro Leu Thr Glu Glu Ala Glu Leu
   1 5
<210> 260
   <211> 11
   <212> PRT
   <213> Human immunodeficiency virus
<400> 260
   Arg Pro Gln Val Pro Leu Arg Pro Met Thr Tyr
   1 5 10
<210> 261
   <211> 9
   <212> PRT
   <213> Human immunodeficiency virus
<400> 261
   Phe Pro Val Arg Pro Gln Val Pro Leu
   1 5
<210> 262
   <211> 9
   <212> PRT
   <213> Human immunodeficiency virus
<400> 262
   Asp Pro Asn Pro Gln Glu Val Val Leu
   1 5
<210> 263
   <211> 9
   <212> PRT
   <213> Human immunodeficiency virus
<400> 263
   Arg Pro Ile Val Ser Thr Gln Leu Leu
   1 5
<210> 264
   <211> 9
   <212> PRT
   <213> Human immunodeficiency virus
<400> 264
   Ile Pro Leu Thr Glu Glu Ala Glu Leu
   1 5
<210> 265
   <211> 9
   <212> PRT
   <213> Human immunodeficiency virus
<400> 265
   Asp Pro Asn Pro Gln Glu Val Val Leu
   1 5
<210> 266
   <211> 9
   <212> PRT
   <213> Human immunodeficiency virus type 2
<400> 266
   Ala Met Gln Met Leu Lys Glu Thr Ile
   1 5
<210> 267
   <211> 9
   <212> PRT
   <213> Human immunodeficiency virus type 2
<400> 267
   Thr Pro Tyr Asp Ile Asn Gln Met Leu
   1 5
<210> 268
   <211> 10
   <212> PRT
   <213> Human immunodeficiency virus type 1
<400> 268
   Arg Arg Trp Ile Gln Leu Gly Leu Gln Lys
   1 5 10
<210> 269
   <211> 15
   <212> PRT
   <213> Human immunodeficiency virus type 1
<400> 269
   Gly Ile Trp Gly Cys Ser Gly Lys Leu Ile Cys Thr Thr Ala Val
   1 5 10 15
<210> 270
   <211> 15
   <212> PRT
   <213> Human immunodeficiency virus type 1
<400> 270
   Ala Leu Ile Trp Glu Asp Leu Arg Ser Leu Cys Leu Phe Ser Tyr
   1 5 10 15
<210> 271
   <211> 9
   <212> PRT
   <213> Human papillomavirus type 6b
<400> 271
   Gly Leu His Cys Tyr Glu Gln Leu Val
   1 5
<210> 272
   <211> 9
   <212> PRT
   <213> Human papillomavirus type 6b
<400> 272
   Pro Leu Lys Gln His Phe Gln Ile Val
   1 5
<210> 273
   <211> 9
   <212> PRT
   <213> Human papillomavirus type 11
<400> 273
   Arg Leu Val Thr Leu Lys Asp Ile Val
   1 5
<210> 274
   <211> 9
   <212> PRT
   <213> Human papillomavirus type 16
<400> 274
   Thr Leu Gly Ile Val Cys Pro Ile Cys
   1 5
<210> 275
   <211> 9
   <212> PRT
   <213> Human papillomavirus type 16
<400> 275
   Gly Thr Leu Gly Ile Val Cys Pro Ile
   1 5
<210> 276
   <211> 9
   <212> PRT
   <213> Human papillomavirus type 16
<400> 276
   Met Leu Asp Leu Gln Pro Glu Thr Thr
   1 5
<210> 277
   <211> 10
   <212> PRT
   <213> Human papillomavirus type 16
<400> 277
   Tyr Met Leu Asp Leu Gln Pro Glu Thr Thr
   1 5 10
<210> 278
   <211> 8
   <212> PRT
   <213> Human papillomavirus type 16
<400> 278
   Arg Pro Arg Lys Leu Pro Gln Leu
   1 5
<210> 279
   <211> 9
   <212> PRT
   <213> Human papillomavirus type 16
<400> 279
   Arg Ala His Tyr Asn Ile Val Thr Phe
   1 5
<210> 280
   <211> 8
   <212> PRT
   <213> HSV
<400> 280
   Ser Ser Ile Glu Phe Ala Arg Leu
   1 5
<210> 281
   <211> 9
   <212> PRT
   <213> HSV-1
<400> 281
   Gly Ile Gly Ile Gly Val Leu Ala Ala
   1 5
<210> 282
   <211> 9
   <212> PRT
   <213> HSV-1
<400> 282
   Asp Tyr Ala Thr Leu Gly Val Gly Val
   1 5
<210> 283
   <211> 11
   <212> PRT
   <213> HSV-1
<400> 283
   Leu Tyr Arg Thr Phe Ala Gly Asn Pro Arg Ala
   1 5 10
<210> 284
   <211> 8
   <212> PRT
   <213> HSV-1
<400> 284
   Gln Thr Phe Asp Phe Gly Arg Leu
   1 5
<210> 285
   <211> 9
   <212> PRT
   <213> HSV-2
<400> 285
   Gly Ala Gly Ile Gly Val Ala Val Leu
   1 5
<210> 286
   <211> 9
   <212> PRT
   <213> Human T-cell lymphotropic virus type 1
<400> 286
   Leu Leu Phe Gly Tyr Pro Val Tyr Val
   1 5
<210> 287
   <211> 9
   <212> PRT
   <213> Haemophilus influenzae
<400> 287
   Gly Ile Leu Gly Phe Val Phe Thr Leu
   1 5
<210> 288
   <211> 10
   <212> PRT
   <213> Haemophilus influenzae
<400> 288
   Ile Leu Gly Phe Val Phe Thr Leu Thr Val
   1 5 10
<210> 289
   <211> 9
   <212> PRT
   <213> Haemophilus influenzae
<400> 289
   Ile Leu Arg Gly Ser Val Ala His Lys
   1 5
<210> 290
   <211> 9
   <212> PRT
   <213> Haemophilus influenzae
<400> 290
   Lys Thr Gly Gly Pro Ile Tyr Lys Arg
   1 5
<210> 291
   <211> 9
   <212> PRT
   <213> Haemophilus influenzae
<400> 291
   Glu Leu Arg Ser Arg Tyr Trp Ala Ile
   1 5
<210> 292
   <211> 8
   <212> PRT
   <213> Haemophilus influenzae
<400> 292
   Leu Arg Ser Arg Tyr Trp Ala Ile
   1 5
<210> 293
   <211> 9
   <212> PRT
   <213> Haemophilus influenzae
<400> 293
   Glu Asp Leu Arg Val Leu Ser Phe Ile
   1 5
<210> 294
   <211> 9
   <212> PRT
   <213> Haemophilus influenzae
<400> 294
   Gly Glu Ile Ser Pro Leu Pro Ser Leu
   1 5
<210> 295
   <211> 9
   <212> PRT
   <213> Haemophilus influenzae
<400> 295
   Phe Glu Asp Leu Arg Val Leu Ser Phe
   1 5
<210> 296
   <211> 9
   <212> PRT
   <213> Haemophilus influenzae
<400> 296
   Val Ser Asp Gly Gly Pro Asn Leu Tyr
   1 5
<210> 297
   <211> 9
   <212> PRT
   <213> Haemophilus influenzae
<400> 297
   Cys Thr Glu Leu Lys Leu Ser Asp Tyr
   1 5
<210> 298
   <211> 9
   <212> PRT
   <213> Haemophilus influenzae
<400> 298
   Ala Ile Met Asp Lys Asn Ile Ile Leu
   1 5
<210> 299
   <211> 10
   <212> PRT
   <213> Haemophilus influenzae
<400> 299
   Ile Met Asp Lys Asn Ile Ile Leu Lys Ala
   1 5 10
<210> 300
   <211> 9
   <212> PRT
   <213> Haemophilus influenzae
<400> 300
   Ser Arg Tyr Trp Ala Ile Arg Thr Arg
   1 5
<210> 301
   <211> 9
   <212> PRT
   <213> Haemophilus influenzae
<400> 301
   Thr Tyr Gln Arg Thr Arg Ala Leu Val
   1 5
<210> 302
   <211> 10
   <212> PRT
   <213> Haemophilus influenzae
<400> 302
   Thr Tyr Val Ser Val Ser Thr Ser Thr Leu
   1 5 10
<210> 303
   <211> 9
   <212> PRT
   <213> Haemophilus influenzae
<400> 303
   Ile Tyr Ser Thr Val Ala Ser Ser Leu
   1 5
<210> 304
   <211> 8
   <212> PRT
   <213> Haemophilus influenzae
<400> 304
   Phe Glu Ala Asn Gly Asn Leu Ile
   1 5
<210> 305
   <211> 9
   <212> PRT
   <213> Haemophilus influenzae
<400> 305
   Ile Glu Gly Gly Trp Thr Gly Met Ile
   1 5
<210> 306
   <211> 8
   <212> PRT
   <213> Haemophilus influenzae
<400> 306
   Ser Asp Tyr Glu Gly Arg Leu Ile
   1 5
<210> 307
   <211> 9
   <212> PRT
   <213> Haemophilus influenzae
<400> 307
   Glu Glu Gly Ala Ile Val Gly Glu Ile
   1 5
<210> 308
   <211> 9
   <212> PRT
   <213> Haemophilus influenzae
<400> 308
   Ala Ser Asn Glu Asn Met Glu Thr Met
   1 5
<210> 309
   <211> 9
   <212> PRT
   <213> Haemophilus influenzae
<400> 309
   Ala Ser Asn Glu Asn Met Asp Ala Met
   1 5
<210> 310
   <211> 10
   <212> PRT
   <213> Haemophilus influenzae
<400> 310
   Lys Leu Gly Glu Phe Tyr Asn Gln Met Met
   1 5 10
<210> 311
   <211> 10
   <212> PRT
   <213> Haemophilus influenzae
<400> 311
   Lys Ala Gly Glu Phe Tyr Asn Gln Met Met
   1 5 10
<210> 312
   <211> 9
   <212> PRT
   <213> Haemophilus influenzae
<400> 312
   Leu Tyr Gln Asn Val Gly Thr Tyr Val
   1 5
<210> 313
   <211> 10
   <212> PRT
   <213> Haemophilus influenzae
<400> 313
   Thr Tyr Val Ser Val Gly Thr Ser Thr Leu
   1 5 10
<210> 314
   <211> 9
   <212> PRT
   <213> Haemophilus influenzae
<400> 314
   Val Tyr Gln Ile Leu Ala Ile Tyr Ala
   1 5
<210> 315
   <211> 9
   <212> PRT
   <213> Haemophilus influenzae
<400> 315
   Ile Tyr Ala Thr Val Ala Gly Ser Leu
   1 5
<210> 316
   <211> 10
   <212> PRT
   <213> Haemophilus influenzae
<400> 316
   Thr Tyr Val Ser Val Gly Thr Ser Thr Ile
   1 5 10
<210> 317
   <211> 8
   <212> PRT
   <213> Haemophilus influenzae
<400> 317
   Phe Glu Ser Thr Gly Asn Leu Ile
   1 5
<210> 318
   <211> 9
   <212> PRT
   <213> JHMV
<400> 318
   Ala Pro Thr Ala Gly Ala Phe Phe Phe
   1 5
<210> 319
   <211> 9
   <212> PRT
   <213> LCMV
<400> 319
   Arg Pro Gln Ala Ser Gly Val Tyr Met
   1 5
<210> 320
   <211> 9
   <212> PRT
   <213> LCMV Phe Gln Pro Gln Asn Gly Gln Phe Ile 1 5
<210> 321 <211> 11 <212> PRT <213> LCMV
<210> 322 <211> 10 <212> PRT <213> LCMV Lys Ala Val Tyr Asn Phe Ala Thr Cys Gly 1 5 10
<210> 323 <211> 9 <212> PRT <213> MCMV Tyr Pro His Phe Met Pro Thr Asn Leu 1 5
<210> 324 <211> 9 <212> PRT <213> MHV Cys Leu Ser Trp Asn Gly Pro His Leu 1 5
<210> 325 <211> 9
   <212> PRT
   <213> MMTV
<400> 325
   Ser Phe Ala Val Ala Thr Thr Ala Leu
   1 5
<210> 326
   <211> 9
   <212> PRT
   <213> MMTV
<400> 326
   Ser Tyr Glu Thr Phe Ile Ser Arg Leu
   1               5
<210> 327
   <211> 8
   <212> PRT
   <213> MMTV
<400> 327
   Ala Asn Tyr Asp Phe Ile Cys Val
   1               5
<210> 328
   <211> 8
   <212> PRT
   <213> Murine leukemia virus
<400> 328
   Lys Ser Pro Trp Phe Thr Thr Leu
   1               5
<210> 329
   <211> 8
   <212> PRT
   <213> Murine leukemia virus
<400> 329
   Ser Ser Trp Asp Phe Ile Thr Val
   1               5
<210> 330
   <211> 9
   <212> PRT
   <213> Murine leukemia virus
<400> 330
   Cys Cys Leu Cys Leu Thr Val Phe Leu
   1               5
<210> 331
   <211> 9
   <212> PRT
   <213> Polio virus
<400> 331
   Ser Pro Ser Tyr Val Tyr His Gln Phe
   1 5
<210> 332
   <211> 11
   <212> PRT
   <213> Polio virus
<400> 332
   Ser Arg Arg Tyr Pro Asp Ala Val Tyr Leu His
   1 5 10
<210> 333
   <211> 9
   <212> PRT
   <213> MV
<400> 333
   Arg Arg Tyr Pro Asp Ala Val Tyr Leu
   1 5
<210> 334
   <211> 9
   <212> PRT
   <213> MV
<400> 334
   Tyr Pro Ala Leu Gly Leu His Glu Phe
   1 5
<210> 335
   <211> 9
   <212> PRT
   <213> Rotavirus sp.
<400> 335
   Asp Pro Val Ile Asp Arg Leu Tyr Leu
   1 5
<210> 336
   <211> 9
   <212> PRT
   <213> Rotavirus sp.
<400> 336
   Ser Pro Gly Arg Ser Phe Ser Tyr Phe
   1 5
<210> 337
   <211> 8
   <212> PRT
   <213> Rotavirus sp.
<400> 337
   Thr Tyr Lys Asp Thr Val Gln Leu
   1 5
<210> 338
   <211> 10
   <212> PRT
   <213> polio virus
<400> 338
   Phe Tyr Asp Gly Phe Ser Lys Val Pro Leu
   1 5 10
<210> 339
   <211> 9
   <212> PRT
   <213> Pseudorabies virus
<400> 339
   Ile Ala Gly Ile Gly Ile Leu Ala Ile
   1 5
<210> 340
   <211> 9
   <212> PRT
   <213> SV
<400> 340
   Val Glu Ala Glu Ile Ala His Gln Ile
   1 5
<210> 341
   <211> 8
   <212> PRT
   <213> SV
<400> 341
   Ile Ile Tyr Arg Phe Leu Leu Ile
   1 5
<210> 342
   <211> 9
   <212> PRT
   <213> Simian virus 40
<400> 342
   Val Gly Pro Val Phe Pro Pro Gly Met
   1 5
<210> 343
   <211> 9
   <212> PRT
   <213> Simian virus 40
<400> 343
   Tyr Ser Gly Tyr Ile Phe Arg Asp Leu
   1 5
<210> 344
   <211> 9
   <212> PRT
   <213> Simian virus 40
<400> 344
   Ser Tyr Ile Gly Ser Ile Asn Asn Ile
   1 5
<210> 345
   <211> 12
   <212> PRT
   <213> Simian virus 40
<400> 345
   Glu Gly Cys Thr Pro Tyr Asp Ile Asn Gln Met Leu
   1 5 10
<210> 346
   <211> 9
   <212> PRT
   <213> Simian virus 40
<400> 346
   Phe Ala Pro Gly Asn Tyr Pro Ala Leu
   1 5
<210> 347
   <211> 8
   <212> PRT
   <213> Simian virus 40
<400> 347
   Val Val Tyr Asp Phe Leu Lys Cys
   1 5
<210> 348
   <211> 10
   <212> PRT
   <213> Simian virus 40
<400> 348
   Ser Ala Ile Asn Asn Tyr Ala Gln Lys Leu
   1 5 10
<210> 349
   <211> 9
   <212> PRT
   <213> Simian virus 40
<400> 349
   Cys Lys Gly Val Asn Lys Glu Tyr Leu
   1 5
<210> 350
   <211> 9
   <212> PRT
   <213> Simian virus 40
<400> 350
   Gln Gly Ile Asn Asn Leu Asp Asn Leu
   1 5
<210> 351
   <211> 9
   <212> PRT
   <213> Simian virus 40
<220>
   <221> VARIANT
   <222> (9)
   <223> leu
<400> 351
   Asn Asn Leu Asp Asn Leu Arg Asp Tyr
   1 5
<210> 352
   <211> 9
   <212> PRT
   <213> Haemophilus influenzae
<400> 352
   Ser Glu Phe Leu Leu Glu Lys Arg Ile
   1 5
<210> 353
   <211> 8
   <212> PRT
   <213> Haemophilus influenzae
<400> 353
   Arg Gly Tyr Val Tyr Gln Gly Leu
   1 5
<210> 354
   <211> 9
   <212> PRT
   <213> human
<400> 354
   Glu Ala Asp Pro Thr Gly His Ser Tyr
   1 5
<210> 355
   <211> 9
   <212> PRT
   <213> Haemophilus influenzae
<400> 355
   Val Ser Asp Gly Gly Pro Asn Leu Tyr
   1 5
<210> 356
   <211> 9
   <212> PRT
   <213> Haemophilus influenzae
<400> 356
   Cys Thr Glu Leu Lys Leu Ser Asp Tyr
   1 5
<210> 357
   <211> 9
   <212> PRT
   <213> human
<400> 357
   Glu Val Asp Pro Ile Gly His Leu Tyr
   1 5
<210> 358
   <211> 10
   <212> PRT
   <213> human
<400> 358
   Met Leu Leu Ser Val Pro Leu Leu Leu Gly
   1 5 10
<210> 359
   <211> 9
   <212> PRT
   <213> Hepatitis B virus
<400> 359
   Ser Thr Asx Xaa Gln Ser Gly Xaa Gln
   1 5
<210> 360
   <211> 9
   <212> PRT
   <213> human
<400> 360
   Tyr Met Asp Gly Thr Met Ser Gln Val
   1 5
<210> 361
   <211> 9
   <212> PRT
   <213> Human immunodeficiency virus type 1
<400> 361
   Ile Leu Lys Glu Pro Val His Gly Val
   1 5
<210> 362
   <211> 10
   <212> PRT
   <213> Haemophilus influenzae
<400> 362
   Ile Leu Gly Phe Val Phe Thr Leu Thr Val
   1 5 10
<210> 363
   <211> 9
   <212> PRT
   <213> HTLV-1
<400> 363
   Leu Leu Phe Gly Tyr Pro Val Tyr Val
   1 5
<210> 364
   <211> 10
   <212> PRT
   <213> Hepatitis B virus
<400> 364
   Gly Leu Ser Pro Thr Val Trp Leu Ser Val
   1 5 10
<210> 365
   <211> 9
   <212> PRT
   <213> Hepatitis B virus
<400> 365
   Trp Leu Ser Leu Leu Val Pro Phe Val
   1 5
<210> 366
   <211> 10
   <212> PRT
   <213> Hepatitis B virus
<400> 366
   Phe Leu Pro Ser Asp Phe Phe Pro Ser Val
   1 5 10
<210> 367
   <211> 9
   <212> PRT
   <213> EBV
<400> 367
   Cys Leu Gly Gly Leu Leu Thr Met Val
   1 5
<210> 368
   <211> 11
   <212> PRT
   <213> Human cytomegalovirus
<400> 368
   Phe Leu Ala Gly Asn Ser Ala Tyr Glu Tyr Val
   1 5 10
<210> 369
   <211> 10
   <212> PRT
   <213> Haemophilus influenzae
<400> 369
   Lys Leu Gly Glu Phe Tyr Asn Gln Met Met
   1 5 10
<210> 370
   <211> 10
   <212> PRT
   <213> Hepatitis C virus
<400> 370
   Lys Leu Val Ala Leu Gly Ile Asn Ala Val
   1 5 10
<210> 371
   <211> 9
   <212> PRT
   <213> Hepatitis C virus
<400> 371
   Asp Leu Met Gly Tyr Ile Pro Leu Val
   1 5
<210> 372
   <211> 9
   <212> PRT
   <213> Human papillomavirus type 11
<400> 372
   Arg Leu Val Thr Leu Lys Asp Ile Val
   1 5
<210> 373
   <211> 9
   <212> PRT
   <213> human
<400> 373
   Met Leu Leu Ala Val Leu Tyr Cys Leu
   1 5
<210> 374
   <211> 9
   <212> PRT
   <213> human
<400> 374
   Ala Ala Gly Ile Gly Ile Leu Thr Val
   1 5
<210> 375
   <211> 9
   <212> PRT
   <213> human
<400> 375
   Tyr Leu Glu Pro Gly Pro Val Thr Ala
   1 5
<210> 376
   <211> 10
   <212> PRT
   <213> human
<400> 376
   Ile Leu Asp Gly Thr Ala Thr Leu Arg Leu
   1 5 10
<210> 377
   <211> 10
   <212> PRT
   <213> human
<400> 377
   Leu Leu Asp Gly Thr Ala Thr Leu Arg Leu
   1   5 10
<210> 378
   <211> 9
   <212> PRT
   <213> human
<400> 378
   Ile Thr Asp Gln Val Pro Phe Ser Val
   1 5
<210> 379
   <211> 9
   <212> PRT
   <213> human
<400> 379
   Lys Thr Trp Gly Gln Tyr Trp Gln Val
   1 5
<210> 380
   <211> 10
   <212> PRT
   <213> human
<400> 380
   Thr Ile Thr Asp Gln Val Pro Phe Ser Val
   1 5 10
<210> 381
   <211> 9
   <212> PRT
   <213> Human immunodeficiency virus type 1
<400> 381
   Ala Phe His His Val Ala Arg Glu Leu
   1 5
<210> 382
   <211> 9
   <212> PRT
   <213> Plasmodium falciparum
<400> 382
   Tyr Leu Asn Lys Ile Gln Asn Ser Leu
   1 5
<210> 383
   <211> 10
   <212> PRT
   <213> Plasmodium falciparum
<400> 383
   Met Met Arg Lys Leu Ala Ile Leu Ser Val
   1 5 10
<210> 384
   <211> 10
   <212> PRT
   <213> Haemophilus influenzae
<400> 384
   Lys Ala Gly Glu Phe Tyr Asn Gln Met Met
   1 5 10
<210> 385
   <211> 9
   <212> PRT
   <213> EBV
<400> 385
   Asn Ile Ala Glu Gly Leu Arg Ala Leu
   1 5
<210> 386
   <211> 9
   <212> PRT
   <213> EBV
<400> 386
   Asn Leu Arg Arg Gly Thr Ala Leu Ala
   1 5
<210> 387
   <211> 9
   <212> PRT
   <213> EBV
<400> 387
   Ala Leu Ala Ile Pro Gln Cys Arg Leu
   1 5
<210> 388
   <211> 9
   <212> PRT
   <213> EBV
<400> 388
   Val Leu Lys Asp Ala Ile Lys Asp Leu
   1 5
<210> 389
   <211> 9
   <212> PRT
   <213> EBV
<400> 389
   Phe Met Val Phe Ile Gln Thr His Ile
   1 5
<210> 390
   <211> 9
   <212> PRT
   <213> EBV
<400> 390
   His Leu Ile Val Asp Thr Asp Ser Leu
   1 5
<210> 391
   <211> 9
   <212> PRT
   <213> EBV
<400> 391
   Ser Leu Gly Asn Pro Ser Leu Ser Val
   1 5
<210> 392
   <211> 9
   <212> PRT
   <213> EBV
<400> 392
   Pro Leu Ala Ser Ala Met Arg Met Leu
   1 5
<210> 393
   <211> 9
   <212> PRT
   <213> EBV
<400> 393
   Arg Met Leu Trp Met Ala Asn Tyr Ile
   1 5
<210> 394
   <211> 9
   <212> PRT
   <213> EBV
<400> 394
   Met Leu Trp Met Ala Asn Tyr Ile Val
   1 5
<210> 395
   <211> 9
   <212> PRT
   <213> EBV
<400> 395
   Ile Leu Pro Gln Gly Pro Gln Thr Ala
   1 5
<210> 396
   <211> 9
   <212> PRT
   <213> EBV
<400> 396
   Pro Leu Arg Pro Thr Ala Pro Thr Ile
   1 5
<210> 397
   <211> 9
   <212> PRT
   <213> EBV
<400> 397
   Pro Leu Pro Pro Ala Thr Leu Thr Val
   1 5
<210> 398
   <211> 9
   <212> PRT
   <213> EBV
<400> 398
   Arg Met His Leu Pro Val Leu His Val
   1 5
<210> 399
   <211> 9
   <212> PRT
   <213> EBV
<400> 399
   Pro Met Pro Leu Pro Pro Ser Gln Leu
   1 5
<210> 400
   <211> 9
   <212> PRT
   <213> EBV
<400> 400
   Gln Leu Pro Pro Pro Ala Ala Pro Ala
   1 5
<210> 401
   <211> 9
   <212> PRT
   <213> EBV
<400> 401
   Ser Met Pro Glu Leu Ser Pro Val Leu
   1 5
<210> 402
   <211> 9
   <212> PRT
   <213> EBV
<400> 402
   Asp Leu Asp Glu Ser Trp Asp Tyr Ile
   1 5
<210> 403
   <211> 9
   <212> PRT
   <213> EBV
<400> 403
   Pro Leu Pro Cys Val Leu Trp Pro Val
   1 5
<210> 404
   <211> 9
   <212> PRT
   <213> EBV
<400> 404
   Ser Leu Glu Glu Cys Asp Ser Glu Leu
   1 5
<210> 405
   <211> 9
   <212> PRT
   <213> EBV
<400> 405
   Glu Ile Lys Arg Tyr Lys Asn Arg Val
   1 5
<210> 406
   <211> 9
   <212> PRT
   <213> EBV
<400> 406
   Gln Leu Leu Gln His Tyr Arg Glu Val
   1 5
<210> 407
   <211> 9
   <212> PRT
   <213> EBV
<400> 407
   Leu Leu Gln His Tyr Arg Glu Val Ala
   1 5
<210> 408
   <211> 9
   <212> PRT
   <213> EBV
<400> 408
   Leu Leu Lys Gln Met Cys Pro Ser Leu
   1 5
<210> 409
   <211> 9
   <212> PRT
   <213> EBV
<400> 409
   Ser Ile Ile Pro Arg Thr Pro Asp Val
   1 5
<210> 410
   <211> 9
   <212> PRT
   <213> Haemophilus influenzae
<400> 410
   Ala Ile Met Asp Lys Asn Ile Ile Leu
   1 5
<210> 411
   <211> 10
   <212> PRT
   <213> Haemophilus influenzae
<400> 411
   Ile Met Asp Lys Asn Ile Ile Leu Lys Ala
   1 5 10
<210> 412
   <211> 10
   <212> PRT
   <213> Hepatitis C virus
<400> 412
   Leu Leu Ala Leu Leu Ser Cys Leu Thr Val
   1 5 10
<210> 413
   <211> 8
   <212> PRT
   <213> Hepatitis C virus
<400> 413
   Ile Leu His Thr Pro Gly Cys Val
   1 5
<210> 414
   <211> 10
   <212> PRT
   <213> Hepatitis C virus
<400> 414
   Gln Leu Arg Arg His Ile Asp Leu Leu Val
   1 5 10
<210> 415
   <211> 9
   <212> PRT
   <213> Hepatitis C virus
<400> 415
   Asp Leu Cys Gly Ser Val Phe Leu Val
   1 5
<210> 416
   <211> 9
   <212> PRT
   <213> Hepatitis C virus
<400> 416
   Ser Met Val Gly Asn Trp Ala Lys Val
   1 5
<210> 417
   <211> 9
   <212> PRT
   <213> Hepatitis C virus
<400> 417
   His Leu His Gln Asn Ile Val Asp Val
   1 5
<210> 418
   <211> 9
   <212> PRT
   <213> Hepatitis C virus
<400> 418
   Phe Leu Leu Leu Ala Asp Ala Arg Val
   1 5
<210> 419
   <211> 13
   <212> PRT
   <213> Hepatitis C virus
<400> 419
   Gly Leu Arg Asp Leu Ala Val Ala Val Glu Pro Val Val
   1 5 10
<210> 420
   <211> 11
   <212> PRT
   <213> Hepatitis C virus
<400> 420
   Ser Leu Leu Ala Pro Gly Ala Lys Gln Asn Val
   1 5 10
<210> 421
   <211> 10
   <212> PRT
   <213> Hepatitis C virus
<400> 421
   Leu Leu Ala Pro Gly Ala Lys Gln Asn Val
   1 5 10
<210> 422
   <211> 9
   <212> PRT
   <213> Hepatitis B virus
<400> 422
   Phe Leu Leu Ser Leu Gly Ile His Leu
   1 5
<210> 423
   <211> 9
   <212> PRT
   <213> Hepatitis B virus
<400> 423
   Ser Leu Tyr Ala Asp Ser Pro Ser Val
   1 5
<210> 424
   <211> 9
   <212> PRT
   <213> Hepatitis B virus
<400> 424
   Gly Leu Ser Arg Tyr Val Ala Arg Leu
   1 5
<210> 425
   <211> 9
   <212> PRT
   <213> human
<400> 425
   Lys Ile Phe Gly Ser Leu Ala Phe Leu
   1 5
<210> 426
   <211> 9
   <212> PRT
   <213> human
<400> 426
   Glu Leu Val Ser Glu Phe Ser Arg Met
   1 5
<210> 427
   <211> 9
   <212> PRT
   <213> Human immunodeficiency virus type 1
<400> 427
   Lys Leu Thr Pro Leu Cys Val Thr Leu
   1 5
<210> 428
   <211> 10
   <212> PRT
   <213> Human immunodeficiency virus type 1
<400> 428
   Ser Leu Leu Asn Ala Thr Asp Ile Ala Val
   1 5 10
<210> 429
   <211> 10
   <212> PRT
   <213> human
<400> 429
   Val Leu Tyr Arg Tyr Gly Ser Phe Ser Val
   1 5 10
<210> 430
   <211> 9
   <212> PRT
   <213> human
<400> 430
   Tyr Ile Gly Glu Val Leu Val Ser Val
   1 5
<210> 431
   <211> 10
   <212> PRT
   <213> Hepatitis C virus
<400> 431
   Leu Leu Phe Asn Ile Leu Gly Gly Trp Val
   1 5 10
<210> 432
   <211> 10
   <212> PRT
   <213> Hepatitis B virus
<400> 432
   Leu Leu Val Pro Phe Val Gln Trp Phe Trp
   1 5 10
<210> 433
   <211> 9
   <212> PRT
   <213> Hepatitis B virus
<400> 433
   Ala Leu Met Pro Leu Tyr Ala Cys Ile
   1 5
<210> 434
   <211> 9
   <212> PRT
   <213> Hepatitis C virus
<400> 434
   Tyr Leu Val Ala Tyr Gln Ala Thr Val
   1 5
<210> 435
   <211> 9
   <212> PRT
   <213> Human papillomavirus type 16
<400> 435
   Thr Leu Gly Ile Val Cys Pro Ile Cys
   1 5
<210> 436
   <211> 10
   <212> PRT
   <213> Hepatitis C virus
<400> 436
   Tyr Leu Leu Pro Arg Arg Gly Pro Arg Leu
   1 5 10
<210> 437
   <211> 10
   <212> PRT
   <213> Hepatitis B virus
<400> 437
   Leu Leu Pro Ile Phe Phe Cys Leu Trp Val
   1 5 10
<210> 438
   <211> 9
   <212> PRT
   <213> Hepatitis B virus
<400> 438
   Tyr Met Asp Asp Val Val Leu Gly Ala
   1 5
<210> 439
   <211> 9
   <212> PRT
   <213> Human papillomavirus type 16
<400> 439
   Gly Thr Leu Gly Ile Val Cys Pro Ile
   1 5
<210> 440
   <211> 10
   <212> PRT
   <213> Hepatitis C virus
<400> 440
   Leu Leu Ala Leu Leu Ser Cys Leu Thr Ile
   1 5 10
<210> 441
   <211> 9
   <212> PRT
   <213> Human papillomavirus type 16
<400> 441
   Met Leu Asp Leu Gln Pro Glu Thr Thr
   1 5
<210> 442
   <211> 9
   <212> PRT
   <213> Hepatitis C virus
<400> 442
   Ser Leu Met Ala Phe Thr Ala Ala Val
   1 5
<210> 443
   <211> 9
   <212> PRT
   <213> Hepatitis C virus
<400> 443
   Cys Ile Asn Gly Val Cys Trp Thr Val
   1 5
<210> 444
   <211> 10
   <212> PRT
   <213> human
<400> 444
   Val Met Asn Ile Leu Leu Gln Tyr Val Val
   1 5 10
<210> 445
   <211> 9
   <212> PRT
   <213> human
<400> 445
   Ile Leu Thr Val Ile Leu Gly Val Leu
   1 5
<210> 446
   <211> 9
   <212> PRT
   <213> human
<400> 446
   Phe Leu Trp Gly Pro Arg Ala Leu Val
   1 5
<210> 447
   <211> 9
   <212> PRT
   <213> Hepatitis C virus
<400> 447
   Leu Leu Cys Pro Ala Gly His Ala Val
   1 5
<210> 448
   <211> 9
   <212> PRT
   <213> Hepatitis C virus
<400> 448
   Ile Leu Asp Ser Phe Asp Pro Leu Val
   1 5
<210> 449
   <211> 9
   <212> PRT
   <213> Hepatitis B virus
<400> 449
   Leu Leu Leu Cys Leu Ile Phe Leu Leu
   1 5
<210> 450
   <211> 10
   <212> PRT
   <213> Hepatitis B virus
<400> 450
   Leu Ile Asp Tyr Gln Gly Met Leu Pro Val
   1 5 10
<210> 451
   <211> 10
   <212> PRT
   <213> Hepatitis B virus
<400> 451
   Ser Ile Val Ser Pro Phe Ile Pro Leu Leu
   1 5 10
<210> 452
   <211> 9
   <212> PRT
   <213> Hepatitis B virus
<400> 452
   Phe Leu Leu Thr Arg Ile Leu Thr Ile
   1 5
<210> 453
   <211> 9
   <212> PRT
   <213> Plasmodium falciparum
<400> 453
   His Leu Gly Asn Val Lys Tyr Leu Val
   1 5
<210> 454
   <211> 9
   <212> PRT
   <213> Plasmodium falciparum
<400> 454
   Gly Ile Ala Gly Gly Leu Ala Leu Leu
   1 5
<210> 455
   <211> 9
   <212> PRT
   <213> Hepatitis C virus
<400> 455
   Ile Leu Ala Gly Tyr Gly Ala Gly Val
   1 5
<210> 456
   <211> 9
   <212> PRT
   <213> Hepatitis C virus
<400> 456
   Gly Leu Gln Asp Cys Thr Met Leu Val
   1 5
<210> 457
   <211> 10
   <212> PRT
   <213> Hepatitis C virus
<400> 457
   Thr Gly Ala Pro Val Thr Tyr Ser Thr Tyr
   1 5 10
<210> 458
   <211> 9
   <212> PRT
   <213> Human immunodeficiency virus type 1
<400> 458
   Val Ile Tyr Gln Tyr Met Asp Asp Leu
   1 5
<210> 459
   <211> 10
   <212> PRT
   <213> human
<400> 459
   Val Leu Pro Asp Val Phe Ile Arg Cys Val
   1 5 10
<210> 460
   <211> 9
   <212> PRT
   <213> human
<400> 460
   Val Leu Pro Asp Val Phe Ile Arg Cys
   1 5
<210> 461
   <211> 9
   <212> PRT
   <213> human
<400> 461
   Ala Val Gly Ile Gly Ile Ala Val Val
   1 5
<210> 462
   <211> 9
   <212> PRT
   <213> human
<400> 462
   Leu Val Val Leu Gly Leu Leu Ala Val
   1 5
<210> 463
   <211> 9
   <212> PRT
   <213> human
<400> 463
   Ala Leu Gly Leu Gly Leu Leu Pro Val
   1 5
<210> 464
   <211> 9
   <212> PRT
   <213> HSV-2
<400> 464
   Gly Ala Gly Ile Gly Val Ala Val Leu
   1 5
<210> 465
   <211> 9
   <212> PRT
   <213> Pseudorabies virus
<400> 465
   Ile Ala Gly Ile Gly Ile Leu Ala Ile
   1 5
<210> 466
   <211> 9
   <212> PRT
   <213> Adenovirus 3
<400> 466
   Leu Ile Val Ile Gly Ile Leu Ile Leu
   1 5
<210> 467
   <211> 9
   <212> PRT
   <213> S. lincolnensis
<400> 467
   Leu Ala Gly Ile Gly Leu Ile Ala Ala
   1 5
<210> 468
   <211> 9
   <212> PRT
   <213> yeast
<400> 468 1 5
<210> 469
   <211> 9
   <212> PRT
   <213> B. polymyxa
<400> 469
   Gly Ala Gly Ile Gly Val Leu Thr Ala
   1 5
<210> 470
   <211> 9
   <212> PRT
   <213> Escherichia coli
<400> 470
   Ala Ala Gly Ile Gly Ile Ile Gln Ile
   1 5
<210> 471
   <211> 9
   <212> PRT
   <213> Escherichia coli
<400> 471
   Gln Ala Gly Ile Gly Ile Leu Leu Ala
   1 5
<210> 472
   <211> 11
   <212> PRT
   <213> human
<400> 472
   Lys Ala Arg Asp Pro His Ser Gly His Phe Val
   1 5 10
<210> 473
   <211> 11
   <212> PRT
   <213> human
<400> 473
   Lys Ala Cys Asp Pro His Ser Gly His Phe Val
   1 5 10
<210> 474
   <211> 10
   <212> PRT
   <213> human
<400> 474
   Ala Cys Asp Pro His Ser Gly His Phe Val
   1 5 10
<210> 475
   <211> 9
   <212> PRT
   <213> Human immunodeficiency virus type 1
<400> 475
   Ser Leu Tyr Asn Thr Val Ala Thr Leu
   1 5
<210> 476
   <211> 9
   <212> PRT
   <213> human
<400> 476
   Glu Leu Val Ser Glu Phe Ser Arg Val
   1 5
<210> 477
   <211> 10
   <212> PRT
   <213> Human immunodeficiency virus type 1
<400> 477
   Arg Gly Pro Gly Arg Ala Phe Val Thr Ile
   1 5 10
<210> 478
   <211> 9
   <212> PRT
   <213> Hepatitis C virus
<400> 478
   His Met Trp Asn Phe Ile Ser Gly Ile
   1 5
<210> 479
   <211> 10
   <212> PRT
   <213> Human cytomegalovirus
<400> 479
   Asn Leu Val Pro Met Val Ala Thr Val Gln
   1 5 10
<210> 480
   <211> 9
   <212> PRT
   <213> Human papillomavirus type 6b
<400> 480
<210> 481
   <211> 9
   <212> PRT
   <213> Human papillomavirus type 6b
<400> 481
   Pro Leu Lys Gln His Phe Gln Ile Val
   1 5
<210> 482
   <211> 10
   <212> PRT
   <213> EBV
<400> 482
   Leu Leu Asp Phe Val Arg Phe Met Gly Val
   1 5 10
<210> 483
   <211> 9
   <212> PRT
   <213> Haemophilus influenzae
<400> 483
   Ala Ile Met Glu Lys Asn Ile Met Leu
   1 5
<210> 484
   <211> 9
   <212> PRT
   <213> Plasmodium falciparum
<400> 484
   Tyr Leu Lys Thr Ile Gln Asn Ser Leu
   1 5
<210> 485
   <211> 9
   <212> PRT
   <213> Plasmodium falciparum
<400> 485
   Tyr Leu Asn Lys Ile Gln Asn Ser Leu
   1 5
<210> 486
   <211> 10
   <212> PRT
   <213> Human papillomavirus type 16
<400> 486
   Tyr Met Leu Asp Leu Gln Pro Glu Thr Thr
   1 5 10
<210> 487
   <211> 9
   <212> PRT
   <213> Human papillomavirus type 16
<400> 487
   Leu Leu Met Gly Thr Leu Gly Ile Val
   1 5
<210> 488
   <211> 8
   <212> PRT
   <213> Human papillomavirus type 16
<400> 488
   Thr Leu Gly Ile Val Cys Pro Ile
   1 5
<210> 489
   <211> 9
   <212> PRT
   <213> Human immunodeficiency virus type 1
<400> 489
   Thr Leu Thr Ser Cys Asn Thr Ser Val
   1 5
<210> 490
   <211> 9
   <212> PRT
   <213> Human papillomavirus type 16
<400> 490
   Lys Leu Pro Gln Leu Cys Thr Glu Leu
   1 5
<210> 491
   <211> 9
   <212> PRT
   <213> Human papillomavirus type 16
<400> 491
   Thr Ile His Asp Ile Ile Leu Glu Cys
   1 5
<210> 492
   <211> 9
   <212> PRT
   <213> Human papillomavirus type 16
<400> 492
   Leu Gly Ile Val Cys Pro Ile Cys Ser
   1 5
<210> 493
   <211> 9
   <212> PRT
   <213> human
<400> 493
   Val Ile Leu Gly Val Leu Leu Leu Ile
   1 5
<210> 494
   <211> 9
   <212> PRT
   <213> 'Axial Seamount' polynoid polychaete
<400> 494
   Ala Leu Met Asp Lys Ser Leu His Val
   1 5
<210> 495
   <211> 9
   <212> PRT
   <213> human
<400> 495
   Gly Ile Leu Thr Val Ile Leu Gly Val
   1 5
<210> 496
   <211> 9
   <212> PRT
   <213> Plasmodium falciparum
<400> 496
   Met Ile Asn Ala Tyr Leu Asp Lys Leu
   1 5
<210> 497
   <211> 9
   <212> PRT
   <213> human
<400> 497
   Ala Ala Gly Ile Gly Ile Leu Thr Val
   1 5
<210> 498
   <211> 10
   <212> PRT
   <213> human
<400> 498
   Phe Leu Pro Ser Asp Phe Phe Pro Ser Val
   1 5 10
<210> 499
   <211> 9
   <212> PRT
   <213> EBV
<400> 499
   Ser Val Arg Asp Arg Leu Ala Arg Leu
   1 5
<210> 500
   <211> 9
   <212> PRT
   <213> human
<400> 500
   Phe Ala Tyr Asp Gly Lys Asp Tyr Ile
   1 5
<210> 501
   <211> 9
   <212> PRT
   <213> human
<400> 501
   Ala Leu Leu Ala Val Gly Ala Thr Lys
   1 5
<210> 502
   <211> 11
   <212> PRT
   <213> Human immunodeficiency virus type 1
<400> 502
   Arg Leu Arg Asp Leu Leu Leu Ile Val Thr Arg
   1 5 10
<210> 503
   <211> 10
   <212> PRT
   <213> Human immunodeficiency virus type 1
<400> 503
   Gln Val Pro Leu Arg Pro Met Thr Tyr Lys
   1 5 10
<210> 504
   <211> 10
   <212> PRT
   <213> Human immunodeficiency virus type 1
<400> 504
   Thr Val Tyr Tyr Gly Val Pro Val Trp Lys
   1 5 10
<210> 505
   <211> 9
   <212> PRT
   <213> Human immunodeficiency virus type 1
<400> 505
   Arg Leu Arg Pro Gly Gly Lys Lys Lys
   1 5
<210> 506
   <211> 9
   <212> PRT
   <213> Haemophilus influenzae
<400> 506
   Ile Leu Arg Gly Ser Val Ala His Lys
   1 5
<210> 507
   <211> 9
   <212> PRT
   <213> EBV
<400> 507
   Arg Leu Arg Ala Glu Ala Gly Val Lys
   1 5
<210> 508
   <211> 9
   <212> PRT
   <213> Human immunodeficiency virus type 1
<400> 508
   Val Tyr Tyr Gly Val Pro Val Trp Lys
   1 5
<210> 509
   <211> 9
   <212> PRT
   <213> Hepatitis C virus
<400> 509
   Arg Val Cys Glu Lys Met Ala Leu Tyr
   1 5
<210> 510
   <211> 9
   <212> PRT
   <213> human
<400> 510
   Lys Ile Phe Ser Glu Val Thr Leu Lys
   1 5
<210> 511
   <211> 9
   <212> PRT
   <213> Hepatitis B virus
<400> 511
   Tyr Val Asn Val Asn Met Gly Leu Lys
   1 5
<210> 512
   <211> 9
   <212> PRT
   <213> EBV
<400> 512
   Ile Val Thr Asp Phe Ser Val Ile Lys
   1 5
<210> 513
   <211> 8
   <212> PRT
   <213> human
<400> 513
   Glu Leu Asn Glu Ala Glu Leu Lys
   1 5
<210> 514
   <211> 9
   <212> PRT
   <213> Human immunodeficiency virus type 1
<400> 514
   Val Pro Leu Arg Pro Met Thr Tyr Lys
   1 5
<210> 515
   <211> 9
   <212> PRT
   <213> Human immunodeficiency virus type 1
<400> 515
   Ala Ile Phe Gln Ser Ser Met Thr Lys
   1 5
<210> 516
   <211> 10
   <212> PRT
   <213> Human immunodeficiency virus type 1
<400> 516
   Gln Val Pro Leu Arg Pro Met Thr Tyr Lys
   1 5 10
<210> 517
   <211> 8
   <212> PRT
   <213> Hepatitis C virus
<400> 517
   Thr Ile Asn Tyr Thr Ile Phe Lys
   1 5
<210> 518
   <211> 12
   <212> PRT
   <213> Human immunodeficiency virus type 1
<400> 518
   Ala Ala Val Asp Leu Ser His Phe Leu Lys Glu Lys
   1 5 10
<210> 519
   <211> 12
   <212> PRT
   <213> Human immunodeficiency virus type 1
<400> 519
   Ala Cys Gln Gly Val Gly Gly Pro Gly Gly His Lys
   1 5 10
<210> 520
   <211> 9
   <212> PRT
   <213> human
<400> 520
   Ser Tyr Leu Asp Ser Gly Ile His Phe
   1 5
<210> 521
   <211> 9
   <212> PRT
   <213> Human immunodeficiency virus type 1
<400> 521
   Arg Tyr Leu Lys Asp Gln Gln Leu Leu
   1 5
<210> 522
   <211> 9
   <212> PRT
   <213> human
<400> 522
   Ala Tyr Gly Leu Asp Phe Tyr Ile Leu
   1 5
<210> 523
   <211> 10
   <212> PRT
   <213> human
<400> 523
   Ala Phe Leu Pro Trp His Arg Leu Phe Leu
   1 5 10
<210> 524
   <211> 9
   <212> PRT
   <213> human
<400> 524
   Ala Phe Leu Pro Trp His Arg Leu Phe
   1 5
<210> 525
   <211> 8
   <212> PRT
   <213> EBV
<400> 525
   Arg Tyr Ser Ile Phe Phe Asp Tyr
   1 5
<210> 526
   <211> 10
   <212> PRT
   <213> Human immunodeficiency virus type 1
<400> 526
   Glu Thr Ile Asn Glu Glu Ala Ala Glu Trp
   1 5 10
<210> 527
   <211> 11
   <212> PRT
   <213> Hepatitis B virus
<400> 527
   Ser Thr Leu Pro Glu Thr Thr Val Val Arg Arg
   1 5 10
<210> 528
   <211> 9
   <212> PRT
   <213> human
<400> 528
   Met Ser Leu Gln Arg Gln Phe Leu Arg
   1 5
<210> 529
   <211> 9
   <212> PRT
   <213> human
<400> 529
   Leu Leu Pro Gly Gly Arg Pro Tyr Arg
   1 5
<210> 530
   <211> 9
   <212> PRT
   <213> Human immunodeficiency virus type 1
<400> 530
   Ile Val Gly Leu Asn Lys Ile Val Arg
   1 5
<210> 531
   <211> 9
   <212> PRT
   <213> human
<400> 531
   Ala Ala Gly Ile Gly Ile Leu Thr Val
   1 5
<210> 532
   <211> 9
   <212> PRT
   <213> human
<400> 532
   Glu Val Asp Pro Ala Ser Asn Thr Tyr
   1 5
<210> 533
   <211> 9
   <212> PRT
   <213> human
<400> 533
   Glu Val Asp Pro Thr Ser Asn Thr Tyr
   1 5
<210> 534
   <211> 9
   <212> PRT
   <213> human
<400> 534
   Glu Ala Asp Pro Thr Ser Asn Thr Tyr
   1 5
<210> 535
   <211> 9
   <212> PRT
   <213> human
<400> 535
   Glu Val Asp Pro Ile Gly His Val Tyr
   1 5
<210> 536
   <211> 11
   <212> PRT
   <213> human
<400> 536
   Met Leu Leu Ala Val Leu Leu Tyr Cys Leu Leu
   1 5 10
<210> 537
   <211> 9
   <212> PRT
   <213> human
<400> 537
   Met Leu Leu Ala Val Leu Tyr Cys Leu
   1 5
<210> 538
   <211> 10
   <212> PRT
   <213> human
<400> 538
   Ser Glu Ile Trp Arg Asp Ile Asp Phe Ala
   1 5 10
<210> 539
   <211> 9
   <212> PRT
   <213> human
<400> 539
   Ser Glu Ile Trp Arg Asp Ile Asp Phe
   1 5
<210> 540
   <211> 9
   <212> PRT
   <213> human
<400> 540
   Xaa Glu Ile Trp Arg Asp Ile Asp Phe
   1 5
<210> 541
   <211> 11
   <212> PRT
   <213> human
<400> 541
   Ser Thr Leu Val Glu Val Thr Leu Gly Glu Val
   1 5 10
<210> 542
   <211> 9
   <212> PRT
   <213> human
<400> 542
   Leu Val Glu Val Thr Leu Gly Glu Val
   1 5
<210> 543
   <211> 10
   <212> PRT
   <213> human
<400> 543
   Val Ile Phe Ser Lys Ala Ser Glu Tyr Leu
   1 5 10
<210> 544
   <211> 9
   <212> PRT
   <213> human
<400> 544
   Ile Ile Val Leu Ala Ile Ile Ala Ile
   1 5
<210> 545
   <211> 11
   <212> PRT
   <213> human
<400> 545
   Lys Ile Trp Glu Glu Leu Ser Met Leu Glu Val
   1 5 10
<210> 546
   <211> 9
   <212> PRT
   <213> human
<400> 546
   Leu Ile Glu Thr Ser Tyr Val Lys Val
   1 5
<210> 547
   <211> 9
   <212> PRT
   <213> human
<400> 547
   Phe Leu Trp Gly Pro Arg Ala Leu Val
   1 5
<210> 548
   <211> 10
   <212> PRT
   <213> human
<400> 548
   Thr Leu Val Glu Val Thr Leu Gly Glu Val
   1 5 10
<210> 549
   <211> 10
   <212> PRT
   <213> human
<400> 549
   Ala Leu Val Glu Thr Ser Tyr Val Lys Val
   1 5 10
<210> 550
   <211> 9
   <212> PRT
   <213> human
<400> 550
   Lys Ile Trp Glu Glu Leu Ser Val Leu
   1 5
<210> 551
   <211> 9
   <212> PRT
   <213> human
<400> 551
   Glu Val Asp Pro Ile Gly His Leu Tyr
   1 5
<210> 552
   <211> 9
   <212> PRT
   <213> human
<400> 552
   Glu Xaa Asp Xaa Xaa Xaa Xaa Xaa Tyr
   1 5
<210> 553
   <211> 9
   <212> PRT
   <213> human
<400> 553
   Glu Ala Asp Pro Thr Gly His Ser Tyr
   1 5
<210> 554
   <211> 9
   <212> PRT
   <213> human
<220>
   <221> VARIANT
   <222> (2)
   <223> val
<400> 554
   Glu Ala Asp Xaa Xaa Xaa Xaa Xaa Tyr
   1 5
<210> 555
   <211> 9
   <212> PRT
   <213> human
<220>
   <221> VARIANT
   <222> (2)
   <223> val
<400> 555
   Glu Ala Asp Pro Xaa Xaa Xaa Xaa Tyr
   1 5
<210> 556
   <211> 9
   <212> PRT
   <213> human
<220>
   <221> VARIANT
   <222> (2)
   <223> val
<220>
   <221> VARIANT
   <222> (5)
   <223> ala or thr
<400> 556
   Glu Ala Asp Pro Ile Xaa Xaa Xaa Tyr
   1 5
<210> 557
   <211> 9
   <212> PRT
   <213> human
<220>
   <221> VARIANT
   <222> (2)
   <223> val
<220>
   <221> VARIANT
   <222> (5)
   <223> ala or thr
<220>
   <221> VARIANT
   <222> (6)
   <223> ser
<400> 557
   Glu Ala Asp Pro Ile Gly Xaa Xaa Tyr
   1 5
<210> 558
   <211> 9
   <212> PRT
   <213> human
<220>
   <221> VARIANT
   <222> (2)
   <223> val
<220>
   <221> VARIANT
   <222> (5)
   <223> ala or thr
<220>
   <221> VARIANT
   <222> (6)
   <223> ser
<220>
   <221> VARIANT
   <222> (7)
   <223> asn
<400> 558
   Glu Ala Asp Pro Ile Gly His Xaa Tyr
   1 5
<210> 559
   <211> 9
   <212> PRT
   <213> human
<220>
   <221> VARIANT
   <222> (2)
   <223> val
<220>
   <221> VARIANT
   <222> (5)
   <223> ala or thr
<220>
   <221> VARIANT
   <222> (6)
   <223> ser
<220>
   <221> VARIANT
   <222> (7)
   <223> asn
<220>
   <221> VARIANT
   <222> (8)
   <223> thr or val
<400> 559
   Glu Ala Asp Pro Ile Gly His Leu Tyr
   1 5
<210> 560
   <211> 16
   <212> PRT
   <213> human
<400> 560
   Glu Leu His Ser Ala Tyr Gly Glu Pro Arg Lys Leu Leu Thr Gln Asp
   1 5 10 15
<210> 561
   <211> 12
   <212> PRT
   <213> human
<400> 561
   Glu His Ser Ala Tyr Gly Glu Pro Arg Lys Leu Leu
   1 5 10
<210> 562
   <211> 9
   <212> PRT
   <213> human
<400> 562
   Ser Ala Tyr Gly Glu Pro Arg Lys Leu
   1 5
<210> 563
   <211> 9
   <212> PRT
   <213> human
<400> 563
   Glu Ala Asp Pro Thr Gly His Ser Tyr
   1 5
<210> 564
   <211> 22
   <212> PRT
   <213> human
<400> 564
   Met Ala Ala Arg Ala Val Phe Leu Ala Leu Ser Ala Gln Leu Leu Gln
   1 5 10 15
   Ala Arg Leu Met Lys Glu
   20
<210> 565
   <211> 16
   <212> PRT
   <213> human
<400> 565
   Met Ala Ala Arg Ala Val Phe Leu Ala Leu Ser Ala Gln Leu Leu Gln
   1 5 10 15
<210> 566
   <211> 9
   <212> PRT
   <213> human
<400> 566
   Ala Ala Arg Ala Val Phe Leu Ala Leu
   1 5
<210> 567
   <211> 8
   <212> PRT
   <213> human
<400> 567
   Tyr Arg Pro Arg Pro Arg Arg Tyr
   1 5
<210> 568
   <211> 9
   <212> PRT
   <213> Human immunodeficiency virus type 1
<400> 568
   Asp Leu Asn Thr Met Leu Asn Thr Val
   1 5
<210> 569
   <211> 9
   <212> PRT
   <213> LCMV
<400> 569
   Lys Ala Val Tyr Asn Phe Ala Thr Cys
   1 5